# EUROPEAN PATENT APPLICATION

(11) **EP 1 739 584 A1**
(43) Date of publication of application: **03.01.2007**
(21) Application number: 05727930.9
(22) Date of filing: 30.03.2005
(51) Int. Cl.: G06F 19/00, C12N 15/00

(54) **DOCUMENT INFORMATION PROCESSING SYSTEM**

(30) Priority: 30.03.2004 JP 2004097914
(71) Applicant: Ihara, Shigeo, Tokorozowa-shi Saitama 3590003 (JP); Aburatani, Hiroyuki, Musashino-shi, Tokyo 180-0003 (JP); Tsutsumi, Shuichi, Tokyo 1120001 (JP); Nishikawa, Naoko, Abiko-shi Chiba 2701166 (JP); Yamamoto, Shogo, Takatsu-ku Kawasaki-shi Kanagawa 2130023 (JP); Abe, Koji, Nakano-ku Tokyo (JP); Tsuji, Shingo, Adachi-ku Tokyo 1200024 (JP)
(72) Inventor: Ihara, Shigeo, Tokorozowa-shi Saitama 3590003 (JP); Aburatani, Hiroyuki, Musashino-shi, Tokyo 180-0003 (JP); Tsutsumi, Shuichi, Tokyo 1120001 (JP); Nishikawa, Naoko, Abiko-shi Chiba 2701166 (JP); Yamamoto, Shogo, Takatsu-ku Kawasaki-shi Kanagawa 2130023 (JP); Abe, Koji, Nakano-ku Tokyo (JP); Tsuji, Shingo, Adachi-ku Tokyo 1200024 (JP)
(74) Representative: Kuhnen & Wacker
(86) International application number: PCT/JP2005/006025
(87) International publication number: WO 2005/096207

(57) **Abstract**

A literature information processing system, comprising: a dictionary (16) to store data of element names and verbs indicating mutual interaction relations between the element names; a literature database (14) to store a large number of data for literature information; an input means(12) to enter plural element names; and a data control unit (10) to extract multi-body interaction relations for every plural element name entered in reference to the above dictionary or the above literature database, to extract overlapping parts of the multi-body interaction relations extracted for each of plural element names, and to draw a pathway map indicating the overlapping parts extracted by the above overlapping part extracting means as one information.

## Description

### TECHNICAL FIELD

This invention relates to a Literature Information Processing System that analyzes literature information by natural language processing and provides an output of the analysis result.

### BACKGROUND ART

Generally it becomes possible to reveal genetic function and structure by degrees through the recent development of gene analysis technology. Above all, DNA microarray technology is noted for its superiority in the gene analysis methods. The surface of DNA microarray consists of different DNA (probe) aligned in a very dense state on surface of a flat board (glass, silicon, plastic, etc.).For probes, cDNA, short-chain nucleotides (20-30 base) and so on are ordinarily used.

The basis of DNA microarray is utilization of hybridization, i.e. the forming hydrogen bonding between A (Adenine) and T (Thymine), and that between G (Guanine) and C (Cytosine). On this DNA microarray, we capture the target DNA by the DNA or RNA hybridization that has been marked with fluorescent material. The signal of the captured target is included in the hybridization signals, which can be detected as a fluorescence signal from each spot. By analyzing this data with computers, we can observe the state of 1000 - several tens of thousands of DNA at a time, and for numbers of genes at one time we can monitor the gene expressions.

As for the functions of gene and protein elements etc, numerous studies have already been conducted, and the articles on these studies are stored in a database. The data on the interaction between genes and proteins stores in the text of the articles is important, but it is difficult for users to examine each sentence from articles and find these interactions because there are enormous numbers of articles in the database. Consequently, there are approaches that automatically search articles stored electronically in the database and select the names of the elements described within articles are important issues in the natural language processing. Furthermore, using the natural language processing, these approaches can extract the connections between some of two elements (for instance co-occurrence), called a binary relation, and draw the combined network of the connections as a pathway map.

There is a system that analyzes the pathway of proteins and genes, which is necessary in understanding biological processes. (see http://www.infocom.co.jp/bio/bioinfo.pathway. html) In addition, there also is a network that shows the connection between biological molecules searched via disorder name. (see http://www.immd.co.jp/keymolnet/027k6d2x40/Key Molnet0305Rla.pdf)

### DISCLOSURE OF THE INVENTION

In existing systems, pathway analysis and pathway map drawing are performed for one by one protein and gene, therefore it takes a large amount of time in the effort to analyze and draw pathways of various proteins and genes obtained as a result of DNA microarray. Moreover, because of this, much more time and work is required to analyze and understand the complex relationship between resulting proteins and genes that are obtained as the above existing pathway analysis tools.

The purpose of this invention, referred to henceforth as, "The Literature Information Processing System," is to provide a Literature Information Processing System that can easily analyze the interaction of a large number of element names and draw a pathway map.

The Literature Information Processing System has the following characteristics: 1) the dictionary that stores multiple element names and the verbs that indicate the interactions between element names, 2) the literature database that stores multiple literature information, 3) the input means to enter element names, 4) the multi-body interactions extracting means to extract multi-body interactions of every element name entered in reference to the above dictionary and the above literature database, and 5) the pathway map drawing means to draw the overlapping parts extracted by the multi-body interaction extracting means.

By using Literature Information Processing System, we can obtain the information of the extracted multi-body interactions of every element name entered in reference to the dictionary while the literature database draws pathway maps of the extracted multi-body interactions. In other words, the system can extract multi-body interactions and draw pathway maps simultaneously. Consequently, the system can expeditiously extract the multi-body interactions and draw the pathway map of each multiple element name entered.

The Literature Information Processing System has the following characteristics: 1) the dictionary to store multiple element names and the verbs that indicate the interactions between element names, 2) the literature database to store multiple literature information, 3) the input means to enter element names, 4) the decision making means to determine whether multi-body interactions of the above element names should be extracted or not, 5) the multi-body interactions extracting means to extract the multi-body interactions in reference to the above dictionary and the above literature database, and 6) the pathway map drawing means to draw a pathway map on the basis of the multi-body interactions extracted on the basis of the multi-body interactions extracted by the above decision making means.

The Literature Information Processing System evaluates whether the multi-body interactions are extracted from each multiple element name or not, then extracts the multi-body interactions from the element names whose extractions are incomplete in reference to the dictionary and the literature database. Then, it draws the pathway maps based on the extracted multi-body interactions. As a result, the system does not redundantly extract multi-body interactions, thus the system can extract multi-body interactions and draw pathway maps very quickly for each multiple entered element name.

The Literature Information Processing System includes an additional function of the above dictionary that also stores the noun phrases and the adjective phrases that indicate the interactions between the element names. The system can extract the multiple precise connections extensively because the system drastically increases the vocabulary stored in the dictionary.

Furthermore, the Literature Information Processing System has the following characteristics: 1) the literature database to store the multiple literature information, 2) the input means to enter element names, 3) the multi-body interactions extracting means to extract multi-body interactions of each multiple element name entered in reference to the above literature database on the basis of the verbs indicating the interactions between the above element names, 4) the overlapping part extracting means to extract the overlapping parts of the multi-body interactions extracted for every element name, and 5) the pathway map drawing means to draw the overlapping parts extracted by the above overlapping parts extracting means as one unit of information.

The Literature Information Processing System extracts multi-body interactions of every multi-entered element name in reference to the literature database and draws a pathway map of the extracted multi-body interactions. In other words, the system can extract multi-body interactions of each multiple element name simultaneously and draw the pathway map in reference to the only literature database. Consequently, without having the dictionary that stores multiple element names and contains verbs that indicate interactions between the multiple element names within the system, the system can extract the multi-body interactions and draw pathway maps of each multiple element name entered very quickly with simple system architecture.

Further, the Literature Information Processing System has an extra feature where the above multi-body interactions extracting means extracts multi-body interactions based on noun phrases and adjective phrases that indicate the interactions between the element names. The Literature Information Processing System can vastly extract precise multi-body interactions because the system extracts multi-body interactions not on verbs alone, but also on noun phrases and adjective phrases.

The Literature Information Processing System has following additional features: 1) the literature database means to store multiple literature information, 2) the input means to enter element names, 3) the decision making means to determine whether the multi-body interactions about the above element names are extracted based on the verb that indicates the interaction between the above element names or not, 4) the multi-body interactions extracting means to extract the multi-body interactions of the element names deemed not to be extracted in the multi-body interactions by the above decision making means in reference to the above literature database, and 5) the pathway map drawing means to draw the pathway map of the multi-body interactions extracted by the above multi-body interactions extracting means.

The Literature Information Processing System evaluates whether the multi-body interactions of each multiple element name entered should be extracted or not, and extracts the multi-body interactions from the element names whose multi-body interactions are not extracted the multi-body interactions by the above literature function in reference to the literature database. It then draws the pathway maps based on the multi-body interactions remaining. Consequently, without using the dictionary to store the multiple element names and the verbs that indicate interactions between the multiple element names with the system, the system can extract the multi-body interactions and draw pathway maps of every multiple element name entered very quickly with simple system architecture.

The Literature Information Processing System's decision making means has a feature that evaluates whether the multi-body interactions are extracted based on the noun phrases and the adjective phrases that indicate the interactions between the element names. The Literature Information Processing System of origination can extract a vast number of exact multi-body interactions because the system evaluates whether the extraction of multi-body interactions are done on verbs alone, or include noun phrases and adjective phrases.

The Literature Information Processing System's multi-body interactions extracting means also extracts the multi-body interactions of the element names entered by the above input means and those of the element names extracted as having multi-body interactions, and also those of the element names extracted.

The Literature Information Processing System's extraction range specifying means also specifies the range of extracting the multi-body interactions by the above multi-body interactions extracting function on the element names entered by the above input function.

The Literature Information Processing System can draw a simple pathway map or a detailed pathway map according to need because the system can specify the extraction range of the multi-body interactions on the element names entered.

The Literature Information Processing System's pathway map drawing function also discriminates by the above multiple relations extracting means and shows the element names entered by the above input means and the element names extracted from the element names entered by the above input means.

The Literature Information Processing System can make it easy to understand pathway maps drawn because the system can choose the element names entered by the input means and the element names extracted from the element names entered by the input means and shows them via pathway maps.

Another characteristic of the Literature Information Processing System is that it has the multiple relation indicating means to show the multiple relations extracted by the above multiple relation extracting means. This multiple relation indicating means chooses and shows the multiple positive and negative relationships.

The Literature Information Processing System makes it easy to figure out the multiple relations showed because the system can discriminate and show multiple positive and negative relations.

The Literature Information Processing System of this invention has the further following characteristics: 1) the dictionary to store the verbs that indicate the multiple element names and the interactions between the element names, 2) the literature database to store multiple literature information, 3) the first multi-body interactions extracting means to extract the multi-body interactions of each multiple element name in reference to the above dictionary and the above literature database, 4) the multi-body interactions storing means to store the multi-body interactions extracted by the first multi-body interactions extracting means, 5) the input means to enter element names, 6) the second multi-body interactions extracting means to extract the multi-body interactions of every multiple element name entered in reference to the multi-body interactions stored by the above multi-body interactions storing means, 7) the overlapping part extracting means to extract the overlapping parts of the multi-body interactions extracted by the above overlapping part extracting means, and 8) the pathway map drawing means to draw the overlapping part extracted by the above overlapping part extracting means as one unit of information.

The Literature Information Processing System extracts the multi-body interactions of each multiple element name entered in reference to the multi-body interactions storage that stores and extracts the multi-body interactions in advance, and draws the pathway map on the basis of the extracted multi-body interactions. In other words, the system can extract the multi-body interactions simultaneously and draw the pathway map for each multiple element name. Consequently, the system can extract the multi-body interactions and draw the pathway map for every multiple element name entered very quickly.

The Literature Information Processing System of this invention has the following characteristics: 1) the dictionary to store the verbs that indicate multiple element names and the interactions between the element names, 2) the literature database to store multiple literature information, 3) the first multi-body interaction extracting means to extract the multi-body interactions of each multiple element name in reference to the above dictionary and the above literature database, 4) the multi-body interaction storing means to store the multi-body interactions extracted by the above first multi-body interaction extracting means, 5) the input means to enter element names, 6) the decision making means to decide whether the above element names are extracted for the multi-body interactions or not, 7) the second multi-body interaction extracting means to extract the multi-body interactions of the element names whose multi-body interactions are not extracted by the above decision making means in reference to the multi-body interactions stored by the above multi-body interaction extracting means, and 8) the pathway drawing means to draw the pathway maps on the basis of the multi-body interactions extracted by the multi-body interaction extracting means.

The Literature Information Processing System determines whether the multi-body interactions of each of multiple element name entered are extracted or not, then extracts the multi-body interactions of the element names that are not included in the extraction of multi-body interactions in reference to the multi-body interaction storing storage which extracts and stores the multi-body interactions in advance, and draws the pathway map on the basis of the multi-body interactions extracted. Consequently, the system can extract the multi-body interactions and draw the pathway map very quickly because the system doesn't extract the multi-body interactions of element names redundantly.

Another characteristic of the Literature Information Processing System is that the above dictionary stores the noun phrases and adjective phrases that indicate the interactions between the element names. The Literature Information Processing System can extract vast numbers of precise multi-body interactions because the system can considerably increase vocabulary and expressions stored in the dictionary.

In addition the Literature Information Processing System has also extracts the multi-body interactions of the element names considered to have multi-body interactions with the element names entered by the above input means and extracts the multi-body interactions of the element names extracted.

The Literature Information Processing System of this invention has the extraction range specifying means to extract the range of the multi-body interactions using the above second multi-body interaction extracting means on the basis of the element names entered by the above input means.

The Literature Information Processing System can draw a simple pathway map and a detailed pathway maps according to need because the system can specify the range of the multi-body interactions to extract on the basis of the element names entered.

The Literature Information Processing System of this invention has the characteristic that the above pathway map drawing means identifies the element names entered by the above input means and the element names extracted from the element names entered using the above input means by the above second multi-body interactions extracting means.

The Literature Information Processing System can make it easy to understand the pathway maps drawn because the system can discriminate between the element names entered by the input means and the element names extracted from the element names entered using the input means.

The Literature Information Processing System of this invention has the following characteristics: the multi-body interaction categorizing means to categorize the multi-body interactions stored by the above multi-body interaction storing means on the basis of the verbs that indicate the interactions between the above element names, and the reliability assessment means that assesses the reliability of the multi-body interactions for every verb on the basis of the multi-body interactions of the all the verbs categorized using the above multi-body interactions categorizing means.

The Literature Information Processing System has the characteristic that the above reliability assessment means identifies the above element name as a node, identifies the connection between the above elements, and has the graph drawing means to draw the graph which indicates the connection between the above node and the above edge. It also has a means to assess the reliability on the basis of the graph drawn by the graph drawing means.

The Literature Information Processing System categorizes the multi-body interactions stored by the multi-body interactions storing means on the basis of the verb that indicates the interaction between the element names, and assesses the reliability of the multi-body interactions of every verb on the basis of the multi-body interactions of every verb categorized. In consequence, the system can draw the pathway map on the basis of the multi-body interactions of which reliability is ensured and increases the reliability of the pathway map.

The Literature Information Processing System also includes Internet information, so it can extract multi-body interactions and draw the pathway maps on based the latest literature information.

The Literature Information Processing System has the characteristic that the above element names are protein names and gene names and it can expeditiously draw the pathway maps that indicate the interactions between the protein/gene names, signaling pathways, and metabolic pathways.

The Literature Information Processing System also has the detection result input means to enter the element name based on the detection result by the DNA microarray analysis device.

The Literature Information Processing System's detection result input means enters the element name that is the result of the experiment drawn by at least two experiments of the above DNA microarray analysis device.

The Literature Information Processing System can directly enter the element name based on the detection result of DNA microarray analysis device, extract the multi-body interactions of element names entered, and draw a pathway map. In other words, the system can draw the pathway map very quickly on the basis of the detection results of the DNA microarray analysis device. In addition, because the system can enter the element names gained by more than two experiments at the same time and extract the multi-body interactions of the element names entered simultaneously, the system can draw the pathway map based on the detection result of DNA microarray analysis device very quickly.

The Literature Information Processing System's pathway map drawing means identifies and indicates the element names drawn on the pathway map on the basis of each experiment. The Literature Information Processing System can make it easy to figure out pathway maps because the system identifies and indicates the element names drawn on the pathway map on the basis of each experiment.

The Literature Information Processing System's pathway map drawing means indicates all the element names based on each experiment as element names drawn on the pathway map.

The Literature Information Processing System's pathway map drawing means indicates the intersection of the element names based on each experiment as element names drawn on the pathway map.

The Literature Information Processing System's pathway map drawing means indicates the different points of the element names based on each experiment as element names drawn on the pathway map.

The Literature Information Processing System can make it easy to understand the detection results indicated on the pathway map because the system can change the element names indicated on the pathway map according to need (for example, the system indicates all the element names based on each experiment as element names drawn on the pathway map, or the system indicates the intersection of the element names based on each experiment as element names drawn on the pathway map, and the system indicates the different points of the element names based on each experiment as element names drawn on a pathway map).

The Literature Information Processing System of this invention has the following characteristics: 1) the multi-body interactions storing means to store the multi-body interactions extracted from each multiple element names, 2) the input means to enter the element names, 3) the extraction range specifying means to specify the range to extract the multi-body interactions on the basis of the element names entered using the above input means, 4) a multi-body interaction extracting means to extract the multi-body interactions existing between the element names of the range already extracted as well as extracting the multi-body interactions of the range specified by the above extraction range specifying means in reference to the above multi-body interactions storage means for each element name entered, 5) the pathway map drawing means to draw the pathway map on the basis of the multi-body interactions extracted by the above multi-body interactions extracting means.

As this Literature Information Processing System specifies the extraction range and extracts the multi-body interactions of the range, the system extracts the multi-body interactions existing between the element names already extracted. Consequently, necessary information is not lost because needless element names are excluded, so the necessary information can be easily figured out from pathway maps visually because it is necessary to extract new element names as well as to extract the multi-body interactions existing between the element names already extracted. The processing time of extracting the multi-body interactions can be shortened, and the resources composing the Literature Information Processing System can be reduced. Furthermore, for example, by specifying the extraction range based on specific element names, the characteristic attribute that indicates element, and the connection of the verb that indicates interaction, the range of extracting necessary information can be configured properly.

The Literature Information Processing System of this invention has the following characteristics: 1) the relation pattern storage to store the relation patterns between the element names, 2) the verification means to verify the relationships between element names on pathway maps drawn by the above pathway map drawing means in reference to the relation patterns stored in the above relation pattern storage.

The Literature Information Processing System has the following characteristics: 1) the multi-body interactions storage means to store the multi-body interactions extracted for each multiple element name, 2) the input means to enter element names, 3) the defined condition entering means to enter the defined conditions that limit the range of the pathway map displayed, 4) the multi-body interaction extracting means to extract the multi-body interactions for every multiple element name entered in reference to the multi-body interactions storing means, and 5) the pathway map drawing means to draw pathway maps on the basis of the multi-body interactions extracted by the multi-body interaction extracting means and the defined conditions entered by the above defined condition entering means.

The Literature Information Processing System draws a pathway map on the basis of the defined conditions entered. In consequence, the system reduces the risk that necessary information gets buried and determination becomes difficult because of displaying a large amount of element names and makes it easy to figure out the necessary information accurately from the pathway map drawn.

The Literature Information Processing System also has the specific element name storing storage to store specific element names that interact between a large number of element names. Also the above pathway map drawing means changes the display of the multi-body interactions about the specific element names in reference to the specific names stored in the above specific element name storing storage.

The Literature Information Processing System's pathway map drawing means displays the information indicating the relationship of each element name when the multi-body interactions extracted by the above multi-body interaction extracting means includes at least three element names.

The Literature Information Processing System has a supplementary memorization and information storage area that stores the supplementary information about the above pathway map, and has the pathway map drawing means to draw the above pathway map in reference to the stored supplementary information.

The Literature Information Processing System includes the information indicating the predefined element names that the supplementary information are abbreviated-described and the information indicating predefined figures that are used when displaying the predefined element names. The pathway map drawing means uses the predefined figures to draw the pathway map in reference to the supplementary information when displaying the predefined element names.

The Literature Information Processing System includes the information of the material names that the above supplementary information has predefined connections with the interactions between the above element names, and has the characteristic that the above pathway map drawing means draws the pathway map including the above material name in reference to the above supplementary information.

The Literature Information Processing System has the following characteristics: 1) the literature database to store the multiple literature information, 2) the gene expression information database to store gene expression information, 3) the input means to enter element names, 4) the multi-body interactions extracting means to extract the multi-body interactions for each multiple element names entered by the input means in reference to the literature database and the gene expression information database, and 5) the pathway map drawing means to draw the pathway map on the basis of the multi-body interactions extracted by the multi-body interaction extracting means.

The Literature Information Processing System extracts the multi-body interactions in reference to the literature information and the gene expression information and draws the pathway map.

The Literature Information Processing System includes Internet information in the above literature information.

The Literature Information Processing System has the characteristic that the above element names are protein names or gene names.

The Literature Information Processing System evaluates whether the multi-body interactions that are extracted by the multi-body interactions extracting means are direct interactions or not in reference to the supplementary information storage area that stores the supplementary information that indicates the domain structure of the predefined proteins and the collateral relations between the domain structures of each protein in case the above element name is a protein.

The Literature Information Processing System has the following characteristics: 1) the binary relation storage area to store the binary relations extracted for each multiple protein name and gene name, 2) the input means to enter protein names and gene names, 3) the defined condition input means to enter the binary relations: a) the binary relation indicating that the first protein does the first interaction with the gene transcription factor which is a gene, b) the binary relation indicating that the above transcription factor does the second interaction with genes of probe, and c) the binary relation indicating that the above gene of probe does the third interaction with the above second protein, 4) the binary relation extracting means to extract binary relations for each protein name and gene name entered in reference to the binary relation storage area, and 5) the pathway map drawing means to draw the pathway map on the basis of the defined conditions entered by the binary relations and extracted by the binary relation extracting means and the defined conditions input means.

The above defined conditions input means of the Literature Information Processing System enters the information that limit the specific verb as the verb describing the binary relation.

The Literature Information Processing System defines the relation of subject-predicate of interactions between protein and gene names as a condition to limit the pathway map indicated. In addition, as a defined condition, this system enters the information to limit the specific verbs as verbs describing binary relations. Consequently, this system can draw pathway maps on the basis of protein and gene names that indicate the relation defined as a defined condition. Also, using verbs describing binary relations (for example, limiting "bind" or "interact") this system can indicate defined relations and draw the pathway maps that indicate only necessary information.

The Literature Information Processing System has the following characteristics: 1) the multi-body interactions storage area to store the binary relations that indicate the relationship between two element names and the multi-body interactions that indicate the relationship between more than three element names, 2) the input means to enter element names, 3) the multi-body interaction extracting means to extract the multi-body interactions for each multiple element name entered by the input means in reference to the multi-body interaction storage area, 4) the binary relation extracting means to extract the binary relations for each element name that have multi-body interactions with the entered element names in reference to the multi-body interaction storage area, and 5) the pathway map drawing means to draw the pathway map on the basis of the extracted multi-body interactions and the extracted binary relations.

The Literature Information Processing System's multi-body interaction extracting means extracts the multi-body interactions that indicate the relationship between 3,4,5,or 6 element names as the multi-body interactions.

The Literature Information Processing System extracts the multi-body interactions that indicate the relationship between at least three element names or more, and extracts the binary relations for each element name that have the multi-body interactions extracted to draw the pathway map. That is, the number of element names that have multi-body interactions indicating the relationship between more than three element names is generally less than that of the element name that indicates the multi-body interactions. For this reason the element names that have multi-body interactions indicating the relationships between more the three element names are extracted first, then the binary relations for the extracted element names are extracted, the exclusive objects can be analyzed cyclopaedically. In addition, the appropriate element names in range can be analyzed as objects by extracting the multi-body interactions indicating the relationship between 3, 4, 5 or 6 element names.

### BRIEF DESCRIPTION OF THE DRAWINGS

Figure 1 is the outline configuration diagram of the biomedical Literature Information Processing System,
Figure 2 is the outline configuration diagram of DNA microarray analysis device,
Figure 3 is the block diagram of DNA microarray analysis device
Figure 4 is the figure of process flow diagram for explaining and performing the experiments using DNA microarrays,
Figure 5 is the figure showing hierarchical clustering genes with Euclidean distance,
Figure 6 is the figure showing hierarchical cluster genes by Euclidean distance in the expression diagram,
Figure 7 is the list of genes whose activation of gene expression are recognized by DNA microarray analysis in the expression diagram,
Figure 8 is the list of genes whose activation of gene expression are recognized by DNA microarray analysis,
Figure 9 is the list of genes whose activation of gene expression are recognized by DNA microarray analysis,
Figure 10 is the interface that selects the probe IDs of up-regulation with threshold value of 1.3,
Figure 11 is the interface that selects the probe IDs of up-regulation with threshold value of 1.6,
Figure 12 is the interface that chooses the probe IDs for the pathway analysis,
Figure 13 is the interface that chooses the probe IDs for the pathway analysis,
Figure 14 is the interface that chooses the probe IDs for the pathway analysis,
Figure 15 is the interface that chooses the probe IDs in the intersection for the pathway analysis,
Figure 16 is the interface that chooses the probe IDs for the pathway analysis,
Figure 17 is the flow chart to explain the drawing on the pathway map,
Figure 18 is the figure to explain binary relations
Figure 19 is the figure to explain the drawing of the pathway map,
Figure 20 is the figure to explain the drawing of the pathway map,
Figure 21 is the pathway map drawn by the Literature Information Processing System,
Figure 22 is the pathway map drawn by the Literature Information Processing System,
Figure 23 is the pathway map drawn by the Literature Information Processing System,
Figure 24 is the pathway map drawn by the Literature Information Processing System,
Figure 25 is the pathway map drawn by the Literature Information Processing System,
Figure 26 is the pathway map drawn by the Literature Information Processing System,
Figure 27 is the pathway map drawn by the Literature Information Processing System,
Figure 28 is the pathway map drawn by the Literature Information Processing System,
Figure 29 is the pathway map drawn by the Literature Information Processing System,
Figure 30 is the figure indicating the binary relation shown by the Literature Information Processing System,
Figure 31 is the flow chart for explaining the example of variation of pathway drawing,
Figure 32 is the flow chart for explaining the example of variation of pathway drawing of the first implementation of this invention,
Figure 33 is the flow chart for explaining the pathway map drawing of the third implementation of this invention,
Figure 34 is the flow chart for explaining the pathway map drawing of the form of the forth implementation of this invention,
Figure 35 is the flow chart for explaining the pathway map drawing of the fifth implementation of this invention,
Figure 36 is the node and edge graph of binary relation network of the fifth implementation of this invention,
Figure 37 is the node and edge graph of binary relation network of the fifth implementation of this invention,
Figure 38 is the node and edge graph of binary relation network of the fifth implementation of this invention,
Figure 39 is the table for the parameters of the binary relation network of the fifth implementation of this invention,
Figure 40 is the flow chart for explaining the pathway map drawing of the fifth implementation of this invention,
Figure 41 is the flow chart for explaining the pathway map drawing of the sixth implementation of this invention,
Figure 42 is the list for indicating the relation between the probe ID, protein names, and gene names of the implementation of this invention,
Figure 43 is the flow chart for explaining the pathway map drawing of the seventh implementation of this invention,
Figure 44 is the figure for explaining the pathway map drawing of the seventh implementation of this invention,
Figure 45 is the figure for explaining the pathway map drawing of the seventh implementation of this invention,
Figure 46 is the figure for explaining the pathway map drawing of the seventh implementation of this invention,
Figure 47 is this figure shows an example of pathway map of the seventh implementation of this invention,
Figure 48 is this figure shows other examples of pathway map drawing of the seventh implementation of this invention,
Figure 49 is this figure shows other examples of pathway map drawing of the seventh implementation of this invention,
Figure 50 is this figure shows other examples of pathway map drawing of the seventh implementation of this invention,
Figure 51 is this figure shows the relations of node and edge of the seventh implementation of this invention,
Figure 52 is this figure shows the relations of node and edge of the seventh implementation of this invention,
Figure 53 is this figure shows the relations of node and edge of the seventh implementation of this invention,
Figure 54 is the outline configuration diagram of the Biomedical Literature Information Processing System of the implementation of this invention,
Figure 55 is the flow chart to explain the pathway map drawing of the eighth implementation of this invention,
Figure 56 is the figure to explain the pathway map drawing of the eighth implementation of this invention,
Figure 57 is the figure to explain the pathway map drawing of the eighth implementation of this invention,
Figure 58 is the schematic diagram to explain the pathway map drawing of the eighth implementation of this invention,
Figure 59 is the figure to indicate one example of the pathway map of the eighth implementation of this invention,
Figure 60 is the figure to indicate one example of the mesh term of the eighth implementation of this invention,
Figure 61 is the figure to indicate other examples of the pathway map drawing of the eighth implementation of this invention
Figure 62 is the outline configuration diagram of the Biomedical Literature Information Processing System of the implementation of this invention,
Figure 63 is the figure to indicate one example of the specific element name of the implementation of this invention,
Figure 64 is the figure to explain the display change of the pathway map of the implementation of this invention,

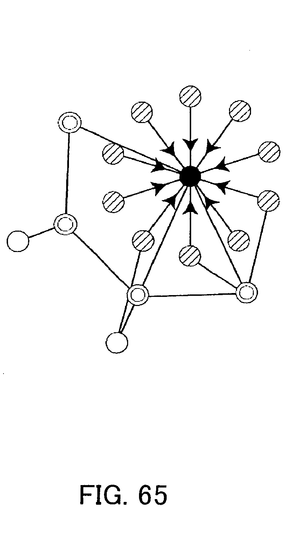

Figure 65 is the figure to explain the display change of the pathway map of the implementation of this invention,
Figure 66 is the figure to explain the display change of the pathway map of the implementation of this invention,
Figure 67 is the figure to indicate other examples of the supplementary information of the implementation of this invention,
Figure 68 is the figure to indicate one example of the pathway map indicating the relationship between the element names of the implementation of this invention,
Figure 69 is the figure to indicate one example of the pathway map where the node with a specific function is divided into clusters,
Figure 70 is the figure to indicate one example of the pathway map that makes the pathway of each type of cell species in the implementation of this invention identifiable,
Figure 71 is the outline configuration diagram of the Biomedical Literature Information Processing System of the implementation of this invention,
Figure 72 is the figure to indicate other examples of the supplementary information of the implementation of this invention,
Figure 73 is the figure to indicate other examples of the pathway map that the prescribed element names of the implementation of this invention are displayed using prescribed figures,
Figure 74 is the figure to indicate one example of the pathway map that display the material names that have a relation with the interaction between the element names of the implementation of this invention,
Figure 75 is the figure to indicate other examples of the pathway map that display the material names that have a relation with the interaction between the element names of the implementation of this invention,
Figure 76 is the figure to indicate one example of the interaction between the element names of the implementation of this invention,
Figure 77 is the figure to indicate the abbreviation of the indirect interactions and nodes between the distant element names of the implementation of this invention,
Figure 78 is the figure to indicate other examples of the pathway map of the implementation of this invention,
Figure 79 is the figure to indicate other examples of the pathway map of the implementation of this invention,
Figure 80 is the figure to explain the corresponding relationship between the domain structures of the implementation of this invention,
Figure 81 is the figure to indicate one example of the interactions between the element names of the implementation of this invention,
Figure 82 is the figure to indicate other examples of the interactions between the element names of the implementation of this invention,
Figure 83 is the figure to indicate other examples of the pathway map of the implementation of this invention,
Figure 84 is the outline configuration diagram of the Biomedical Literature Information Processing System of the ninth implementation of this invention,
Figure 85 is the figure to indicate one example of the representation the probe expression of the ninth implementation of this invention,
Figure 86 is the flow chart to explain the processing of the biomedical literature of the information processing system of the ninth implementation of this invention,
Figure 87 is the figure to indicate other examples of the pathway map of the implementation of this invention,
Figure 88 is the figure to indicate the specific pathway map of organization A of the implementation of this invention
Figure 89 is the figure to indicate the specific pathway map of organ B of the implementation of this invention, and
Figure 90 is the figure to indicate the specific pathway map of organ C of the implementation of this invention.

### BEST MODE FOR WORKING THE INVENTION

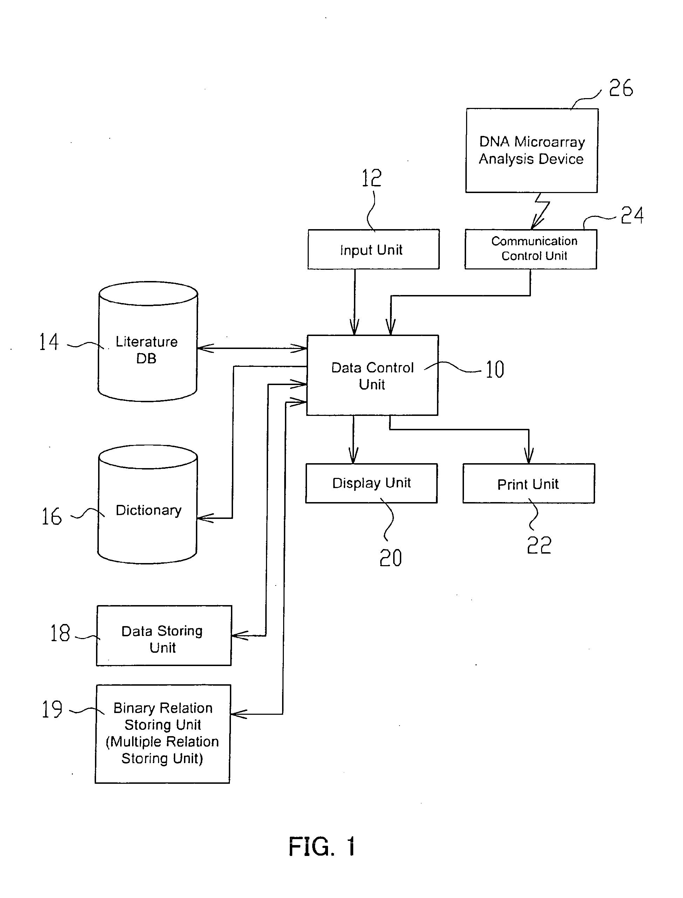

And below, we will explain the Biomedical Literature Information Processing System of the implementation of this invention in reference to the drawings. Figure 1 indicates the configuration diagram of the Biomedical Literature Information Processing System of the first implementation of this invention. This Biomedical Literature Information Processing System has a Data Control Unit 10 that controls the data processing of the Biomedical Literature Information Processing System. This Data Control Unit 10 is plugged into Data Input Unit 12 composed of keyboard and the files. Using the input part 12, element names (protein names, gene names, etc.) are entered and the supplementary information that is necessary to draw pathway maps is entered into the system.

Data Control Unit 10 is plugged into Literature (Database) DB14, Dictionary 16, Data Storage Unit, and Binary Relation Storage Unit (also Multiple Relation Storage Unit) 19. Literature DB14 stores the information of the literature in the medline database that is a public database for the biomedical literature information.

Dictionary 16 stores protein names, gene names (including abbreviated those names), noun phrases, and adjective phrases and the expression that have effects similar to verbs. As protein names, the official names of protein names and the synonyms are stored. That is, there are a large number of synonyms in protein names, and the styles of expression are different depending on the authors of the articles. The variations of synonyms are: 1) modifications of abbreviation, and capital or small letters, 2) Synonyms whose names indicate the roles (When only the same functions are explained, there may be various ways of expressions) and 3) synonyms including preposition and conjunction (modification relation is more complicated) .

The official names of genes and the synonyms are stored as well as the verbs indicating the interactions between proteins as well as genes. The noun phrases, and adjective phrases, and expressions that have similar to these representing the meaning of verbs are also stored. These terms and phrases are stored in Dictionary 16 (the terms are collected by means of analyzing literature information stored in public databases by human or computers). Data Storage Unit 18 stores the element names (protein names, gene names, etc) entered from input part and the element names (protein names, gene names, etc) of the experimental result transmitted from DNA microarray analysis device 26. Binary Relation Storage Unit 19 stores the data of the binary relation extracted by this Biomedical Literature Information Processing System.

Data Control Unit 10 is plugged into Display Unit 20 and Print Unit 22. Display Unit 20 displays entry screens to enter element names and binary relations pathway maps drawn. Print Unit 22 prints pathway maps drawn.

Additionally, Data Control Unit 10 is plugged into Communication Control Unit 24, and received the information of element names or probe names based on the detection result of DNA microarray analysis device 26. Communication Control Unit 24 functions as a detection result input unit.

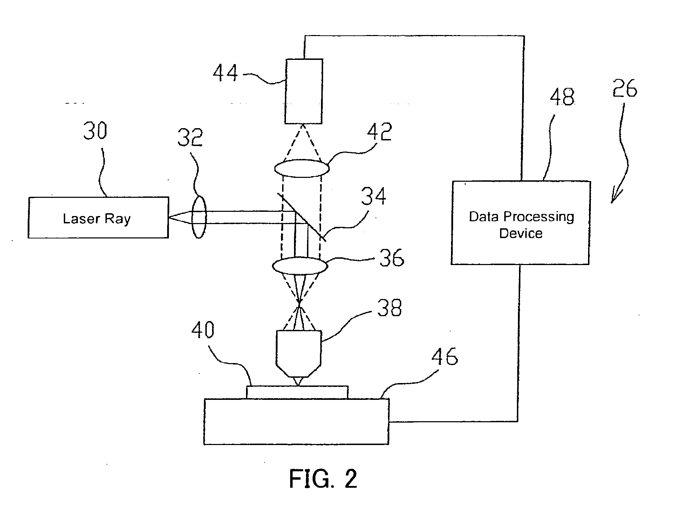

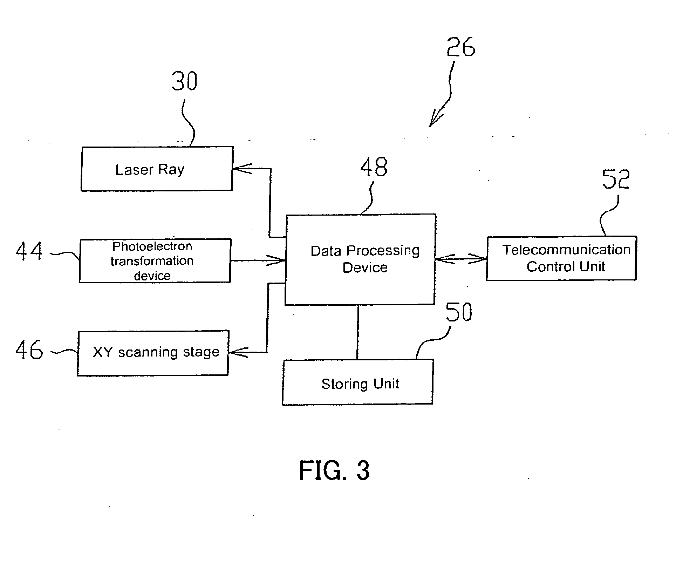

Figure 2 is the outline configuration diagram of DNA microarray analysis device, and Figure 3 is the block diagram of DNA microarray analysis device. The DNA microarray analysis device is organized with a Scanning Optical Measuring Device. The laser launched from Laser Light Source 30 is a parallel beam of light by collimator lens 32, and enters into dichroic mirror 34. The beam of light reflected by dichroic mirror 34 irradiates the top of DNA microarray 40 via lens 39 or objective lens 38. The fluorescence generated by the irradiation of this laser passes a confocal pinhole via objective lens 38, lens 36, dichroic mirror 34, or lens 42, and is led to photoelectric conversion element 44 such as photoelectron multipliable tube (PMT), and then the fluorescence intensity is converted to electronic signal by photoelectric conversion element 44.

At this time, DNA microarray 40 is set on scanning XY stage 46, and transferred to XY direction. For this reason, DNA microarray 40 is scanned to XY direction by the laser launched from Laser Light Source 30, and the electronic signal output from conversion element 44 on the basis of the irradiation of the laser. Process Device 48 converts the electronic signal from conversion element 44 to A/D, and gets it as a scanning image data.

The scanning image data obtained like this is saved as a general-purpose image data such as a Bit Map format to Data Storage Unit 50 once, then read out by the dedicated analysis software and date is processed according to the request from the user to identify the expressed probes, here probes are fragments of DNAs. We can then acquire a probe ID that is an identifier of a DNA fragment (a part of DNA on DNA microarray that generated DNA is located), generated DNA name, and analysis data such as protein names that have the interaction with generated DNA. These analyzed data are stored in Storage Unit 50, and transferred to Data Control Unit 10 via Communication Control Unit 52 and Communication Control Unit 24.

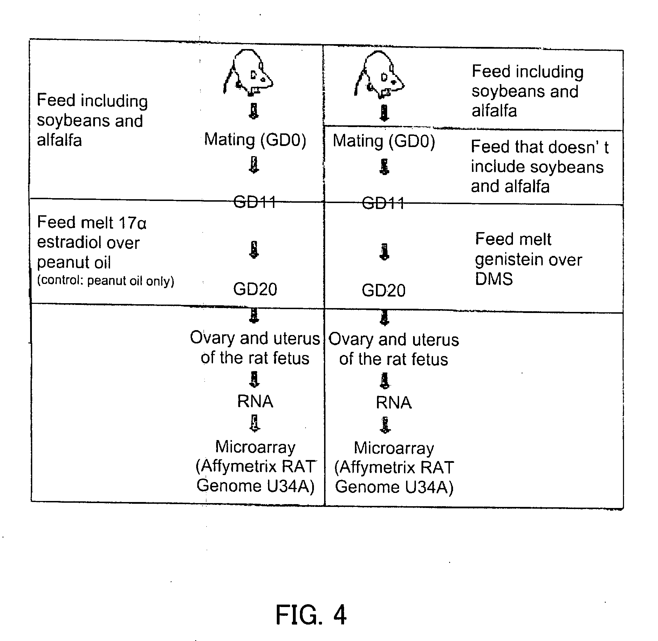

Next, we would like to explain using the microarray experimental data, supposing it is performed by DNA microarray analysis device 26. Figure 4 shows the experimental procedures of Naciff et al. (Naciff J. M, et.al., Toxicol.Sci., 68, 184-199, (2002)), who conducted microarray analysis for the rat experiment described below.

In the experiment, they first gave soybeans and fed feed including alfalfa to 4 female rats (includes Genestine).

Next, at ovulation dates, they mated the female rats with a male rat, and this day counts as the 0 day. After mating, they changed the feed for two of four rats not to include soybeans and alfalfa.

Next, at the 11th day of fertilization (GD11), for the two rats those were fed with soybeans, they gave 17α estradiol melted into peanut oil including once a day for one of the two rats, and for the other rat, they gave peanut oil only as a control. For the other two rats those were fed not to include soybeans among four, they gave the feed with genistein melt over DMSO once a day for one of the two rats, and for the other they gave only DMSO as a control.

Next, at the 20th day of fertilization, they took out the ovary and uterus of the rat fetus to extract RNA, and performed microarray analysis using Rat genome U34A chip of Affymetrix company.

Supposing the result of this microarray analysis is obtained in our system, the result of this microarray analysis should be transmitted to Data Control Unit 10 of the Biomedical Literature Information Processing System via Communication Control Unit 52 of DNA microarray analysis device 26, and stored in Data Storage Unit 18.

The microarray analysis device to analyze usual gene expression, in image scanning device of microarray analysis device, recognizes probe partitions to calculate the signal intensity, and deducts the signal intensity of the background including noises to monitor the signal. Furthermore, the device maps the statistics model of probe expression to find outlier values, and determines the method to obtain the average amount to gain the reliable estimate value. In the example of the Affymetrix company, you can see the protocol to handle the data: http:www.affymetrix.com/support/technical/technotes/statica 1_reference_guide.pdf

To compare two different micaroarray experiments, for example, by monitoring the house keeping gene expressions whose expression is necessary to maintain fundamental function, or structure of a cell whose representations are always considered to be constant using microarray, we perform scaling the results with different experiments by assuming that all amounts of RNA are constant. The expression values of all gene are multiplied by a factor to keep constant values for the house keeping genes in different experiments, thus we can reduce the difference of experimental conditions affecting the expression values. The difference of the expression values usually called fold change since it means of the change of multiplication because the change of expression is relative between different experiments. We can recognize that a gene is up regulated or down regulated, or not changed by the value of fold change from the microarray analysis. Therefore, we must choose the threshold value by which we decide whether the value of fold change is caused by noise or not. If the value of fold change of the expression of a probe exceeds a certain threshold value and higher (lower), we recognize that the gene represented by the probe is up regulated (down regulated) and meaningful, not just noises of the experiments. Actually, it sometimes causes misunderstanding without referring to whether the threshold change is up-regulation or down-regulation. Therefore, we must examine that the change is up or down regulation or not changed by mathematical algorithm such as t-test, ANOVA, those are already developed and well used. The details of these are well documented in "Guide to Analysis of DNA Microarray Data" Steen Knudsen (John-Wily and Sons, 2002)

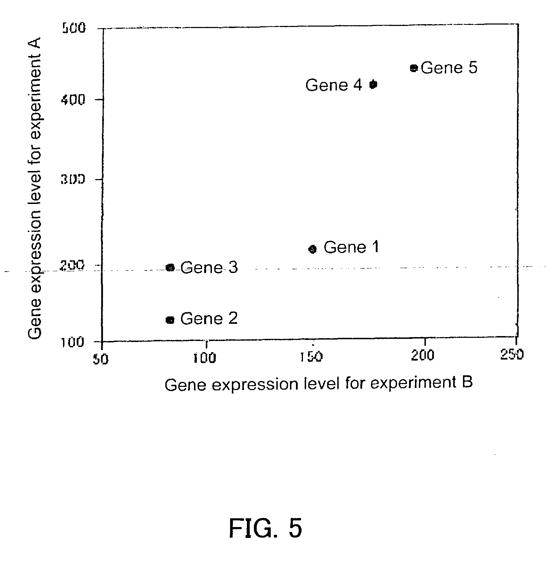

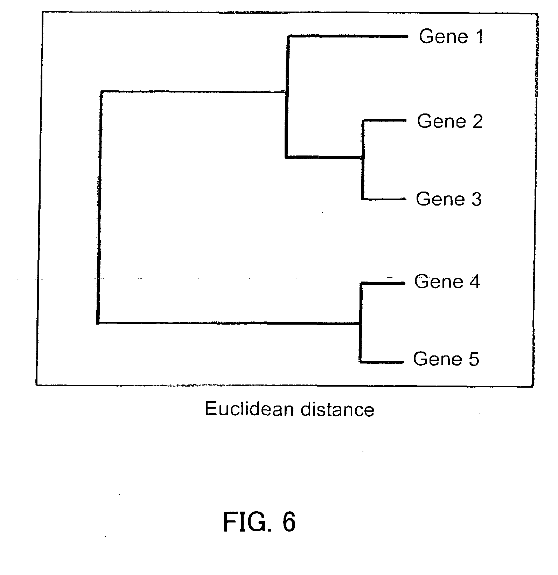

It turns out that the analysis result of microarray is to show a set of up regulating genes or that of down regulating genes. In a comparison of data between many experiments, the clustering that hypothesizes the virtual distance to each gene such as hierarchical type clustering function and categorizes genes is used. For example, Figures 5 and 6 shows the results of hierarchical type clustering genes in the Euclidean space. Figure 5 is of gene 1-5 and is plotted to the expression of different experiment as an axis. From Figure 5, we can recognize that gene 1 - gene 3 are gathered at a short distance as spatial arrangement and gene 4 -gene 5 are gathered as a cluster at a distance. Figure shows the result of hierarchical structure from the distance between genes when putting in genes to Euclidean space that hypothesizes a coordinate as uniformity. The system visually makes it easy to understand the gene clustering by connecting it to gene clustering.

In most experiments, when adding disturbances such as heat, stream, stress, medicine, and chemical reaction, we observe the differences between the static states, and trangent or perturbed states of normal cells and of disease sample cells (or cells of knock out mouse). Thus, microarray data are four types of data: 1) static-normal, 2) static-disease, 3) perturbed -normal, and 4) perturbed-disease state.

In the different types of microarray, which is called genome array, the variants DNA sequence, such as SNPs (Single Nucleotide Polymorphisms) of humans are detected from the DNA probes of microarray that aligns of fragments of genome sequence. We can detect changes of copy numbers of genes from this microarray. We can detect the estimated copy numbers of gene expressions by change of copy numbers from the microarray, and deduct the value from the expression value obtained by an expression experiment of gene expression microarray, then we evaluate the net values of expressions of genes, leading to the network analysis of gene expressions with those information. In these analyses, it is expected that the DNA region that normally should have a function may lose function as a consequence of the removing movement of the portion of the DNA region that contains some genes or promoter regions, or vice versa, DNA region may have additional function as a consequence of the adding movement of some portion of the DNA region to the original DNA region. This invention makes it easy to analyze the responsible parts, which make the change of the function of genes by comparing the pathway obtained by this invention for the gene expression results of normal sample and pathway thus obtained for the gene expression results for the samples with specific DNA movements.

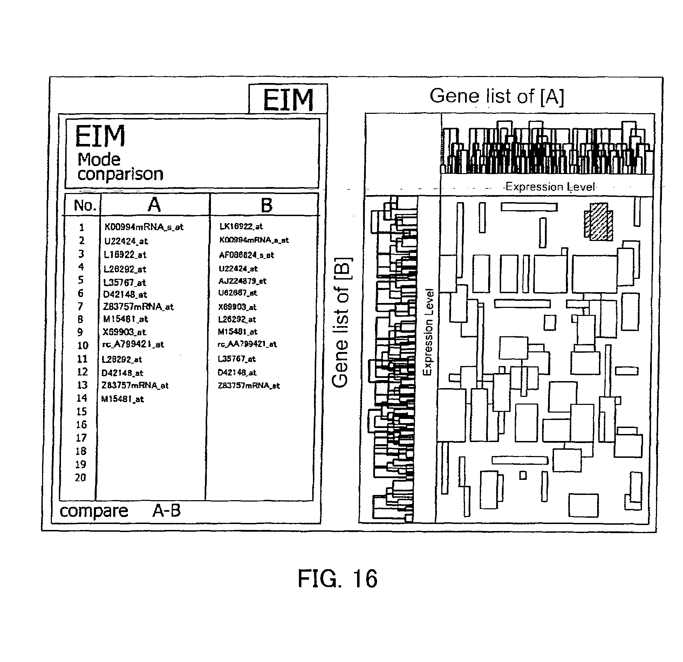

It takes much time to analysis all probe data directly in the experiments, and the purpose of analysis is not clear, but there might be misunderstanding leading to cause severe errors. To avoid this, in this invention, we describe the result of two expressions clustering near each other to vertical axis and horizontal axis, and compare the variation of expression value at the point of genome by using hypergeometric distribution, and use EIM method (literature: Kano et al., Physiol. Genomics 10, 1152(2003)) that classify the regions genomes according to the levels of expression value. Figure 16 shows the clustering results obtained by EIM for gene expression experiments in which no change may occur in the copy number of genome between experiment A under stimulation of a medicine and experiment B which is not under stimulation of a medicine. Shaded area in Figure 16 shows the common part of up regulated part of expression of experiment A and experiment B. Both expression values are shown to be high at each axis of the region surrounded by the shaded area on Figure 16. On the other hand, when the movement of genome are involved and the copy number is changing, if in the samples of experiment A the copy number is changed and in the experiment B no such change is involved, changes of genome and the relationship between expression values for the copy number of genome can be monitored as shaded area in Figure 16. With combining the EIM calculation, our invention system can extract list of genes, extract gene clusters easier, and can see the effect of the genome changes upon the pathways.

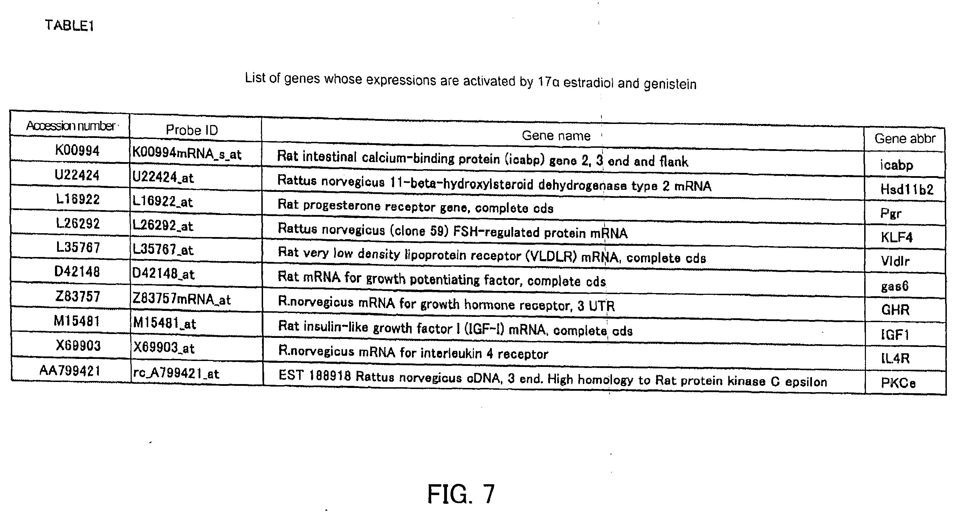

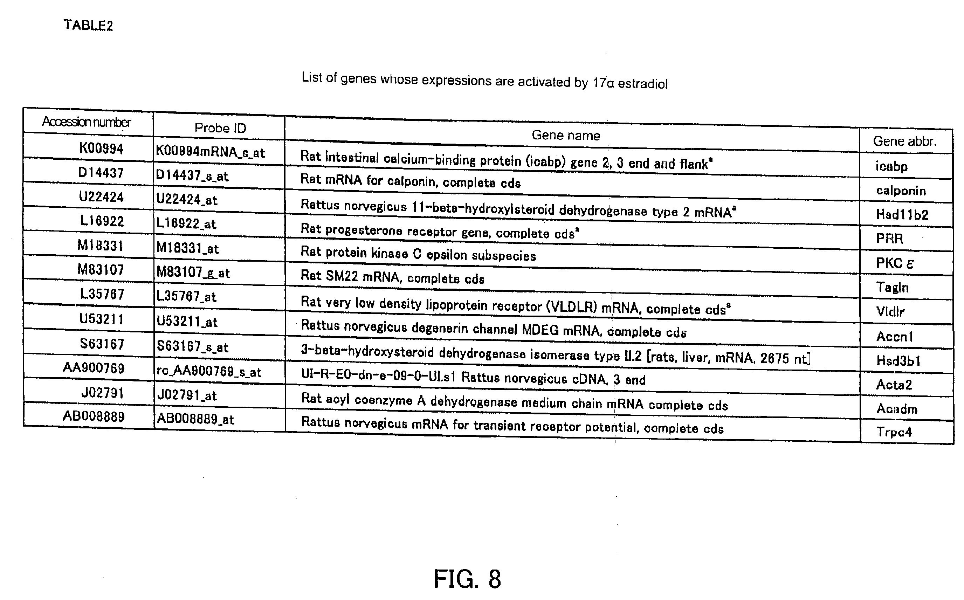

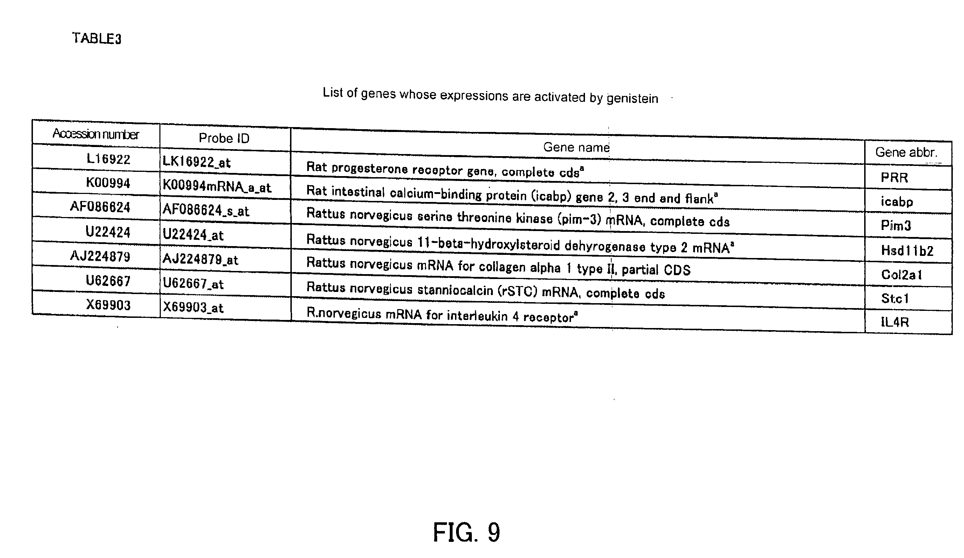

Figure 7-9 indicates the results of the microarray analysis above. Figure 7 is the list of genes whose expressions are up regulated by 17α estradiol and genistein (the result of experiment 1) . Figure 8 is the list of genes whose expressions are up regulated by 17α estradiol (the result of experiment 2). Figure 9 is the list of genes whose expressions are up regulated by genistein (the result of experiment 3). These lists indicate action numbers, probe ID, gene names, and abbreviated gene names from the left. In it is possible to use these probe ID, gene names, and abbreviated gene names for searching.

The results of experiment 1 - 3 are transmitted from the DNA microarray analysis device to the Biomedical Literature Information Processing System, and entered into the system via Communication Control Unit 24. In addition, the result of experiment 1 -3 can be entered with Input Unit 12.

In the Display Unit, the user interface (not shown) are composed of following parts: 1) a part to select data from the part showing the location of data, 2) a part to indicate date, medical status, conditions, and organism species of experimented data, 3) a part to indicate the relation between group of probe ID and expression value, and 4) a part that indicates thresholds and displays up regulations, down regulations, and even the common and uncommon gene lists of different experimental data.

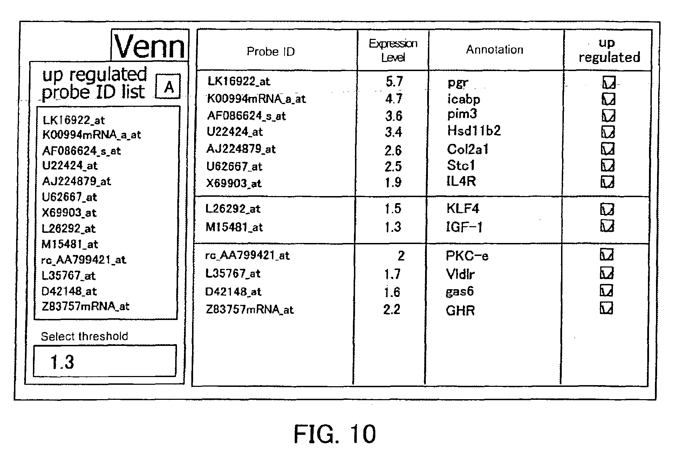

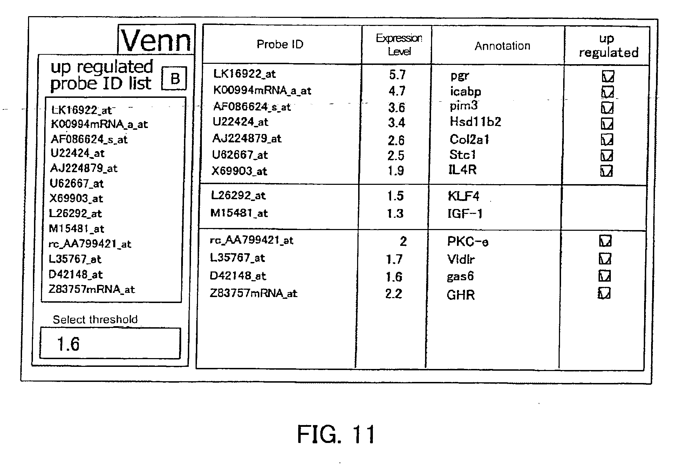

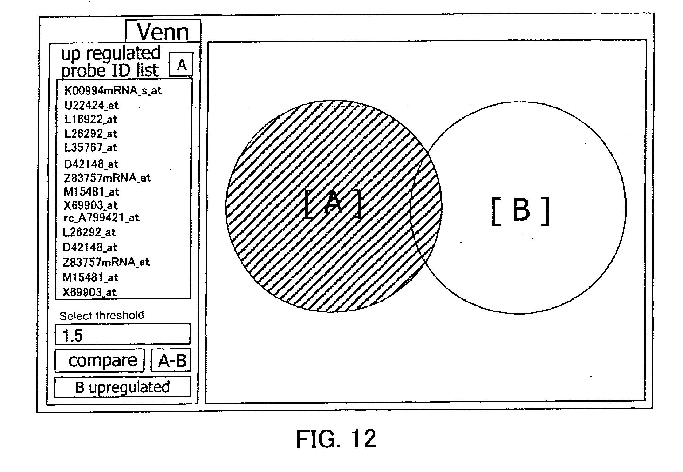

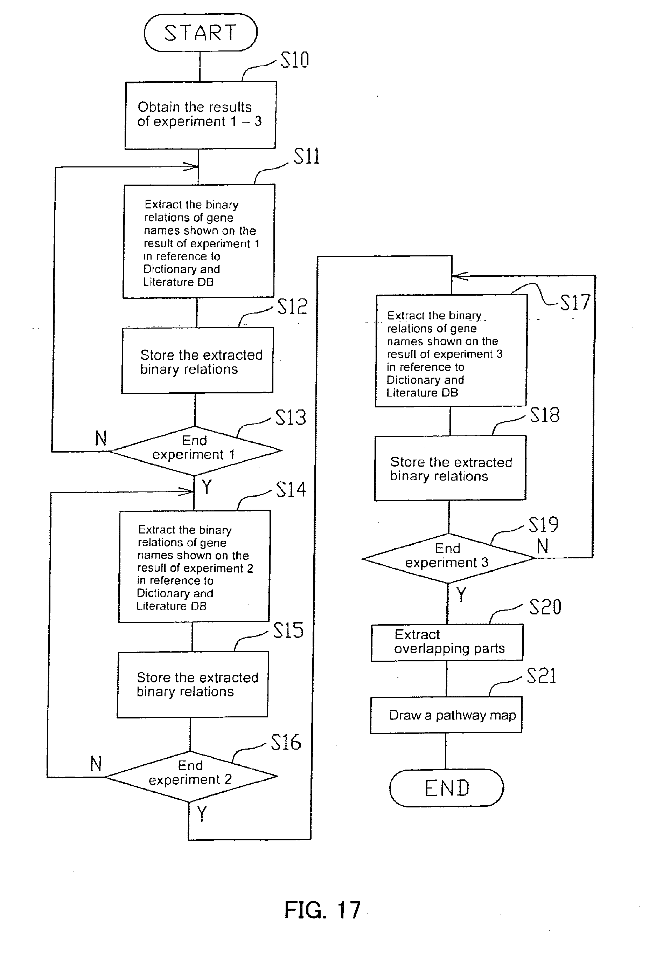

Figure 10 and Figure 11 show an example of probe ID of up regulations that are changed by selecting the threshold in reference to the example of Naciff's experiment. Figure 10 shows the example of probe IDs of KLF4 and IGF-1 (proteins) that are selected when the threshold value is 1.3. And Figure 11 indicates that probe IDs of KLF4 and IGF-1 are not selected when the threshold value is 1.6 (The example of Naciff' s experiment is the value of reference). Figure 12 - 15 indicates the interface of selecting the part such as unions of sets, intersections, and exclusive OR in the up regulation parts between different experiments. In addition, we can draw various pathway maps in the pathway map drawing described below (refer to step S21 of Figure 17) by the use of this interface.

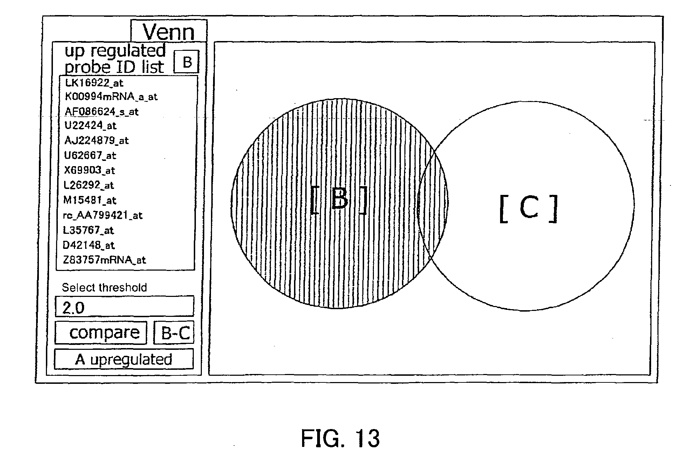

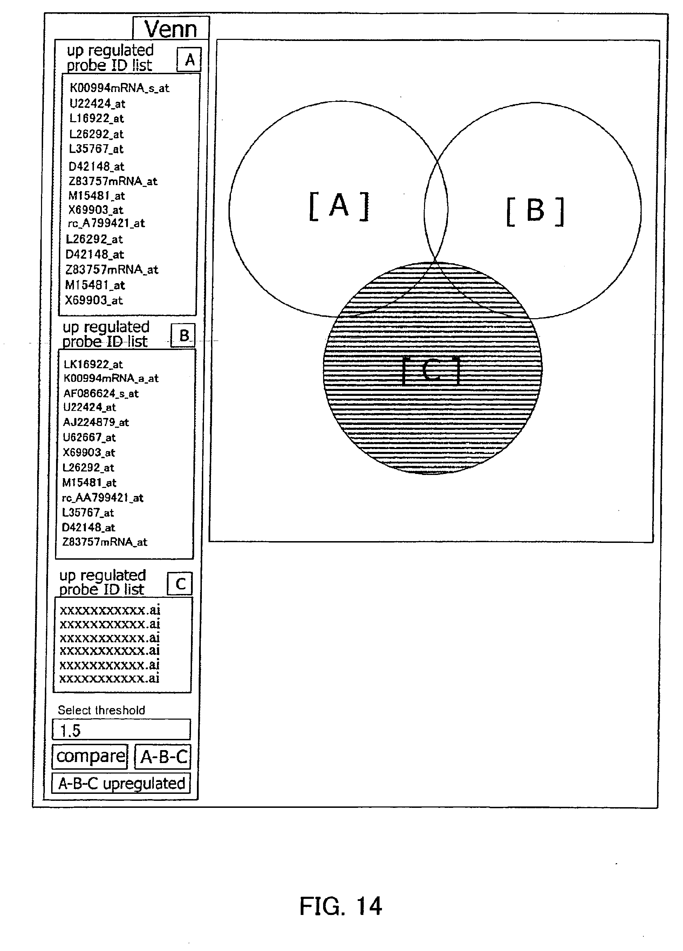

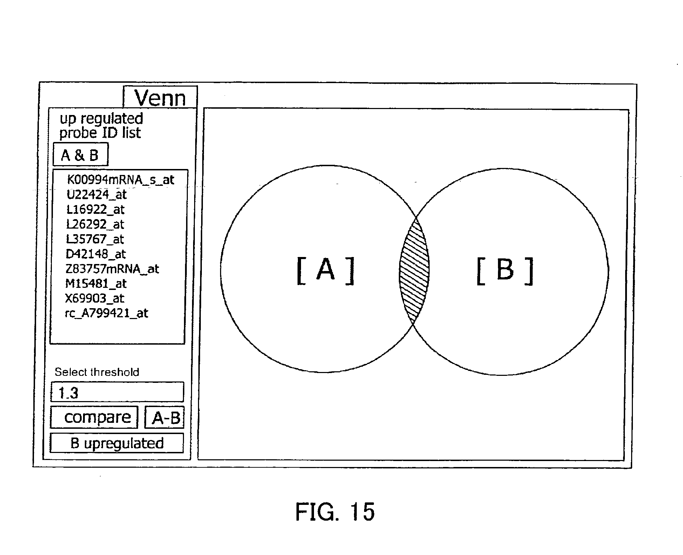

In addition, Figure 12 indicates the interface that selects probe ID groups of up regulations in the list of experiment A to compare with those in the list of experiment B. Figure 13 indicates the interface that selects probe ID groups of up regulations in the list of experiment B to compare with those in the list of experiment C. Figure 14 shows the interface that selects probe ID groups of up regulations in the experiment A, B, C to compare between each experiment. Figure 15 shows the intersection of probe ID groups of up regulation in the list of experiment A, B. Figure 16 shows the interface that obtained from the clustering analysis in the list of experiment A, B, and those obtained from EIM analysis, and extracts the specific region from the intersections among them to select probe IDs for pathway analysis.

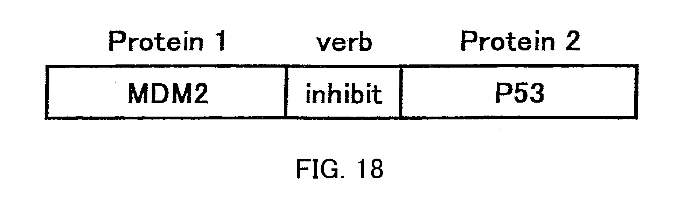

Figure 17 is the flow chart to explain the extraction of binary relations and the process of pathway map drawing on the Biomedical Literature Information Processing System. Here, the extraction of binary relation functions means, as shown Figure 18, extracting the binary relations between gene names and protein names indicated as "noun A (gene name)", "verb", and "noun B (gene name) " with use of natural language processing. In addition, the examples of the verbs indicating the interaction between gene names (and protein names) are as follows: "bind", "inhibit", "interact", "phosphorylate", "mediate", "modulate", "induce", "associate", etc. Here we gave examples of verbs for the sake of simplicity, but it is true in the case of others such as noun phrases and adjective phrases: "the interaction between A and B" and " interaction with".

The Data Control Unit 10 of the Biomedical Literature Information Processing System stores the results of experiment 1 - 3 received from Communication Control Unit 24 on Data Storage Unit 18 (step S10). The results of experiment 1 - 3 are gene name groups selected to set the threshold of gene expression level as discussed previously.

Next, we extract mutual binary relations of gene names and protein names in reference to Dictionary 16 and Literature DB14 for the gene names indicated in the result of experiment 1 (stepS11). That is, we extract the binary relations between gene names and protein names indicated as "noun A (gene name)", "verb", and "noun B (gene name)" using natural language processing for the first name of gene names shown in the result of experiment 1.

And for "noun B (gene name) " extracted as having binary relation with "noun A (gene name)", we also extract the mutual binary relations of gene names and protein names indicated as "noun B (gene name)", "verb", and "noun C (gene name)". That is, we extract the binary relation of the gene name extracted as having a binary relation with the gene name input as an experimental result. This binary interaction extraction or search is performed in our system in the predetermined range (the range of predetermined hierarchy), for example, the range from the entered gene name, for example, up to the third hierarchy, or to the extraction of gene names up to those which directly involve functions.

The extracted binary relations are stored in Binary Relation Storage Unit 19 (Step S12). Next, the system evaluates whether the extractions of binary relations for all the gene names shown on the result of experiment 1 are finished or not (Step S13). In case that the extractions are decided not to be finished, the system goes back to Step S11 to extract binary relations of next gene names.

In Step S13, if the extractions of the binary relations for all the gene names shown on the result of experiment 1 are deemed to be finished, we extract the binary relations of gene names shown on the result of experiment 2 in reference to Dictionary 16 and Literature DB14 (Step S14) to store the extracted binary relations in Binary Relation Storage Unit 19 (Step S15). Here, the process of extracting binary relations in Step S14 is the same as the process of extracting binary relations in Step S11.

If the extractions of the binary relations for all the gene names shown on the result of experiment 2 are finished (Step S16), we extract the mutual binary relations of gene/protein names shown on the result of experiment 3 in reference to Dictionary 16 and Literature DB14 (Step S17) to store the extracted binary relations in Binary Relation Storage Unit 19 (Step S18). Here, the process of extracting binary relations in Step S17 is the same as the process of extracting binary relations in Step S11.

If the extractions or searching of the binary relations for all the gene names appeared in the result of experiment 3 are finished (Step S19), we detect the overlapping parts for the binary relations stored in Binary Relation Storage Unit 19 (Step S20) . That is, the some of the binary relations extracted for the gene names shown in the results of the experiments are redundantly counted because each experimental result includes the same gene names. Consequently, in case overlapping parts are found and removed, the pathway map is drawn regarding the overlapped binary relations as one unit of information (Step 21).

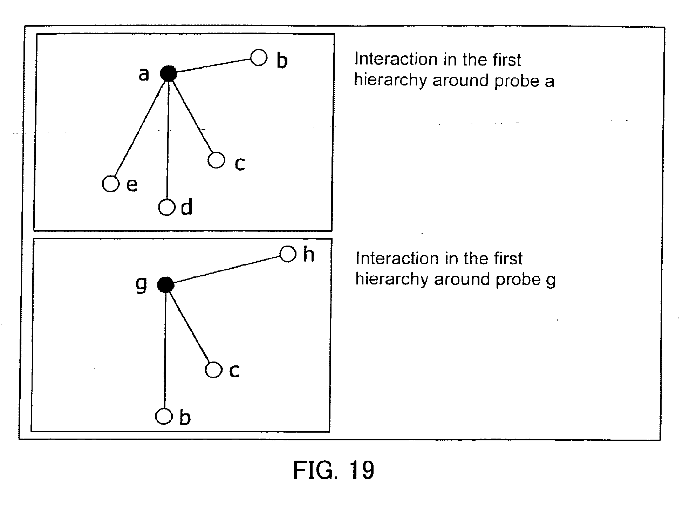

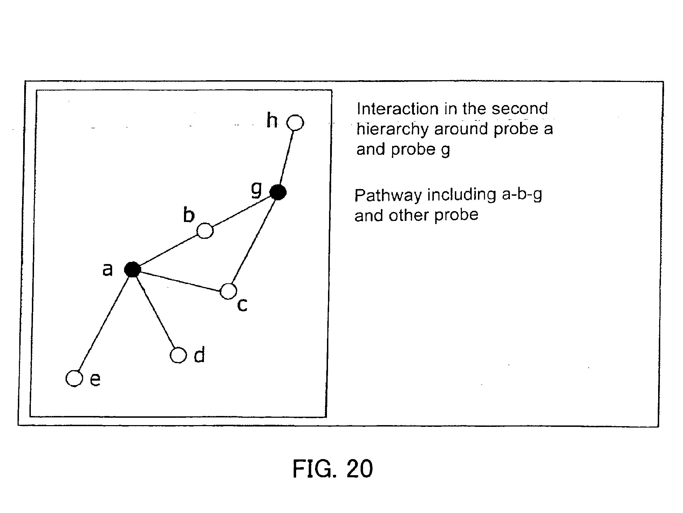

Here we explain how effective our data analysis on the microarray analysis: assuming that we have probe information of two up-regulated gene lists for microarray, and considering the case where in drawing interaction relationships with simple method. For probe 'a', for example, the interaction relations between probe 'a' and proteins are searched just one time, the interaction relations between the proteins of probe a and other proteins (the first interaction around probe 'a') will be g-h, g-c-a, and g-b-a as shown on Figure 19. Furthermore, the interaction relations between the proteins of probe 'g' and other proteins (the first interaction around probe g) will be g-h, g-c, g-b. In such a case, there exists no intersection in the pathways in the map. If the search is performed recursively more than two times, as shown on Figure 20, we can obtain interaction relations as a-b-c, a-c-g, a-d, a-e...or g-h, g-c-a, or g-b-a, ... (the secondary searching interaction partners around probe 'a' and 'g'). Consequently, we can find the intersection in the pathways in the map. For extracting effectively pathway maps in parallel, we have to generate, to some extent, wider region of connected network for drawing pathway map than the region of search. As explained below, our system can generate well-connected pathway with using any of the following ways or some combinations.

(1) Union of different pathways is always taken to generate in combining pathways. (2) Some sets of pathways are stored previously as many templates of pathways so that if one of genes (or proteins) or an interaction is obtained, then a set of group of sequential pathways can automatically generate. (3) Performing recursively search for an input set of obtained partner proteins (or genes) as searched results through the system for the previous input proteins (or genes). Thus the region of intersections of the networks for different input sets of probes (or proteins) increase. Our systems can provide the recursively-generated network plenty of times. However in the real implementation, the region of the recursively-generated network becomes too large if we recursively generate network so many times, therefore we need some restrictions on the region or the number of recursive search. To remove the multiple counts in the intersection, we can remove it as a graph theoretical homology search of at least two of networks with identifying names of the nodes under consideration. (4) The further branches of edges of node in the pathways for proteins are predicted stochastically and statistically by generating network by Monte Carlo method or Bayesian network. (5) The pathways for proteins (or genes) are statistically predicted with use of the motif patterns for them in the database. Using the method described in (1) to (5) and their combinations, we can generate possible network for the nodes in the restricted region in our system, and we can provide some portions of the possible network as user input or the instruction from outside of system.

In addition to previous information, supplementary information (for example, gene names or modes of action of 17 α estradiol, gene names or mode of action of genestein, etc.) are input using Input Unit 12 to draw a pathway map.

A pathway map is drawn using the supplementary information entered by Input Unit 12 and binary relations stored in Binary Relation Storage Unit 19. First, 17α estradiol and gene names that 17α estradiol acts are represented as nodes. Then 17α estradiol and gene names that 17α estradiol acts are linked by edges. Next, gene names that 17α estradiol acts and gene names of interaction partners having binary relations with those are derived from the system are represented as nodes. Then gene names that 17 α estradiol acts and gene names of interaction partners having binary relations with those are derived from the system are linked by edges.

On the other hand, genistein and gene names that genistein acts are represented as nodes. Then genistein and gene names that genistein acts are linked by edges. Next, gene names that genistein acts and gene names of interaction partners having binary relations with those are derived from the system are represented as nodes. Then gene names that genistein acts and gene names of interaction partners having binary relations with those are derived from the system are linked by edges. Here, the shapes of the edges that connect gene names to gene names are provided for each interaction verb that indicates an interaction between genes. The attribute of edge corresponded to "bind" is defined as "-", the attribute of edge corresponded to "inhibit" is defined as "⊥", and the attributes of edges corresponded to other verbs are defined as "→". Consequently, by using edges of these defined attributes, connections between gene names are linked on the basis of verbs in the binary relations. As just described, regarding gene names as nodes, pathway maps of all the binary relations stored in Binary Relation Storage Unit 19 are drawn by linking gene names having binary relations with these genes by edges.

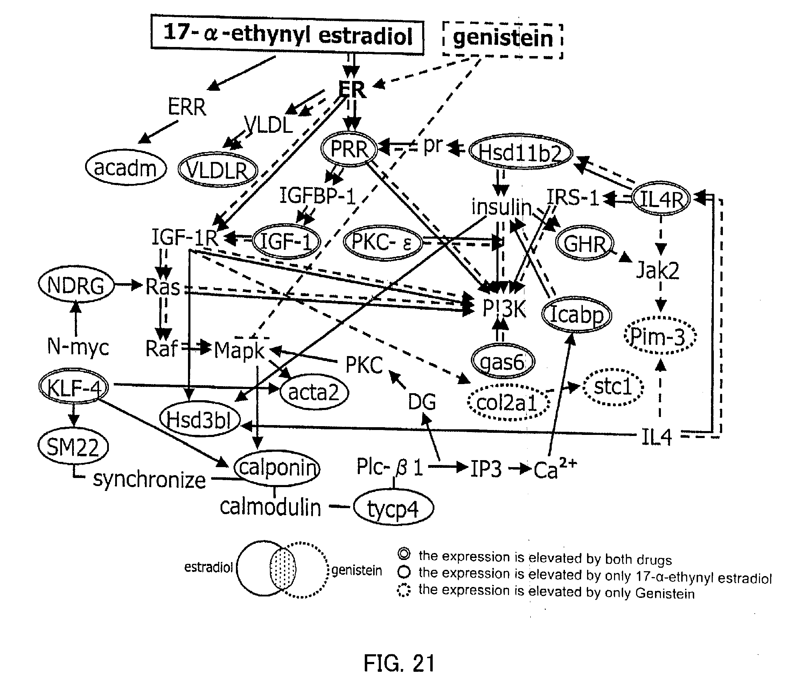

Furthermore, we can select gene names for drawing in a pathway map from gene names stored in Data Storage Unit 18 in Biomedical Literature Information Processing System concerning this embodiment. Consequently, the system can display as follows: 1) all the gene names based on each experiment as gene names drawn on a pathway map, 2) intersections of element names based on each experiment as gene names drawn on a pathway map, and 3) differences (exclusive OR) of element names based on each experiment as gene names drawn on a pathway map. That is, the system can draw pathway maps shown on Figure 21 - 29. Here, selection of gene names showing on a pathway map is done by inputting experiment names or assortments of experiment names from Input Unit 12. In addition, we can also select gene names by using the above input interface, the input interface shown on Figure12 - 16. Consequently, the system can sequentially change pathway maps shown in Figure 21 -29 by entering experiment names to display and assortments of gene names with Input means 12.

The system can discriminate and show those element names input from Input Unit 12 or DNA microarray analysis device 26 via Communication Unit 23 and those element names of interaction partners having binary relations derived from the system. For example, on Figure 21 - 29, abbreviated gene names surrounded by circle (a circle of solid line, double-solid line, or broken line) are entered by Input Unit 29 or by DNA microarray analysis device 26, and the other abbreviated gene names are extracted as gene names that have binary relations with entered element names. In addition, when entering gene names based on more than two experimental results via Communication Control Unit 24 from DNA microarray analysis device 26, the system can discriminate and display gene names drawn on a pathway map for each experiment.

Figure 21 is drawn on the basis of: 1) a gene cluster whose expression increases in response to both 17α estradiol and genistein, 2) a gene cluster whose expression increases only in response to 17α estradiol, and 3) a gene cluster whose expression increases only in response to genistein. In addition, a Venn diagram that displays the content of a pathway map of Figure 21 is shown on Figure 21. On Figure 21, abbreviated gene names whose expression are increased by 17α estradiol are surrounded by a solid line, abbreviated gene names whose expression are increased by genistein are surrounded by a broken line, and abbreviated gene names whose expression are increased by both 17α estradiol and genistein are surrounded by a double-solid line. We can display these figures with different colors for every experiment on a pathway map. For example, abbreviated gene names whose expression are increased by 17 α estradiol may be displayed in gold, abbreviated gene names whose expression are increased by genistein may be displayed in purple, and abbreviated gene names whose expression are increased by both 17 α estradiol and genistein may be displayed in blue.

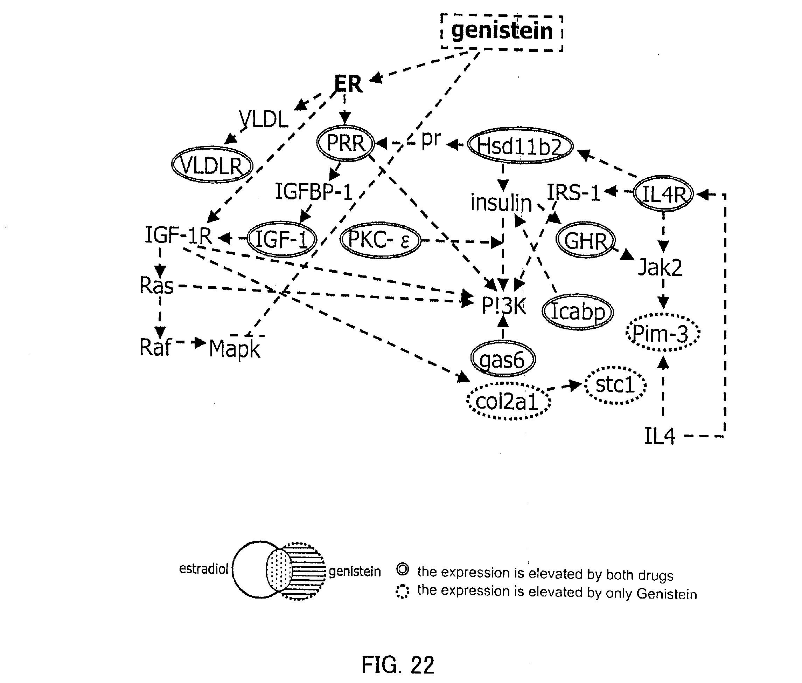

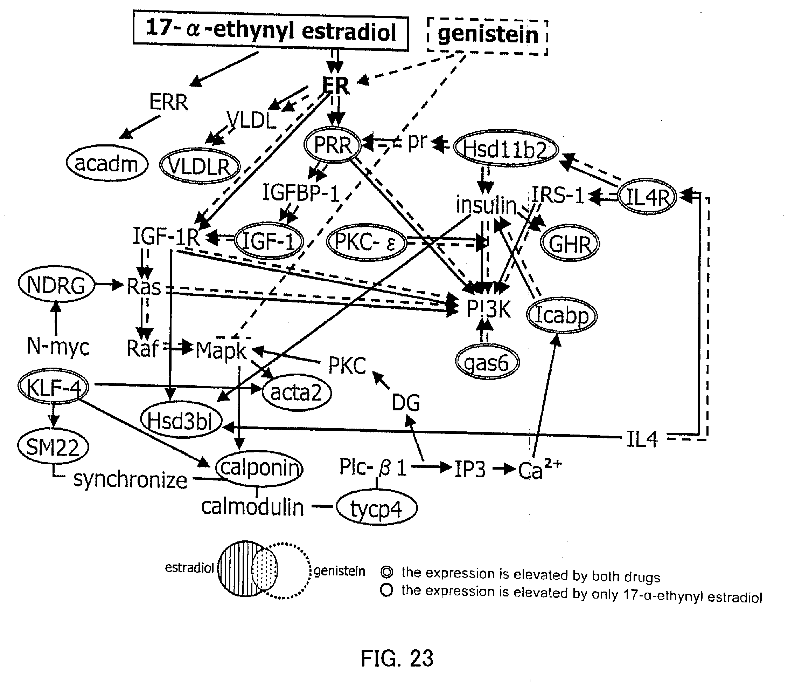

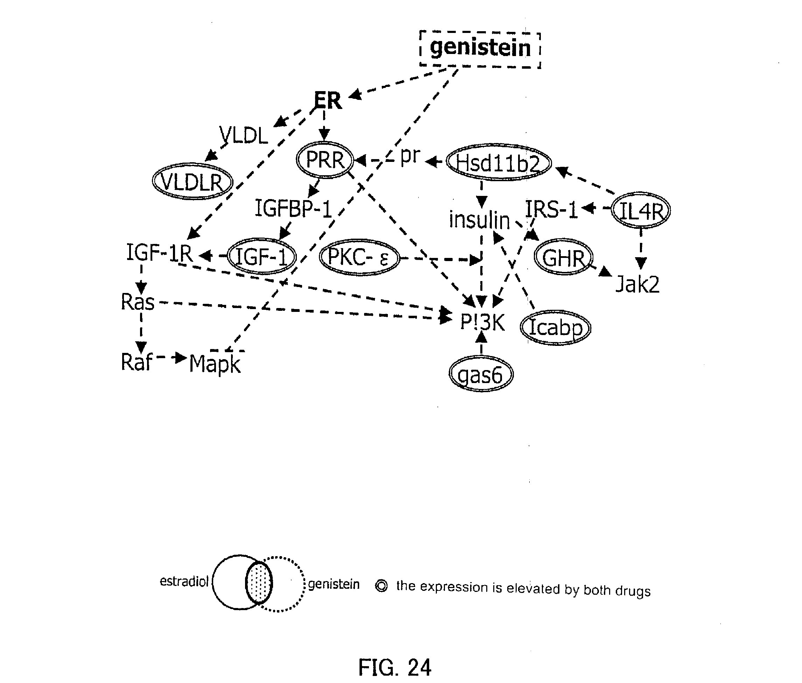

Figure 22 is a pathway map drawn on the basis of a gene cluster whose expression increases in response to genistein, and a gene cluster whose expression increases in response to 17α estradiol and genistein. Figure 23 is a pathway map drawn on the basis of a gene cluster whose expression increases in response to 17α estradiol, and a gene cluster whose expression increases in response to both 17α estradiol and genistein. Figure 24 is a pathway map drawn on the basis of a gene cluster whose expression commonly increases in response to both medicines. (In addition, genes that function as borders are also shown on these figures.) Furthermore, Figure 22, 23, and 24 are shown with Venn diagrams that display each contents of Figure 22, 23, and 24.

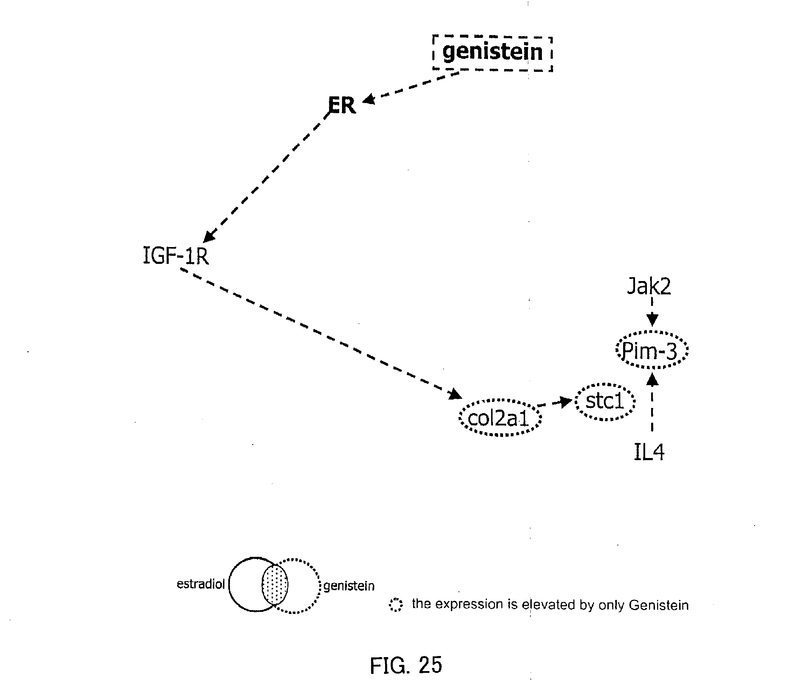

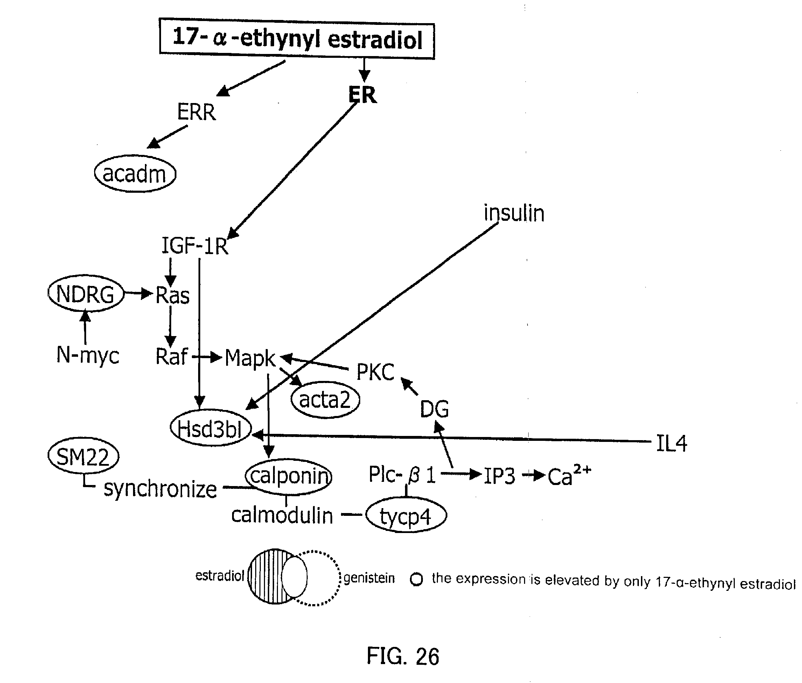

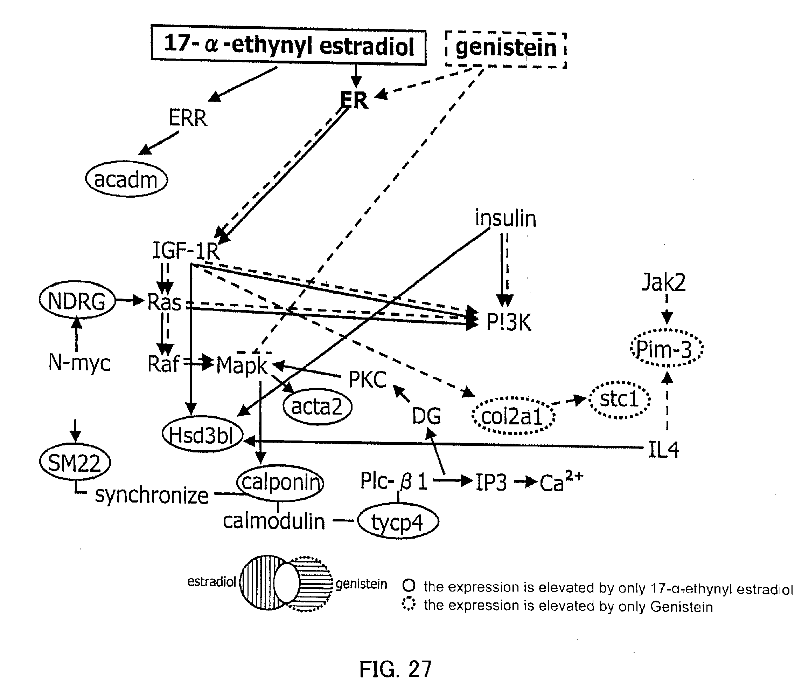

Figure 25 is a pathway map drawn on the basis of a gene cluster whose expression increases only in response to genistein. Figure 26 is a pathway map drawn on the basis of a gene cluster whose expression increases only in response to 17α estradiol. Figure 27 is a pathway map drawn on the basis of gene clusters, which excludes gene clusters whose expressions commonly increase. (In addition, genes that function as borders are shown on these figures.) Furthermore, Figure 25, 26, and 27 are shown with Venn diagrams that display each contents of Figure 25, 26, and 27.

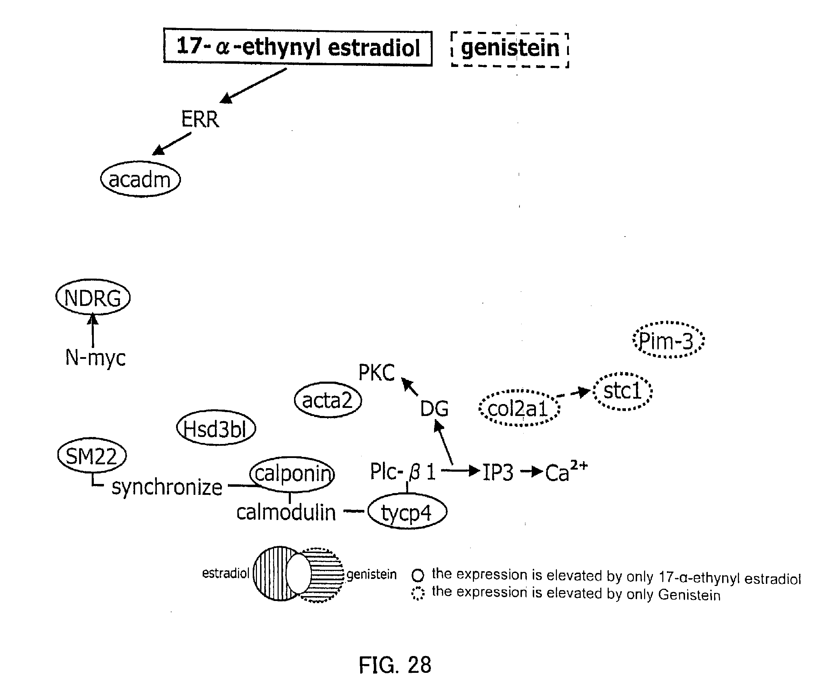

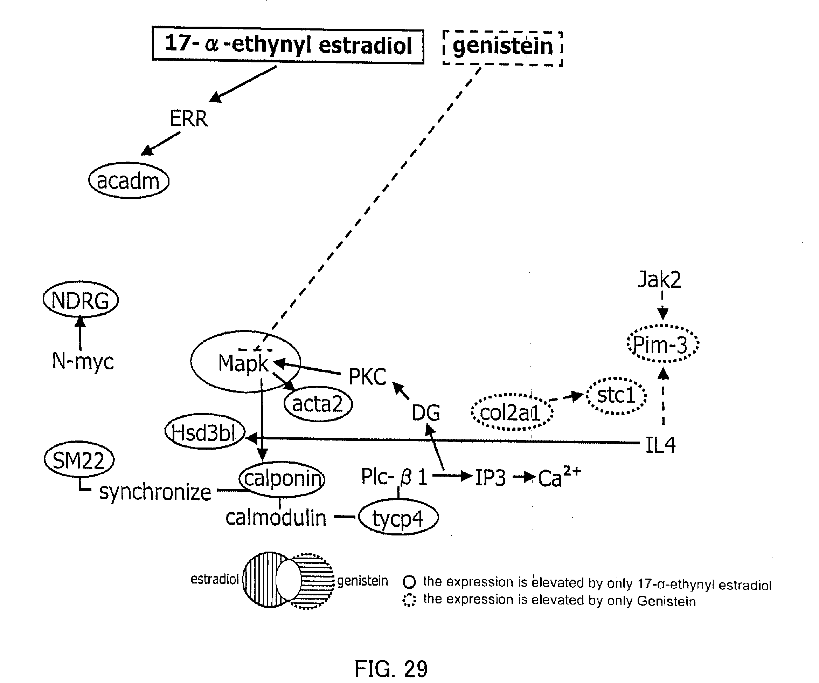

Figure 28 is a pathway map drawn on the basis of gene clusters, which excludes gene clusters whose expressions commonly increase. (In addition, genes that function as borders are excluded from these figures.) Figure 29 shows an example of displaying gene clusters surrounding the gene clusters, which its relationships are especially wanted examined, by using Figure 28.

The Biomedical Literature Information Processing System concerning the first embodiment extracts binary relations in reference to Dictionary 16 and Literature DB 14 for each of the plural element names entered, and draws a pathway map on the basis of extracted binary relations. That is, the system can extract binary relations and draws pathway maps for each of the plural element names in parallel. Consequently, the system can extract binary relations and draw pathway maps for each of the plural element names entered very quickly. That is, the system can draw pathways of interactions between protein names and gene names, signaling pathways, and metabolic pathways very quickly.

The Biomedical Literature Information Processing System concerning this embodiment can draw either a simple pathway map or a detailed pathway map, according to need, because the system can specify the extraction range of binary relations based on element names entered.

The Biomedical Literature Information Processing System concerning this embodiment can make it easy to understand pathway maps drawn, because the system can discriminate the element names entered by the input means, and element names extracted from the element names entered by the input means, to show them on pathway maps.

The Biomedical Literature Information Processing System concerning this embodiment can extract binary relations and draw pathway maps based on the latest literature information, because the literature information includes Internet information.

And the Biomedical Literature Information Processing System concerning this embodiment can directly enter the element name based on the detection result of DNA microarray analysis device 26, extract binary relations of entered element names, and draw pathway maps. That is, the system can draw pathways on the basis of detection results very quickly, because the system can enter element names obtained by more than two experiments at the same time, and extract binary relations of entered element names to draw pathway maps in parallel.

The Biomedical Literature Information Processing System concerning this embodiment makes it easy to figure out pathway maps, because the system identifies and indicates the element name drawn on the pathway map based on each experiment. Furthermore, the system can make it easy to understand analysis results on pathway maps, because the system can change element names shown on pathway maps according to need (for example: 1) displaying all gene names based on each experiment as those drawn on pathway maps, 2) displaying intersections of gene names based on each experiment as those drawn on pathway maps, and 3) displaying differences of gene names based on each experiment as those drawn on pathway maps, etc.).

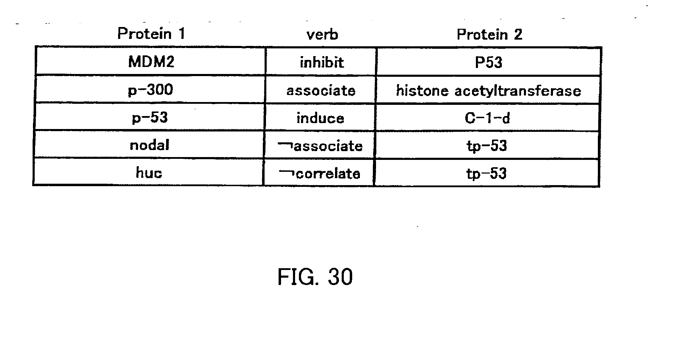

In addition, in the above embodiment, we can display binary relations stored in Binary Relation Storage Unit 19 before we draw pathway maps. Figure 30 shows a state of part of binary relation stored in Binary Relation Storage Unit 19. In the display of this binary relation, binary relations in positive expression and those in denial or negative expression are discriminated to display. That is, the system defines a binary relation of denial by displaying "┐" in front of verbs when displaying it. Consequently, to watch the display of this binary relation makes it easy to understand interactions of proteins and genes.

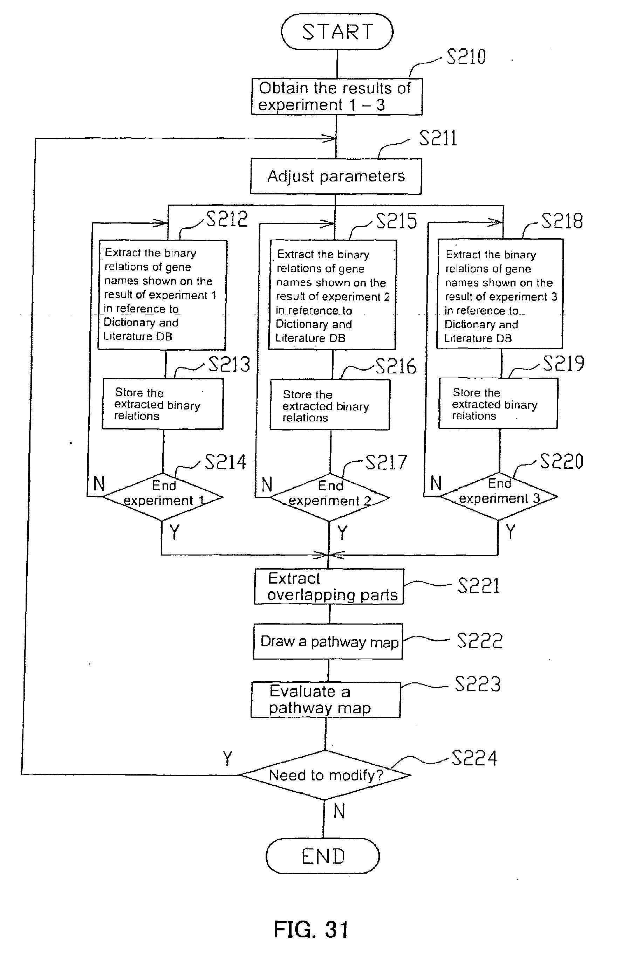

In the above embodiment, after obtaining results of experiment-1, experiment-2 and experiment-3, we can adjust the threshold values for selecting protein names and gene names that are used for pathway map drawing, and may draw pathway maps using selected gene and protein names on the basis of this adjusted threshold value. Here, the threshold value is determined by the degree of gene expressions, and defines the threshold for selecting genes. That is, as shown in Figure 31, Data Control Unit 10 of Biomedical Literature Information Data System stores results of experiment 1 - 3 obtained via Communication Unit 24 in Data Storage Unit 18 (Step S210). Next, the system automatically adjusts threshold values to extract gene names that are used for drawing pathway maps from the gene names shown as results of experiment 1 - 3 stored in Data Storage Unit 18 (Step S211). That is, the system adjusts threshold values to extract optimal gene names because the gene names obtained without any selections from the results of experiments 1 - 3, may correspond to various levels of gene expression values.

For gene names shown on the result of experiment 1, the system extracts binary relations of gene/protein names in reference to Dictionary 16 and Literature DB14 (Step S212). The system stores extracted binary relations in Binary Relation storage Unit 19 (Step 213). For each gene name extracted from the result of experiment 1, the system evaluates whether the extractions of binary relations are finished or not (Step S214) . In cases where the extractions are not finished, the system goes back to Step S212 to extract binary relations of next gene names. Because the process of Step S212 - S214 is the same as that of Step S11 - S13 (Figure 17 reference) concerning the above first embodiment, the detailed explanation of the process is omitted.

For gene names shown on the result of experiment 2, the system extracts binary relations of gene/protein names in reference to Dictionary 16 and Literature DB14 (Step S215). The system stores extracted binary relations in Binary Relation storage Unit 19 (Step 216) . Furthermore, for all of the gene names shown on the result of experiment 1, the system evaluates whether the extractions of binary relations are finished or not (Step S217) . In cases where the extractions are not finished, the system goes back to Step S215 to extract binary relations of next gene names. Because the process of Step S215 - S217 is the same as that of Step S14 - S16 (Figure 17 reference) concerning the above first embodiment, the detailed explanation of the process is omitted.

For gene names shown on the result of experiment 3, the system extracts binary relations of gene/protein names in reference to Dictionary 16 and Literature DB14 (Step S218). The system stores extracted binary relations in Binary Relation storage Unit 19 (Step 219) . Furthermore, for all of the gene names shown on the result of experiment 1, the system evaluates whether the extractions of binary relations are finished or not (Step S220) . In cases where the extractions are not finished, the system goes back to Step S218 to extract binary relations of next gene names. Because the process of Step S218 - S220 is the same as that of Step S17 - S19 (Figure 17 reference) concerning the above first embodiment, the detailed explanation of the process is omitted.

In cases where: 1) the binary relations of all gene names extracted from the result of experiment 1 are deemed to be finished on Step S214, 2) the binary relations of all gene names shown on the result of experiment 2 are deemed to be finished on Step S217, and 3) the binary relations of all gene names shown on the result of experiment 3 are deemed to be finished on Step S220, the overlapping parts of binary relations stored in Binary Relation Storage Unit 19 are extracted (Step S221). When overlapping parts are extracted, the pathway map is drawn regarding the overlapped binary relations as a reference (Step S222). Because the process of Step S221 - S222 is the same as that of Step S21 - S22 (Figure 17 reference) concerning the above first embodiment, the detailed explanation of the process is omitted.

Next, we evaluate whether the drawn pathway is appropriate or not (Step S223). Here, the pathway map is estimated either by the Data Control Unit of this Biomedical Literature Processing system or the user of the system who intends to display the pathway map drawing. That is, gene names shown by the result of experiment 1 are in many cases displayed close to one another on the pathway map. Therefore, in cases where one of the gene names shown by the result of experiment 1 is shown within those shown by the results of other experiments (because the pathway map may not be appropriate), the pathway map needs to be modified (Step S224). Consequently, the system goes back to Step S211 to adjust the threshold values and geometrical threshold values, and draws a pathway map and evaluates it (Step S211 - Step S224). As just described, the system can appropriately discriminate whether gene expressions are increasing or not, and can draw pathway maps including appropriate information that analyzers need by adjusting threshold value to extract gene names that are used for drawing pathway maps.

In addition, for drawing a pathway map interpreted in Figure 31, after obtaining the results of experiment 1 - experiment 3, the system adjusts one of the threshold value to select protein/gene names used for drawing pathway maps, and draws a pathway map using the selected protein/gene names on the basis of the threshold values. We may also adjust the threshold values and geometrical threshold values to select protein/gene names used for drawing pathway maps for each experiment, and draw a pathway map using the selected protein/gene names for each experiment, based on the adjusted threshold values.

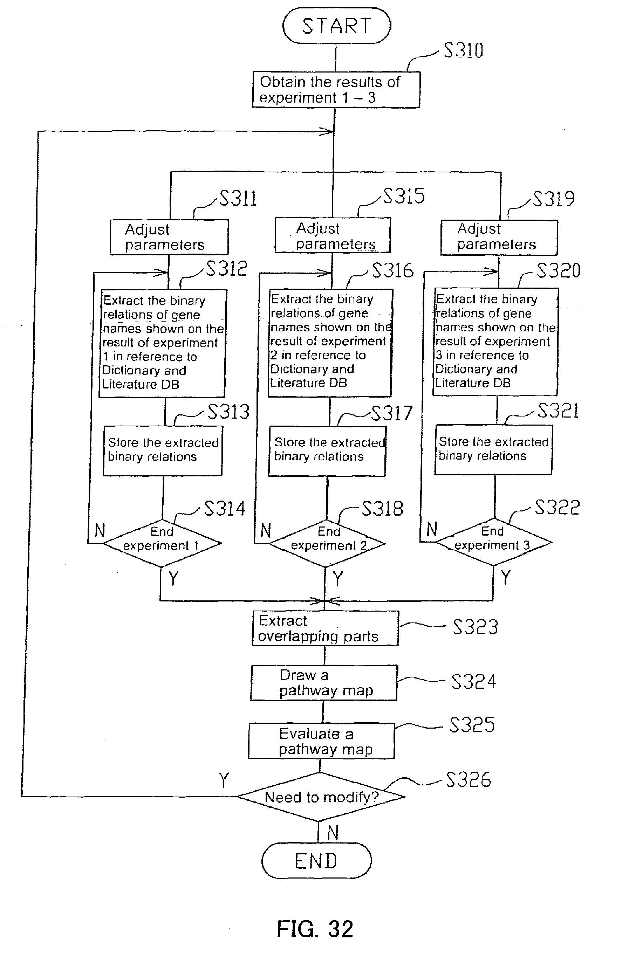

That is, as shown in Figure 32, after obtaining the results of experiment 1 - experiment 3, the system adjusts the threshold values and geometrical threshold values to select protein/gene names used for drawing pathway maps for each experiment (Step S311, S315, and S319), and draws a pathway map using the selected protein/gene names for each experiment, based on the adjusted threshold value (Step S312 - Step S314, Step S316-Step S318, and Step S320 - Step S322). The detailed explanation of the process is omitted because the process of Step 312 - S314 is the same as those of Step S212 - S214 on Figure 31, and the process of Step 320 - S322 is the same as those of Step S218 - S220 on Figure 31.

In cases where: 1) the binary relations of all gene names shown on the result of experiment 1 are deemed to be finished on Step S314, 2) the binary relations of all gene names shown on the result of experiment 2 are deemed to be finished on Step S318, and 3) the binary relations of all gene names shown on the result of experiment 3 are deemed to be finished on Step S322, the overlapping parts of binary relations stored in Binary Relation Storage Unit 19 are extracted (Step S323). If the overlapping parts are extracted, the pathway map is drawn regarding the overlapped binary relation as reference (Step S324).). The detailed explanation of the process is omitted because the process of Step 323 - S324 is the same as those of Step S221 - S222 on Figure 31.

Then, we will estimate whether the drawn pathway is appropriate or not (Step S325). If the pathway map needs to be modified, we go back to Step S311, Step S315, and Step S319 to adjust the configured threshold values of each experiment. Then we can draw a pathway map and evaluate it again. As just described, we can discriminate whether a gene is increased in expression for each experiment or not, and can draw a more appropriate pathway map by adjusting threshold values and geometrical threshold values for each experiment to extract gene names used for drawing pathway maps.

Now, we will explain the second embodiment. In the first embodiment, after extracting binary relations of gene names shown on each experiment result, we extract the overlapping parts of the binary relations and draw pathway maps, regarding the overlapping parts as a reference. In the second embodiment, we discriminate whether extractions of binary relations of gene names shown on each experimental result are finished or not. Then we extract the binary relations of the gene names whose binary relations were not extracted to draw pathway maps.

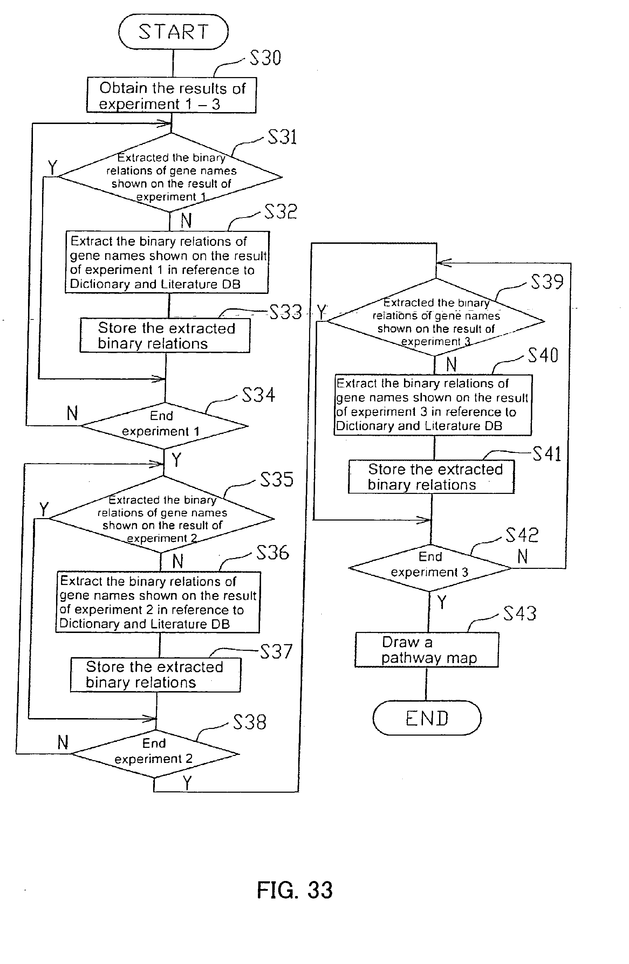

Figure 33 is the flow chart to explain extraction of binary relations and processes of drawing pathway maps on the Biomedical Literature Information Processing System, concerning this embodiment. In addition, the detailed explanation will be omitted because the system architecture of the Biomedical Literature Information Processing System concerning the second embodiment is the same as those concerning the first embodiment.

Data Control Unit 10 of the Biomedical Literature Information Processing System stores the results of experiment 1 - 3 obtained via Communication Control Unit 24 in Data Storage Unit 18 (Step S30). Then, we evaluate whether the extractions of the binary relations of the gene names shown on the result of experiment 1 are finished or not (Step S31). Consequently, we evaluate whether the binary relation is extracted and stored in Binary Relation Storage Unit 19 or not for the first gene name in the gene names shown on the result of experiment 1.

In Step S31, if the extraction of the binary relations is deemed not to be unfinished, we extract the binary relations of gene/protein names in reference to Dictionary 16 and Literature DB14 (Step S32) to store the extracted binary relations in Binary Relation Storage Unit 19 (Step S33). In addition, the extractions of binary relations in Step S32 and storage the binary relations in Step S33 are the same as Step S11 and S12 of the first embodiment.

In Step S32, if the extraction of the binary relations is deemed to be finished, we go to Step S34 and evaluate whether the extraction of binary relations of all the gene names shown in the result of experiment 1 are finished and stored in Binary Relation Storage Unit 19 or not. Here, in case where gene names whose binary relations are not extracted and should be extracted, we go back to Step S34 and extract the binary relations of the rest of the gene names.

In Step S34, if the extraction of binary relations of all the gene names that should be extracted in the result of experiment 1 are deemed to be finished, we evaluate whether the extraction of binary relations of the gene names shown in the result of experiment 2 are finished or not (Step S35), and extract the binary relation of gene/protein names for the gene names whose binary relations are not extracted in reference to Dictionary 16 and Literature DB14, then store the extracted binary relations in Binary Relation Storage Unit 19 (Step S37) . Here, the process of extracting binary relations in Step S36 is the same as that in Step S32.

If the extractions of binary relations for all the gene names that should be extracted in the result of experiment 2 are finished (Step S38), we estimate whether the extractions of binary relations for all the gene names shown in the result of experiment 3 are finished or not (Step S39), and extract binary relations of gene/protein names in reference to Dictionary 16 and Literature DB14, then store the extracted binary relations in Binary Relation Storage Unit 19 (Step S40) . Here, the process of extracting binary relations in Step S40 is the same as that in Step S32.

If the extractions of binary relations for all the gene names that should be extracted in the result of experiment 3 are finished (Step S42), we draw pathway maps of binary relations stored in Binary Relation Storage Unit 19 (Step S43).

In addition, in the Biomedical Literature Information Processing System concerning this embodiment, we can select gene names to draw on pathway maps from the gene names stored in Data Storage Unit 18. That is, we can draw pathway map to show on Figure 21 - 29 the same as the first embodiment.

In addition, the system can discriminate the element names input via Communication Control Unit 24 from DNA microarray analysis device 26 from the element names extracted as interaction partners having binary relations with those are derived from the system with those entered gene names. Furthermore, if gene names based on more than two experimental results are entered via Communication Control Unit 24 from DNA microarray analysis device 26, the system can discriminate gene names to show on pathway maps for every experiment to display.

The Biomedical Literature Information Processing System concerning the second embodiment evaluates whether the extractions of binary relations for each of plural element names entered are finished or not, then extracts the binary relations of the element names whose binary relations are not extracted in reference to Dictionary 16 and Literature DB14, and draws the pathway maps on the basis of extracted binary relations. Consequently, the system can extract binary relations and draw pathway maps very quickly for each of entered plural element names because the system doesn't redundantly extract binary relations of element names. That is, the system can draw pathway maps that show interactions between protein/gene names, signaling pathways, and metabolic pathways very quickly.

In addition, the Biomedical Literature Information Processing System concerning this embodiment can draw simple pathway maps or detailed pathway maps because the system can decide the range of extracting binary relations on the basis of entered element names.

In addition, the Biomedical Literature Information Processing System concerning this embodiment can make it easy to understand the difference in the element names of input and derived by the system using different styles of the drawn pathway maps because the system can discriminate element names entered by input means from element names of interaction or relation partners having binary relations derived from the system entered by the input means and display those element names on pathway maps.

In addition, the Biomedical Literature Information Processing System concerning this embodiment can extract binary relations and draw pathway maps on the basis of the latest literature information because the literature information includes Internet information.

Moreover, the Biomedical Literature Information Processing System concerning this embodiment can directly input element names based on the detection result of DNA microarray analysis device, and extract the binary relations of the entered element names, and draw pathway maps. In addition, the system can enter the element names obtained from more than two experiments at one time and extract the binary relations of entered element names in parallel, then draw pathway maps. Consequently, the system can draw pathway maps based on the detection results of DNA microarray analysis device very quickly.

In addition, the Biomedical Literature Information Processing System concerning this embodiment can make it easy to understand pathway maps because the system discriminates and displays element names to draw on pathway maps on the basis of each experiment. Furthermore, the system can make it easy to understand analysis results because the system can change element names shown on pathway maps according to the instruction by the user.

In addition, in the Biomedical Literature Information Processing System concerning the second embodiment, we may adjust an threshold value to select protein/gene names for drawing pathway maps and draw pathway maps using selected protein/gene names on the basis of this adjusted an threshold values after obtaining the results of experiment 1 - 3. And we may adjust an threshold value to select protein/gene names and select protein/gene names in the pathway maps for each experiment on the basis of this adjusted threshold value to draw pathway maps with selected protein/gene names.

Next, we will explain the third embodiment. In the above first embodiment, we consult Dictionary DB and Literature DB in case of extracting binary relations of gene names shown on each experimental result. However, in the third embodiment, we consult only Literature DB in case of extracting binary relations of gene names shown in each experiment. Consequently, the system architecture of the Biomedical Literature Information Processing System concerning the third embodiment is that Dictionary is removed from that concerning the first embodiment.

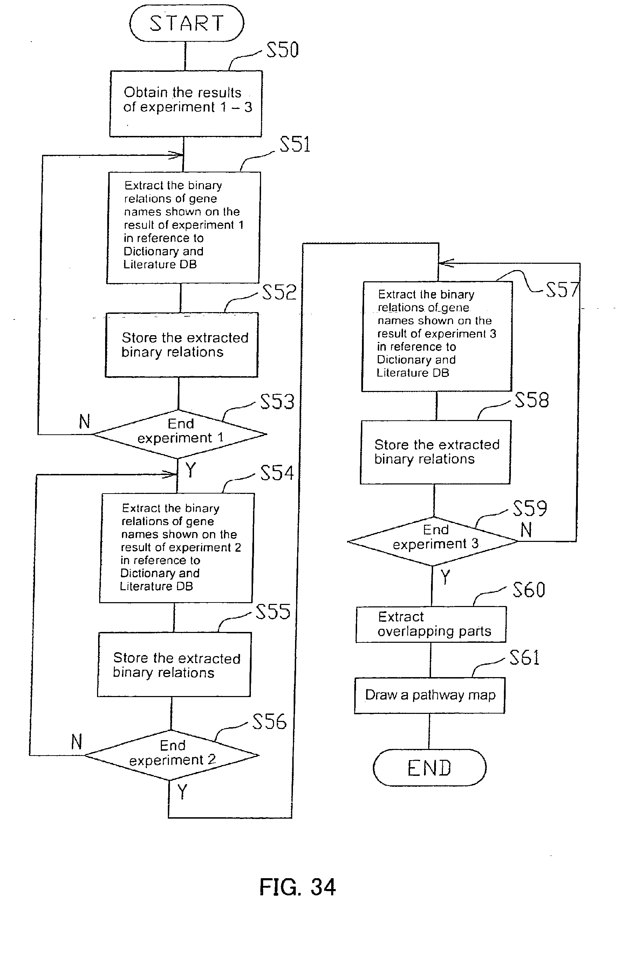

Figure 34 is the flow chart to explain extraction of binary relations and processes of drawing pathway maps on the Biomedical Literature Information Processing System concerning the third embodiment. Data Control Unit 10 of the Biomedical Literature Information Processing System stores the results of experiment 1 - 3 obtained via Communication Control Unit 24 in Data Storage Unit 18 (Step S50). Next, using natural language processing, for the gene names shown in the result of experiment 1,we extract the mutual binary relations between gene/protein names in reference to Literature DB14.

The extracted binary relations are stored in Binary Relation Storage Unit 19 (Step S52). Next, we evaluate whether the extractions of binary relations are finished or not for all the gene names shown in the result of experiment 1 (Step S53). In case where all the extractions are not finished, we go back to Step S51 to extract the binary relations of next gene names.

In Step S53, if the extraction of binary relations of all the gene names shown in the result of experiment 1 are deemed to be finished, we extract the mutual binary relations of gene/protein names in reference to Literature DB14 using natural language processing (Step S54), and store the extracted binary relations in Binary Relation Storage Unit 19 (Step S55) . Here, estimate whether the extraction of binary relations of the gene names shown in the result of experiment 2 are finished or not (Step S35), and extract the binary relation of gene/protein names for the gene names whose binary relations are not extracted in reference to Dictionary 16 and Literature DB14, then store the extracted binary relations in Binary Relation Storage Unit 19 (Step S37). Here, the process of extracting binary relations in Step S54 is the same as that in Step S51.

If the extraction of binary relations of all the gene names shown in the result of experiment 2 are deemed to be finished (Step S56), we extract the binary relations of gene/protein names for gene names shown in the result of experiment 3 in reference to Literature DB14 using natural language processing (Step S57) , and store the extracted binary relations in Binary Relation Storage Unit 19 (Step S58). Here, the process of extracting binary relations in Step S57 is the same as that in Step S51.

If the extractions of binary relations of all the gene names shown in the result of experiment 2 are deemed to be finished (Step S59), we extract the overlapping parts of binary relations stored in Binary Relation Storage Unit 19 (Step S60). If the overlapping parts are detected, the pathway map is drawn regarding the overlapped binary relation as reference information (Step S61).

In addition, in the Biomedical Literature Information Processing System concerning this embodiment, we can select gene names to draw on pathway maps from the gene names stored in Data Storage Unit 18. That is, the same as the first embodiment, the system can draw pathway maps to show on Figure 21 -29. Consequently, the system can show pathway maps on Figure 21 - 29 switching from one to the other.

And the system can discriminate and show element names entered from Input Unit 12 or DNA microarray analysis device 26 via Communication Unit 23 and element names that have binary relations with these entered element names on pathway map. Furthermore, if gene names based on more than two experimental results are entered via Communication Control Unit 24 from DNA microarray analysis device 26, the system can discriminate gene names to show on pathway maps for every experiment to display.

The Biomedical Literature Information Processing System concerning the third embodiment extracts the binary relations for each plural element names entered in reference to literature database, and draws the pathway maps based on extracted binary relations. Consequently, for each plural element names, the system can extract binary relations in parallel, in reference to literature database only, and draw pathway maps. Consequently, without a dictionary that stores the verbs indicating interactions between plural element names and element names (even a simple system architecture), the system can extract binary relations and draw pathway maps very quickly for each plural element names entered. That is, the system can draw pathways of interactions between protein names and gene names, signaling pathways, and metabolic pathways very quickly.

The Biomedical Literature Information Processing System concerning this embodiment can draw a simple pathway map or a detailed pathway map according to need because the system can specify the extraction range of binary relations on the basis of entered element names.

The Biomedical Literature Information Processing System concerning this embodiment can make it easy to understand pathway maps drawn because the system can discriminate the element names entered by the input means and element names extracted from the element names entered by the input means to show them on pathway maps.

The Biomedical Literature Information Processing System concerning this embodiment can extract binary relations and draw pathway maps on the basis of the latest literature information because the literature information includes Internet information.

The Biomedical Literature Information Processing System concerning this embodiment can directly enter the element name based on the detection result of DNA microarray analysis device, extract binary relations of entered element names, and draw pathway maps. That is, the system can draw pathways on the basis of detection results very quickly because the system can enter element names obtained by the more than two experiments at the same time and extract binary relations of entered element names in parallel to draw pathway maps.

In addition, the Biomedical Literature Information Processing System concerning this embodiment can make it easy to understand pathway maps because the system discriminates and displays element names to draw on pathway maps on the basis of each experiment. Furthermore, the system can make it easy to understand analysis results because the system can change element names shown on pathway maps according to the instruction by the user.

In addition, in the Biomedical Literature Information Processing System concerning the fourth embodiment, we may adjust an threshold value to select protein/gene names for drawing pathway maps and draw pathway maps using selected protein/gene names on the basis of this adjusted a threshold values after obtaining the results of experiment 1 - 3. We can adjust an threshold value to select protein/gene names and select protein/gene names for drawing pathway maps for each experiment on the basis of this adjusted threshold value to draw pathway maps with selected protein/gene names.

Now, we will explain the third embodiment. In the above third embodiment, after extracting the binary relations of gene names shown in the results of each experiment, the system extracts the overlapping parts of the gene names and draws pathway maps regarding the overlap as one unit of information. Meanwhile, in the fourth embodiment, the system evaluates whether the binary relations of gene names shown in each experimental result are extracted or not, then extracts the binary relations of the gene names whose binary relations are not extracted and draw the pathway maps.

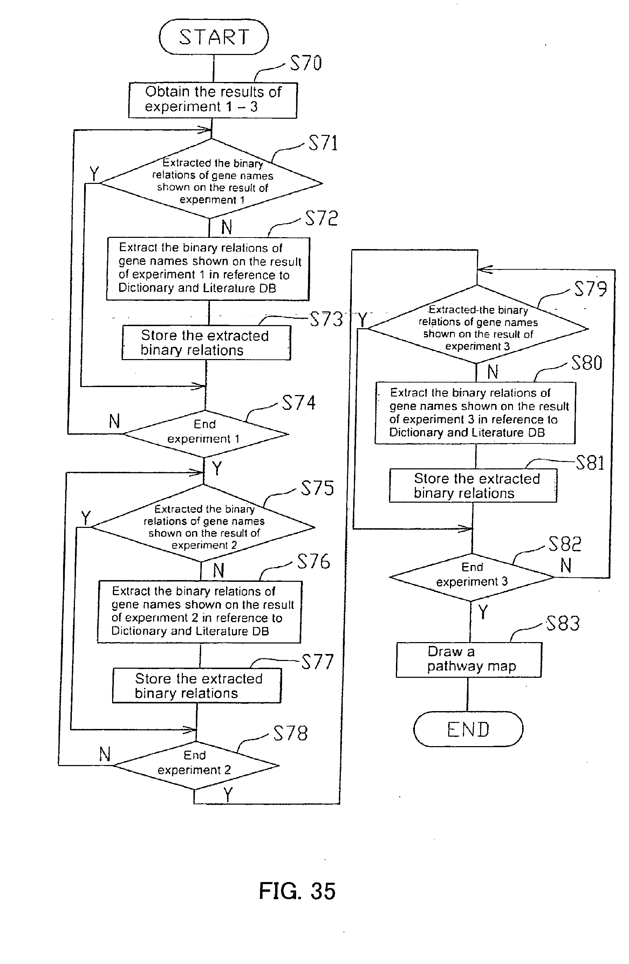

Figure 35 is the flow chart to explain extraction of binary relations and processes of drawing pathway maps on the Biomedical Literature Information Processing System concerning the fourth embodiment. The system architecture of the Biomedical Literature Information Processing System concerning the fourth embodiment, the same as those concerning the third embodiment, removes Dictionary of those concerning the first embodiment.

Data Control Unit 10 of Biomedical Literature Information Processing System stores the results of experiment 1 - 3 obtained via Communication Control Unit 24 in Data Storage Unit 18 (Step S70). Next, we evaluate whether the binary relations of the gene names shown in the results of experiment 1 are extracted or not (Step S71). That is, for the first gene name of those shown in the results of experiment 1, we evaluate whether the binary relation of the gene names is extracted and stored in Binary Relation Storage Unit 19 or not.

If the extraction of the binary relations is deemed not to be finished in Step S32, we extract the binary relations between gene/protein names in reference to Literature DB14, using natural language processing (Step S72) to store the extracted binary relations in Binary Relation Storage Unit 19. The process of extracting binary relations in Step S72 is the same as those in Step S32 of the third embodiment.

On the other hand, in Step S71, if the extraction of the binary relations is deemed to be finished, we go to Step S74 and evaluate whether the extraction of binary relations of all the gene names shown in the result of experiment 1 are finished and stored in Binary Relation Storage Unit 19 or not. In case gene names whose binary relations are not extracted, we go back to Step S71 and extract the binary relations of the rest of the gene names.

In Step S74, if the extraction of binary relations of all the gene names shown in the result of experiment 1 are deemed to be finished, we evaluate whether the extraction of binary relations of the gene names shown in the result of experiment 2 are finished or not (Step S75), and extract the binary relation of gene/protein names for the gene names whose binary relations are not extracted in reference to Dictionary 16 and Literature DB14 with natural language processing (Step S76), then store the extracted binary relations in Binary Relation Storage Unit 19 (Step S37) . Here, the process of extracting binary relations in Step S76 is the same as that in Step S72.

If the extractions of binary relations for all the gene names shown in the result of experiment 2 are finished (Step S78), we evaluate whether the extractions of binary relations for all the gene names shown in the result of experiment 3 are finished or not (Step S79), and the extraction of gene names in the result of experiment 3 is deemed not to be finished, then the system extracts binary relations of gene/protein names for unfinished ones in reference to Dictionary 16 and Literature DB14 with natural language processing (Step S80), then store the extracted binary relations in Binary Relation Storage Unit 19 (Step S81). Here, the process of extracting binary relations in Step S80 is the same as that in Step S72.

If the extractions of binary relations for all the gene names shown in the result of experiment 3 are finished (Step S82), we draw pathway maps of binary relations stored in Binary Relation Storage Unit 19 (Step S83).

In the Biomedical Literature Information Processing System concerning this embodiment, we can select gene names to draw on pathway maps from the gene names stored in Data Storage Unit 18. That is, we can draw the same pathway map to show on Figure 21 - 29 as the first embodiment.

The system can discriminate the element names entered via Communication Control Unit 24 from DNA microarray analysis device 26 from the element names extracted as partner element having binary relations with those entered gene names in depicting them. Furthermore, if gene names based on more than two experimental results are entered via Communication Control Unit 24 from DNA microarray analysis device 26, the system can discriminate gene names to show on pathway maps for every experiment to display.

The Biomedical Literature Information Processing System concerning the fourth embodiment evaluates whether the extractions of binary relations for each of plural element names entered are finished or not, then extracts the binary relations of the element names whose binary relations are not extracted in reference to Literature DB14, and draws the pathway maps on the basis of the extracted binary relations. Consequently, the system can extract binary relations and draw pathway maps very quickly for each of entered plural element names because the system does not extract binary relations of element names redundantly. That is, the system can draw pathway maps that show interaction between protein/gene names, signaling pathways, and metabolic pathways very quickly.

The Biomedical Literature Information Processing System concerning this embodiment can draw a simple pathway map or a detailed pathway map according to the needs because the system can specify the extraction range of binary relations on the basis of entered element names.

The Biomedical Literature Information Processing System concerning this embodiment can make it easy to understand pathway maps, because the system can discriminate the element names entered by the input means and element names extracted by the system from the element names entered by the input means when showing them on pathway maps.

The Biomedical Literature Information Processing System concerning this embodiment can extract binary relations and draw pathway maps based on the latest literature information, because the literature information includes information from the Internet.

The Biomedical Literature Information Processing System concerning this embodiment can directly enter the element name based on the detection result of DNA microarray analysis device, extract binary relations of entered element names, and draw pathway maps. That is, the system can draw pathways on the basis of detection results very quickly because the system can enter element names obtained by the more than two experiments at the same time and extract binary relations of entered element names in parallel to draw pathway maps.

In addition, the Biomedical Literature Information Processing System concerning this embodiment can make it easy to understand pathway maps because the system discriminates and displays element names to draw on pathway maps on the basis of each experiment. Furthermore, the system can make it easy to understand analysis results because the system can change element names shown on pathway maps according to need.

In addition, in the Biomedical Literature Information Processing System concerning the forth embodiment, we may adjust an threshold value to select protein/gene names for drawing pathway maps and draw pathway maps using selected protein/gene names on the basis of this adjusted an threshold values after obtaining the results of experiment 1 - 3. We can adjust an threshold value to select protein/gene names and select protein/gene names for drawing pathway maps for each experiment, based on this adjusted threshold value, to draw pathway maps with selected protein/gene names.

Now, we will explain the fifth embodiment. At the beginning of the fifth embodiment, in reference to Dictionary 16 and Literature DB 14, for the gene names stored in Dictionary 16, we extract the binary relations between protein/gene names (nouns and verbs) by natural language processing and determine the reliability of the extracted binary relations. In addition, we skip the detailed explanation because the system architecture of the Biomedical Literature Information Processing System concerning the fifth embodiment is the same as that concerning the first embodiment.

First of all, the determination process of the reliability of the binary relations in the fifth embodiment is explained as follows. Data Control Unit 10 extracts the binary relations between the element names (protein names, gene names, etc.) for each of element names (nouns and verbs) stored in Dictionary 16 in reference to the literature information stored in Literature DB14. The extracted binary relations are stored in Binary Relation Storage Unit 19.

Next, we will categorize the binary relations stored in Binary Relation Storage Unit 19 on the basis of the verbs in binary relations between element names. For example, we respectively categorize using such the verbs representing interaction between element names as "bind, "inhibit", interact", "phosphorylate", "mediate", "modulate", "induce", associate", etc.

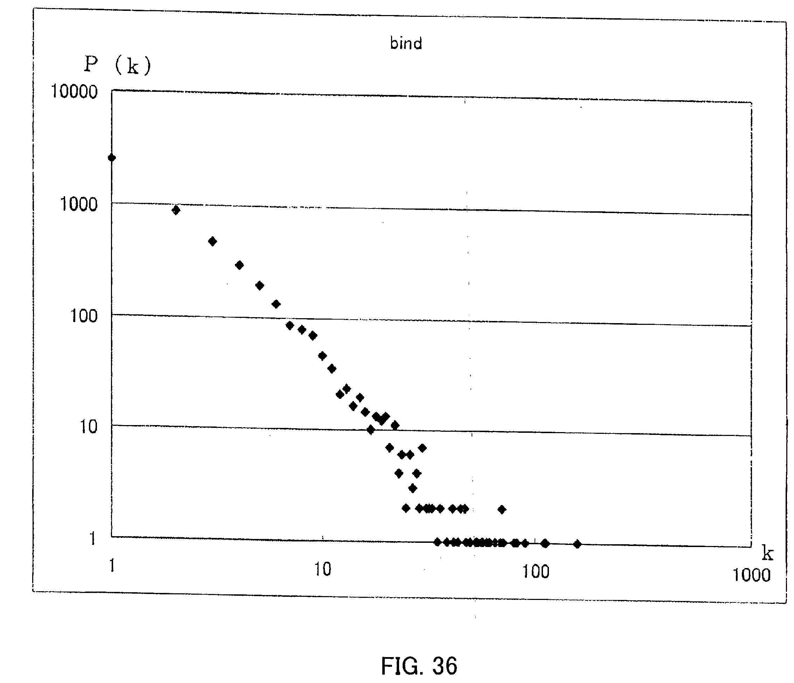

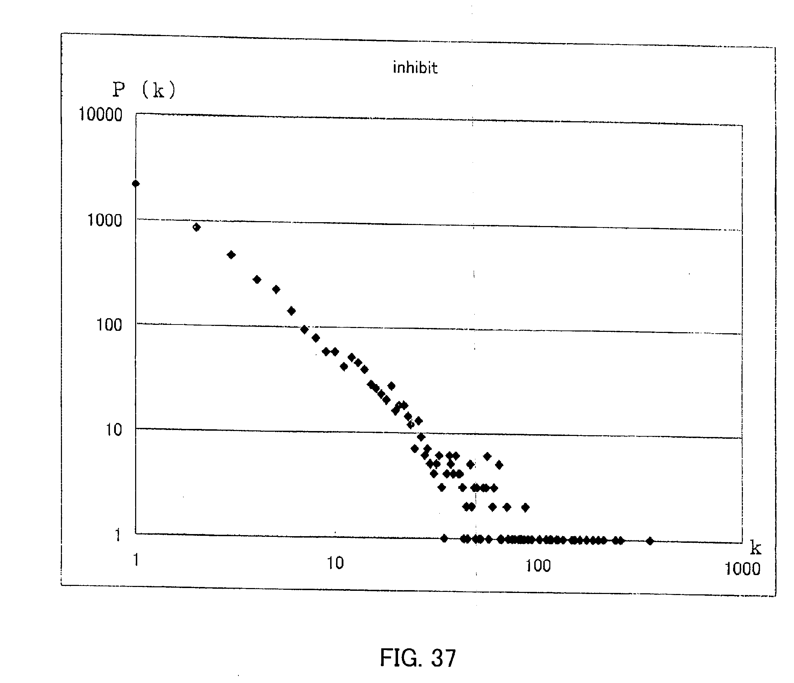

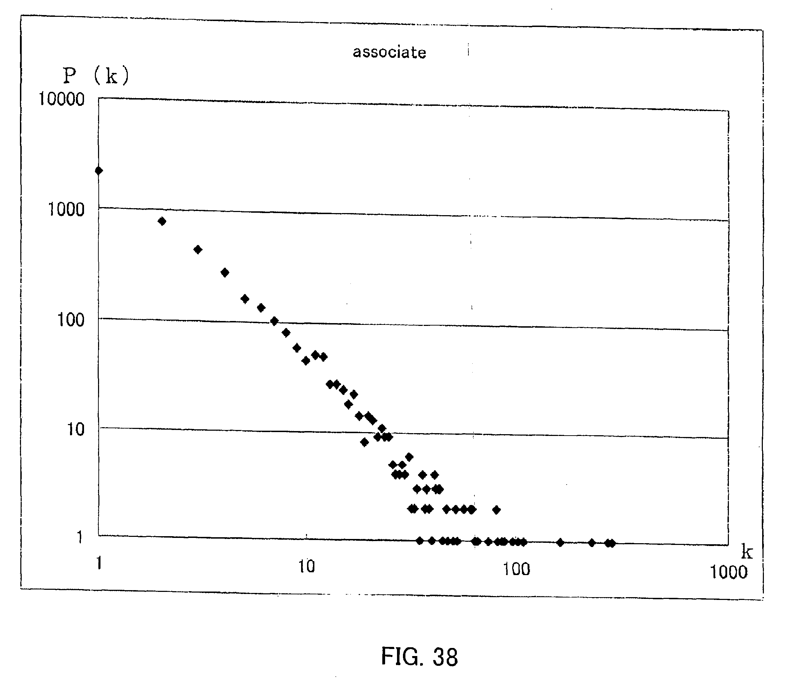

Next, for each categorized binary relation (that means for each verb that indicates an interaction between element names) , we draw the graph that indicates the interaction between a node and an edge (representing an element name as a node and representing a relationship between element names as an edge). Figure 36 is the graph that shows the interaction between a node and an edge, which have binary relation with each other, in the case of using "bind" as the verb that indicates an interaction between element names. Figure 37 is the graph that shows the interaction between a node and an edge, which have a binary relation with each other, in the case of using "inhibit" as the verb that indicates an interaction between element names. Figure 38 is the graph that shows the interaction between a node and an edge; here, "associate" is the verb as representing binary relation between them.

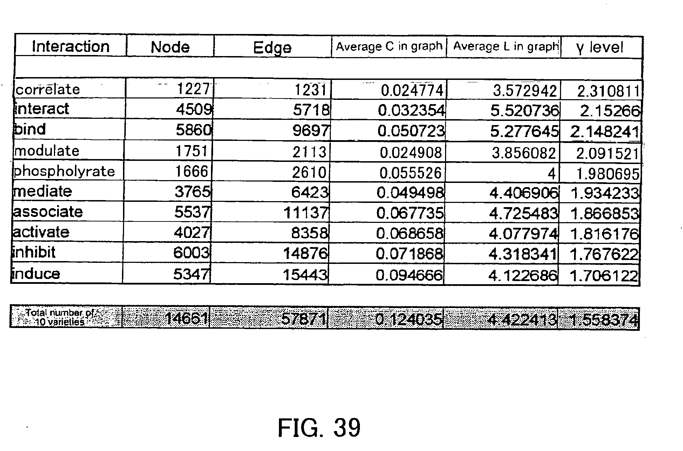

Figure 39 is the table that shows the number of nodes for each verb, the number of edges, the average clustering coefficient C in the graph, the average shortest length L in the graph, and the degree exponent □ value. The sum of 10 types of "Interaction" shown at the bottom of the table is not a simple quantity summation, but a characteristic value of the graph as the union of several graphs regarded as sets. In this table, the average clustering coefficient C of the graph is also called the cluster coefficient, which is a parameter indicating the density of the graph, and the average L in the graph is the average amount of shortest distance between all the nodes. When the number of edges of the network, "k", and the probability distribution of the node possessing the same number of edges plots as logarithm of base10 coordinates i.e., vertical and horizontal axis, and if the curve holds the nature of right-hand-downward linear curve, the network is called as a scale-free, and the slope of the linear curve □ is defined as degree exponent, in proportion to k^{-γ}. When the network has a scale-free nature, specific nodes in the network have an overwhelming number of edges, and these nodes are called "Hub nodes".

When displaying the network that has a scale-free nature for visually analyzing, specific nodes called hubs in the network have an overwhelming number of edges. Therefore the network has so many edges around hubs for example, exceeding more than 1000 edges for some of the top hub nodes, thus network diagram becomes too complex to find out important interaction relations, if we draw the network as it is. To avoid such complication, we can divide the interactions around nodes and separately draw the network if nodes are hubs. So top hubs are identified and the number of edges around top hubs is calculated previously for each hub node, and stores these data into storage. Then if we encounter a hub node having Hh edges, so we draw only the relations around the hub node, by showing Npre edges only. In this case, we can draw hub part of the edges, 1- (int (Nh/Npre) + 1, of a hub nodes with monitoring what part of interactions are drawing , and portioned pictures is drawing int(Nh/Npre) +1 times. Using this function, user is no more worry about explosive network drawing. Without this method, when the network contains hubs, it suddenly has an explosive number of edges. But this system can be used without this kind of worry and inconvenience. Here 'int' means the operation of taking integer value.

In addition, in the graphs shown on Figure 36 - 38, the vertical axis is set as the number of the nodes (P(k)), and the horizontal axis is set as the number of the edges per node(k). When finding the ideal curve from each data shown in the graphs of Figure 36 - 38, the ideal curve can be shown by the mathematical formula "P(k) = (The number of nodes that have an edge of k) "/(1/2(N(N-1))).

Based on the nature of the drawn graphs between nodes and edges, we can determine the reliability of the extracted binary relations. That is, the reliability of the extracted binary relations are guaranteed when each data of the drawn graphs are grouped near the ideal curve, but the reliability is not guaranteed when any data of the drawn graph are remarkably away from the ideal curve. In such case, for example, we correct the content stored in Dictionary 16 and add words, then extract the binary relations again. For re-extracted binary relations, regarding element names as nodes and regarding relationships of element names as nodes, we draw the relations between edges and nodes for each verb that indicate interactions between element names. The reliability of the extracted binary relations for each verb are guaranteed when each data of the drawn graphs are grouped near the ideal curve.

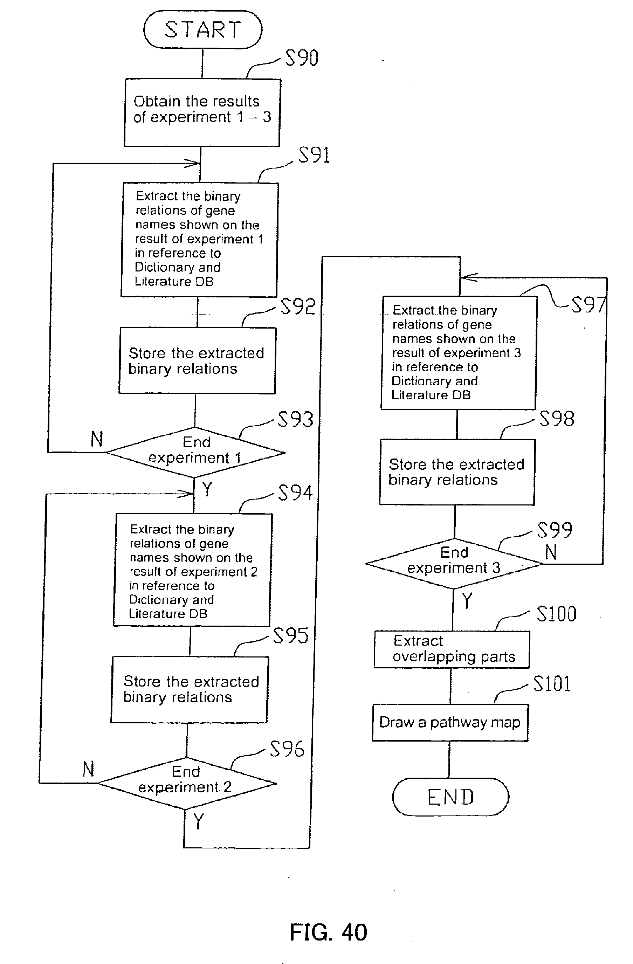

Next, we explain the extractions of the binary relations in the fifth embodiment in reference to Figure 40. Data Control Unit 10 of the Biomedical Literature Information Processing System stores the results of experiment 1 - 3 obtained via Communication Control Unit 24 in Data Storage Unit 24 (Step S90) . Next, for the gene names shown in the result of experiment 1, we extract the binary relations between gene/protein names in reference to Binary Relation Storage Unit 19. That is, for the first gene name of those shown in the results of experiment 1, we extract the binary relation in reference to the binary relations stored in Binary Relation Storage Unit 19 whose reliability is guaranteed.

The extracted binary relations are stored in Binary Relation Storage Unit 19 (Step S92) . Next, we evaluate whether the extractions of binary relations are finished or not for all the gene names shown in the result of experiment 1 (Step S93). In case where all the extractions are not finished, we go back to Step S91 to extract the binary relations of next gene names.

In Step S93, if the extraction of binary relations of all the gene names shown in the result of experiment 1 are deemed to be finished, we extract the binary relations of gene/protein names in reference to Binary Relation Storage Unit 19 (Step S94), and store the extracted binary relations in Binary Relation Storage Unit 19 (Step S95). Here, the process of extracting binary relations in Step S94 is the same as that in Step S91.

If the extraction of binary relations of all the gene names shown in the result of experiment 2 are deemed to be finished (Step S96), we extract the binary relations of gene/protein names for gene names shown in the result of experiment 3 in reference to Binary Relation Storage Unit 19 (Step S97), and store the extracted binary relations in Binary Relation Storage Unit 19 (Step S98). Here, the process of extracting binary relations in Step S97 is the same as that in Step S91.

If the extractions of the binary relations for all the genes shown in the result of experiment 3 are finished (Step S99), the overlapping parts of the binary relations (the binary relations extracted in Step S92 and stored in Step S92, the binary relations extracted in Step S94 and stored in Step S95, the binary relations extracted in Step S97and stored in Step S98) are extracted (Step S100). If the overlapping parts are extracted, the pathway map is drawn regarding the overlapped binary relations as reference information (Step S101). Here, the processes of Step S100 and Step S101 are the same as those of Step S20 and Step S21 in the first embodiment (in reference to Figure 17).

In addition, in the Biomedical Literature Information Processing System concerning this embodiment, we can select gene names to draw on pathway maps from the gene names stored in Data Storage Unit 18. That is, the same as the first embodiment, the system can draw pathway maps to show on Figure 21 -29. Consequently, the system can show pathway maps on Figure 21 - 29 switching in rotation.

And the system can discriminate and show element names entered from Input Unit 12 or DNA microarray analysis device 26 via Communication Unit 23 and element names that have binary relations with these entered element names on pathway maps. Furthermore, if gene names based on more than two experimental results are entered via Communication Control Unit 24 from DNA microarray analysis device 26, the system can discriminate gene names to show on pathway maps for every experiment to display.

The Biomedical Literature Information Processing System concerning the fifth embodiment extracts the binary relations for each of plural element names entered in reference to Binary Relation Storage Unit 19 that extracts binary relations to store beforehand, and draws the pathway maps on the basis of extracted binary relations. Consequently, for each plural element names, the system can extract the binary relations in parallel and draw the pathway maps. Consequently, the system can extract binary relations and draw pathway maps for each of plural element names entered very quickly.

The Biomedical Literature Information Processing System concerning this embodiment categorizes binary relations stored in Binary Relation Storage Unit on the basis of verbs that indicate interactions between element names, and determines the reliability of binary relation for each verb on the basis of binary relations for each of categorized verb. Consequently, the system can draw a pathway map on the basis of binary relations whose reliabilities are guaranteed, and improve the reliability of a pathway map.

In addition, in the Biomedical Literature Information Processing System concerning the embodiment, we may adjust an threshold value that is used to select protein and gene names for drawing pathway maps and draw pathway maps using selected protein and gene names on the basis of this adjusted threshold values after obtaining the results of experiment 1 - 3. We may adjust an threshold value that is used to select protein and gene names, and select protein and gene names for drawing pathway maps for each experiment based on this adjusted threshold value to draw pathway maps with selected protein/gene names.

Next, we will explain the sixth embodiment. In the above fifth embodiment, after extracting the binary relations of gene names shown in the results of each experiment, the system extracts the overlapping parts of the gene names and draws pathway maps regarding the overlapping parts as one unit of information. In the sixth embodiment, the system evaluates whether the binary relations of gene names shown in each experimental result are extracted or not, then extracts the binary relations of the gene names whose binary relations are not extracted and draw the pathway maps.

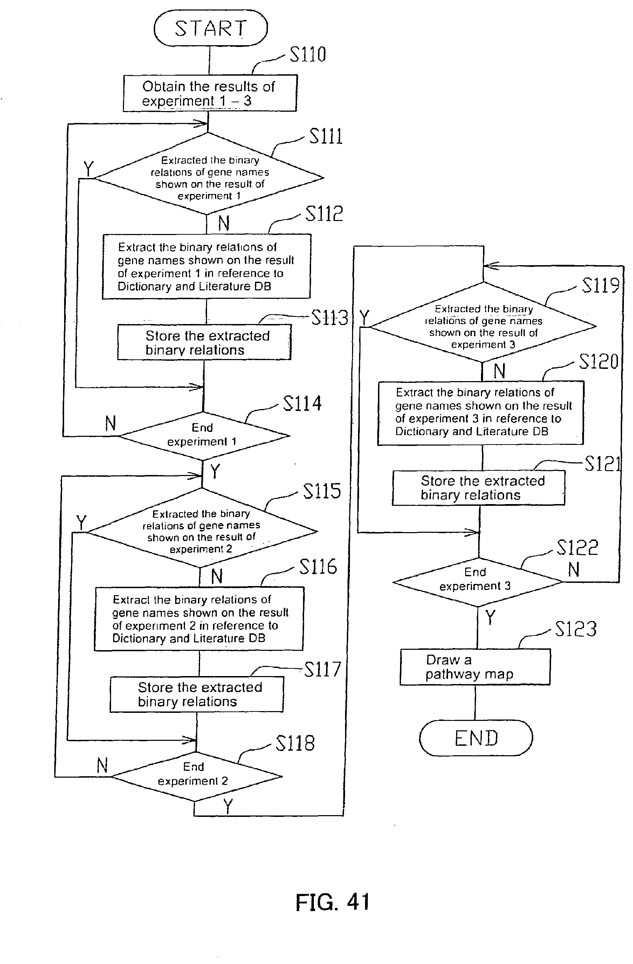

Figure 41 is the flow chart to explain binary relations and processes of drawing pathway maps on the Biomedical Literature Information Processing System concerning the sixth embodiment. We skip the detailed explanation because the system architecture of the Biomedical Literature Information Processing System concerning the sixth embodiment is the same as those concerning the fifth embodiment.

Data Control Unit 10 of Biomedical Literature Information Processing System stores the results of experiment 1 - 3 obtained via Communication Control Unit 24 in Data Storage Unit 18 (Step S110). Next, we evaluate whether the binary relations of the gene names shown in the results of experiment 1 are extracted or not (Step S111) . That is, for the first gene name of those shown in the results of experiment 1, we evaluate whether the binary relation of the gene names is extracted and stored in Binary Relation Storage Unit 19 or not.

If the extraction of the binary relations is deemed not to be finished in Step S111, we extract the binary relations between gene/protein names in reference to Literature DB19 (Step S112) to store the extracted binary relations in Binary Relation Storage Unit 19 (Step S113). In Step S111, if the extraction of the binary relations is deemed to be finished, we go to Step S114 and evaluate whether the extraction of binary relations of all the gene names shown in the result of experiment 1 are finished and stored in Binary Relation Storage Unit 19 or not. Here, in case where gene names whose binary relations are not extracted are left, we go back to Step S111 and extract the binary relations of the rest of the gene names.

In Step S114, if the extraction of binary relations of all the gene names shown in the result of experiment 1 are deemed to be finished, we evaluate whether the extraction of binary relations of the gene names shown in the result of experiment 2 are finished or not (Step S115), and extract the binary relation of gene/protein names for the gene names whose binary relations are not extracted in reference to Binary Relation Storage Unit 19 (Step S116), then store the extracted binary relations in Binary Relation Storage Unit 19 (Step S117) . Here, the process of extracting binary relations in Step S116 is the same as that in Step S112.

If the extractions of binary relations for all the gene names shown in the result of experiment 2 are finished (Step S118), we evaluate whether the extractions of binary relations for all the gene names shown in the result of experiment 3 are finished or not (Step S119), and in case where the extractions are not finished, we extract the binary relations of those gene/protein names in reference to Binary Relation Storage Unit 19 (Step S120), then store those extracted binary relations in Binary Relation Storage Unit 19 (Step S121). Here, the process of extracting binary relations in Step S120 is the same as that in Step S112.

If the extractions of binary relations for all the gene names shown in the result of experiment 3 are finished (Step S122), we draw pathway maps of binary relations (the binary relation that is extracted in Step S112 and stored in Step S113, the binary relation that is extracted in Step S116 and stored in Step S117, and the binary relation that is extracted in Step S120 and stored in Step S121) stored in Binary Relation Storage Unit 19. Here, the process of extracting binary relations in Step S123 is the same as that in Step S20 (in reference to Figure 17) of the first embodiment.

In the Biomedical Literature Information Processing System concerning this embodiment, we can select gene names to draw on pathway maps from the gene names stored in Data Storage Unit 18. That is, we can draw pathway map to show in Figure 21 - 29 the same as the first embodiment.

The system can discriminate the element names entered via Communication Control Unit 24 from DNA microarray analysis device 26 from the element names extracted as interaction partners having binary relations those are derived from the system. If gene names based on more than two experimental results are entered via Communication Control Unit 24 from DNA microarray analysis device 26, the system can discriminate gene names to show on pathway maps for every experiment to display.

The Biomedical Literature Information Processing System concerning this embodiment evaluates whether the extractions of binary relations for each of plural element names entered are finished or not, then extracts the binary relations of the element names whose binary relations are not extracted in reference to Binary Relation Storage Unit 19 that extract the binary relations to store beforehand, and draws the pathway maps on the basis of the extracted binary relations. Consequently, the system can extract binary relations and draw the pathway maps very quickly for each entered plural element names because the system doesn't redundantly extract binary relations of element names.

Moreover, in the Biomedical Literature Information Processing System concerning this embodiment, the binary relations stored in Binary Relation Storage Unit are categorized on the basis of verbs that indicate interactions between element names, and the reliability of the binary relations for each verb are determined on the basis of the binary relations of each categorized verbs. Consequently, on the basis of the binary relations whose reliability are guaranteed, we can draw the pathway map and improve the reliability of the pathway maps.

In addition, the above embodiment has a dictionary that stores verbs indicating the interaction between plural element names or element names, and a literature database that stores multiple literature information, and extracts the binary relations for each of plural element names entered in reference to the dictionary and the literature database. Although, with a database that stores a lot of literature information, we can extract the binary relations for each of the plural element names entered in reference to the database.

In addition, in the Biomedical Literature Information Processing System concerning the sixth embodiment, we can adjust a threshold value to select protein/gene names for drawing pathway maps and draw pathway maps using selected protein/gene names on the basis of this adjusted an threshold values after getting the results of experiment 1 - 3. And we can adjust a threshold value to select protein/gene names and select protein/gene names for drawing pathway maps for each experiment on the basis of this adjusted threshold value to draw pathway maps with selected protein/gene names.

The Biomedical Literature Information Processing System concerning each embodiment, as noted above, can make it easy to compare experiments whose conditions are different, because the system is able to process a large amount of data at the same time. Whether in the field of diagnosis or in clinics, the system can analyze experimental data very quickly with microarray analysis for the ability to gather experimental results and literature information at the same time, and can be used in fields of discovery of drug, elucidation of disease, and molecular biology.

In the above embodiment, we extract binary relations from biomedical literatures regarding proteins and genes as nodes (elements) and draw pathway maps, but in addition, we can also extract multiple relations, such as three-body or four-body and many-number-body relations, from biomedical literatures regarding proteins and genes as nodes (elements) and draw pathway maps. We have analyzed binary relations between proteins and genes in the above embodiment. Even if extending this to the case of generalizing and extracting pathway information attributed to many-body interactions between multiple proteins and genes, the effect of this invention will be useful as those in the case of binary relations. We will take transcriptional control as a cooperative operation of many-body interactions between multiple proteins. In T cell receptor α gene enhancer, AML-1 and Ets-1 binds to transcription start sites of genes first, and ATF binds to DNA in the same way, then DNA is folded back to about 130 degrees by LEF-1 binding to DNA. Hereby, the transcription starts after the binding of ATF, AML-1, and Ets-1. We can clearly understand the function from the viewpoints of multiple relations involving 6 elements (including DNA). This invention has a characteristic in advantage of analyzing complicated phenomena in life concerning complicated interactions (such as a transcription initiation) from multiple proteins and multiple interaction relations.

In addition, three-body interaction relation means the interactions between gene and protein names indicated, such as "A (gene name) associate (verb) with B (gene name) and C (gene name)", or "cooperative interactions among A (gene name), B (gene name) and C (gene name) ". Four-body interaction relation means the interactions between gene and protein names indicated such as "A (gene name) - B (gene name) - C (gene name) - D (gene name) complex". By extracting the multiple interaction relations just described, we can study phenomena caused by complex interactions between multiple gene and protein names, such as transcription activity, epigenetic effect such as methylation, and protein complex, etc.

In the previous interaction extraction, we have extracted binary relations within multiple relations, a combination of single verb and two nouns "noun - verb - noun", from literature information, and analyzed to draw a pathway map in the above embodiment. Here we can extract the multiple relations from literature information, where the same combinations of element names and verbs, the different combinations of element names and verbs, such as "noun - verb - noun - verb - noun", or more variations of repeating of nouns and verbs combinations. This multiple interactions improve the results of extractions and the accuracy of searching literature information, and accurately give the meaning of the extracted results from literature.

In the field of molecular biology, the time sequences of signaling in cells, which can be represented by combinations of nouns that indicate many proteins and verbs that indicate interactions between proteins, are the time series in specific events involving many interacting proteins. In this case, the specific order of specific set of verbs is important. In the case of "noun - verb - noun", it is often observed in the literature that the function of a protein is induced after the other protein binds to this protein. In particular, using NFkB as an example, NFkB in the in the cell cytoplasm move into the nucleus and begins to function:
'Activation of NF-kappa B to move into the nucleus is controlled by the targeted phosphorylation and subsequent degradation of IkkB (I kappa B). Exciting new research has elaborated several important and unexpected findings that explain mechanisms involved in the activation of NF-kappa B. In the nucleus, NF-kappa B dimers bind to target DNA elements and activate transcription of genes encoding proteins involved with immune or inflammation responses and with cell growth control. '(Annu Rev Immunol. 1996;14: 649-83.)
The example of the protein called JNK is:
'we conclude that the minimal stimulation of one-third PH activates JNK, which phosphorylates the c-Jun activation domain in hepatocytes, resulting in enhanced transcription of AP - dependent genes. (J Clin Invest. 1995 Feb; 95(2): 803-10.)

Here is another example where a protein in a cell membrane translocate to a nucleus:
'Unprocessed, full-length APP has been proposed to have a role in axonal transport of membrane-associated cargo [7]. In addition, the intracellular C-terminal fragment that results from APP processing by γ-secretase functions in gene expression as a transcription factor [8 and 9].' (TRENDS in Neurosciences, 27, 1-3 (2004))

More example of this is that a protein in the cytoplasma moves to Golgi and some of the portion was *cleaved* and the portion moves to nucleus:
'The sterol regulatory element binding protein (SREBP) precursor is inserted into membranes of the endoplasmic reticulum (ER). Both the amino-terminal transcription-factor domain (bHLH-zip) and the carboxy-terminal regulatory domain (Reg) are located in the cytoplasmic compartment. When the cellular demand for sterols rises, the SREBP precursor protein travels to the Golgi apparatus, where the site-1 protease (S1P) cleaves at site-1 in the luminal loop (red line) , producing the membrane-bound intermediate form. The intermediate form is the substrate for the site-2 protease (S2P), which cleaves the intermediate at site-2 (double red line) , which is located three amino acids into the membrane-spanning helix. This second cleavage releases the transcription-factor domain from the membrane, freeing it to enter the nucleus and direct the increased transcription of target genes. BHLH-zip, basic helixloophelix leucine-zipper.' (nature review 4, 631-640 (2003))

The expressions of the concept of time flow in the biology literature can be found in the terms such as G1 phase, S phase, or M phase in a cell cycle. However in many cases, time flow is represented by the order of multiple events, such as the order of interactions and movements of specific proteins. Therefore, the extraction of the same or the different combinations of protein (or gene) names and verbs in a sentence from literature information, such as "some protein nouns of interactions that indicate protein names - verb of an interaction - protein noun - verb of an interaction - verb that indicates a function", provides significant sentences relating time dependent complex phenomena, which lead to the deep understanding of life, that we cannot obtain from using the extraction for the binary relations.

In the same way, by extracting a set of the noun that indicates a cell name or localization in a cell with the above noun -verb - noun for the reason that those emerge in a text at the same time, from a text, we can clearly specify the protein interaction place in a cell. Here, we can replace a verb by a noun phrase or an adjective phrase. According to the extracted binary relations, we can mathematically analyze correlations between protein and gene names by the scalar field. We can also analyze the correlations matrix, as a vector or tensor field for the results of extracted multiple (or binary) relations.

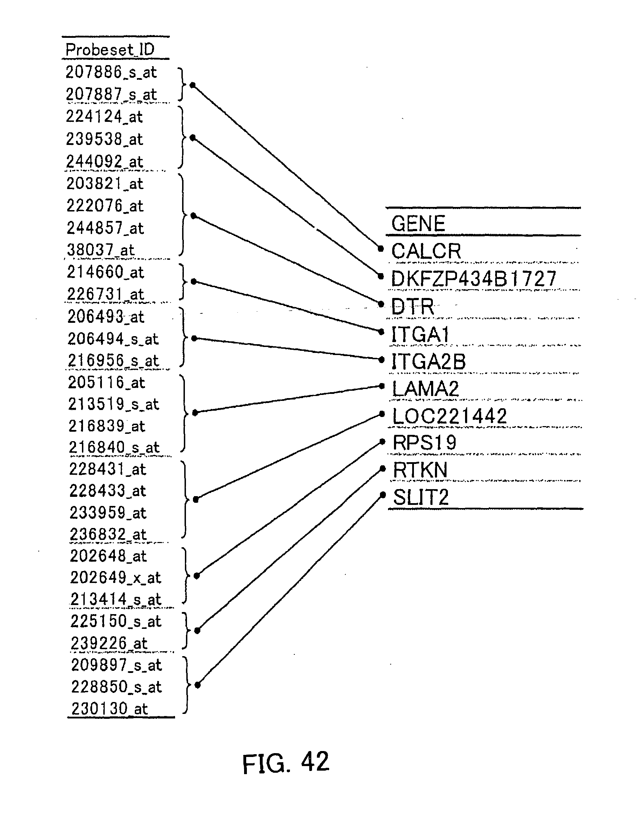

Additionally, we can store the list that indicates relationships from probe IDs obtained as experimental results by microarray analysis device to the substantial mRNAs or genes, and the relationships from protein/gene names that have the reverse relations to probe IDs. Figure 42 shows the list that indicates relationships between probe IDs, gene names, and protein names. This list shows the many-to-one (probe IDs to gene/protein name) relation. When drawing pathway maps that are networks of relations between gene and protein names from literature information, we can easily find expression information of proteins on pathway maps by storing this kind of list. Moreover, we can easily convert these relations to expression information of proteins on pathway maps.

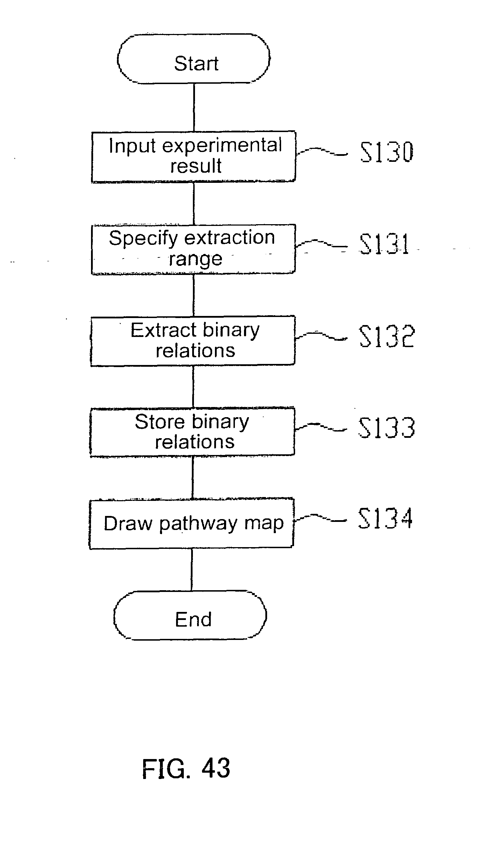

Next, we will explain the seventh embodiment. Figure 43 is a flow chart to explain extractions of binary relations in Biomedical Literature Information Processing System concerning the seventh embodiment and drawing processes of pathway maps. In addition, we will explain in reference to the first embodiment, because the system architecture of the Biomedical Literature Information Processing System concerning the seventh embodiment is the same as those concerning the first embodiment.

Data Control Unit 10 of the Biomedical Literature Information Processing System stores the experimental results obtained via Communication Control Unit 18 in Data Storage Unit 18 (Step S130). And in the following, we will explain by taking an example of the case in which protein A is obtained as an experimental result in DNA microarray analysis device.

Next, we specify the extraction range of binary relations on the basis of protein A stored as an experimental result (Step S131). Consequently, we specify the range (hierarchy) of proteins that are extracted as having binary relations with protein A.

Next, in the range specified on Step S131, we extract binary relations between gene names and protein names for protein names stored as experimental results in reference to Dictionary 16 and Literature 14 (Step S132). That is, for protein A, with using natural language processing, we extract binary relations of protein/gene names indicated by "noun (protein A)", "verb", and "noun (protein name)".

In addition, for "noun (protein name)" extracted as having binary relations with "noun (protein A)", we extract binary relations of protein/gene names indicated by "noun (protein name) ", "verb", and "noun (protein name) " That is, we extract not only binary relations of protein names obtained as experimental results, but also those of protein names extracted as having binary relation with the protein name (protein A) obtained as an experimental result. In the extraction range (the range of extracted hierarchy) specified on Step S131, for example, this extraction of the binary relation is complete within the range of the second hierarchy from the entered protein name (protein A), or within the range of extracting protein names that are directly involved in functions.

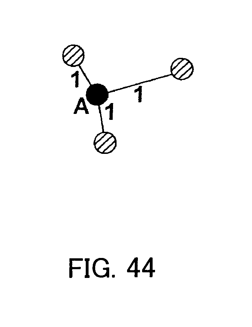

Here, in the case where pathway map is drawn with using protein A and the protein (of the first hierarchy) that has binary relation with protein A, regarding protein A (black circle in Figure 44) as a node as shown in Figure 44, the distance between protein A and the extracted protein (circle of diagonal line in Figure 44) is connected with solid line. That is, the edge that indicates the binary relation on the first hierarchy (the solid line indicated with 'number 1' in Figure 44) is formed. Here, the binary relations are not extracted even if binary relations exist between proteins of the first hierarchy because what extracted is proteins that have binary relations with protein A. Consequently, the binary relations that exist between proteins of the first hierarchy in the pathway maps are drawn at this stage.

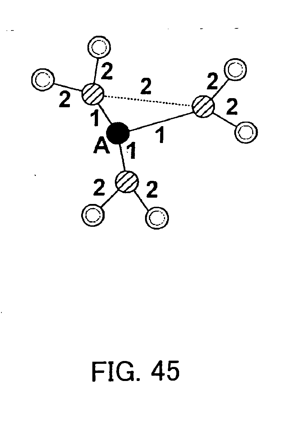

On the other hand, in the case of extracting the proteins (of the second hierarchy) that have binary relations with proteins of the first hierarchy, the binary relations between proteins of the first hierarchy, which are not extracted when extracting proteins of the first hierarchy, are extracted. That is, as shown in Figure 45, when extracting the proteins of the second hierarchy (double circle in Figure 45) that have binary relations with proteins of the first hierarchy, the binary relations between proteins of the first hierarchy are extracted at the same time. And the distance between proteins of the first hierarchy and those of the second hierarchy and the distance between proteins of the first hierarchy are connected by the edges (solid line shown with 'number 2' in Figure 45) that indicate the binary relations of the second hierarchy. In the same way, we cannot extract the binary relations between the proteins of the second hierarchy unless extracting the proteins (of the third hierarchy) that have binary relations with the proteins of the second hierarchy. We cannot extract the binary relations even if the binary relations exist between the proteins of the second hierarchy that are already extracted because the binary relations of the third hierarchy are not extracted in the case where the predetermined range of extraction is limited to the second hierarchy.

Consequently, in Step S132, the extraction of proteins is performed to the hierarchy specified as an extraction range from protein A that is obtained as experimental result. At the same time, the binary relations between the proteins of the hierarchy already extracted are extracted. In the case where the extraction range is limited to the second hierarchy, for example, the system extracts binary relations that exist between proteins of the second hierarchy that are already extracted in parallel with extracting to the range of the proteins of the second hierarchy.

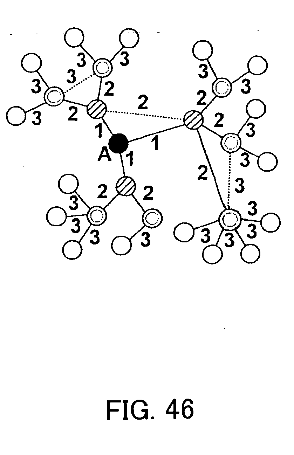

The binary relations extracted on Step S132 are stored in Binary Relation Storage Unit 19 (Step 133). Next, we draw a pathway map on the basis of binary relations stored in Binary Relation Storage Unit 19 (Step S134). Here, even in the case where the range of necessary pathway map is the binary relations between the proteins of second hierarchy, and in the case of extracting binary relations within usual procedure, we cannot draw the edge that indicates the binary relations between the proteins of second hierarchy without extracting to the extent of the third hierarchy. Consequently, as shown in Figure 46, it is difficult to obtain necessary information from the pathway map because the proteins of the third hierarchy that are not essentially needed are drawn on a pathway map and necessary information are buried. Especially, in the case where the number of proteins that are obtained as an experimental result is large, or in the case where the number of proteins extracted is large, it is quite difficult to determine the necessary information.

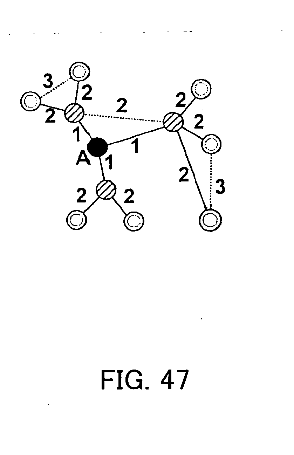

With that, as defined in the above Step S132, by extracting binary relations that exist between proteins that are already extracted as well as extracting binary relations from protein A in the range of specified extraction, the pathway map as shown in Figure 47 is drawn. The edge is shown with assigning the 'number' that indicates the binary relations on either hierarchy. (For example, 'number 1' in the case of the binary relation extracted on the first hierarchy, 'number 2' in the case of the binary relation extracted on the second hierarchy, 'number 3' in the case of the binary relation extracted on the third hierarchy)

The Biomedical Literature Information Processing System concerning the seventh embodiment extracts only multiple relations between element names already extracted without extracting new element names, in extracting multiple relations that exist between element names extracted as having multiple relations (binary relations). Consequently, the system can make it easy to visually figure out necessary information from the pathway map because necessary information are not buried by drawing of proteins not needed.

The Biomedical Literature Information Processing System concerning the seventh embodiment extracts binary relations that exist between proteins already extracted and draws a pathway map, as well as extracting binary relations in the specified range of extraction based on protein A obtained as experimental result. Consequently, there is no need for extracting proteins with another new hierarchy, for extracting binary relations that exist between proteins are already extracted. Therefore we can shorten the process time of extracting binary relations and reduce the resources that compose the Biomedical Literature Information Processing System.

In addition, in the Biomedical Literature Information Processing System concerning the above seventh embodiment, we gave an explanation with the example of the case of protein A being obtained as an experimental result. We can obtain plural proteins such as protein A and protein B and so on as an experimental result. Here, in the case that protein A or protein B is obtained as an experimental result, we specify each range of extraction on protein A and protein B (for example, for protein A, the extraction range to the proteins of the second hierarchy and to the binary relations that exist between the proteins of the second hierarchy. For protein B, the extraction range to the proteins of the second hierarchy) and extract binary relations. After extracting the overlaps of the extracted binary relations, we can draw the pathway map regarding the overlapped binary relations as one unit of information.

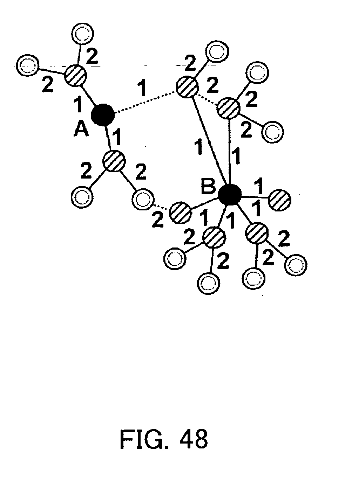

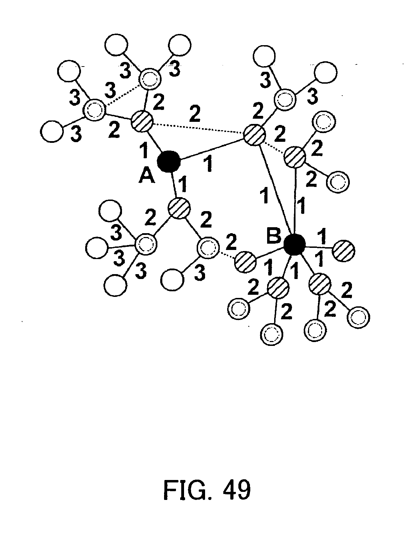

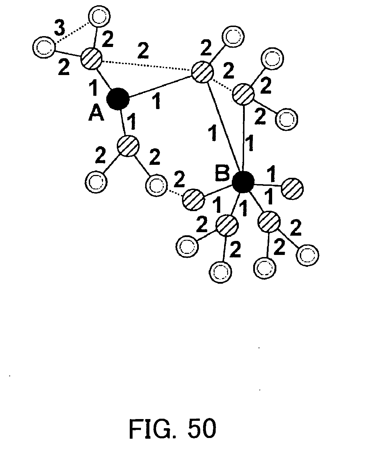

Here, for protein A and protein B, in the case of extracting in the range to the second hierarchy, the pathway map is drawn as shown in Figure 48. In the case of extracting in the range to the third hierarchy for only protein A, the pathway map is drawn as shown in Figure 49. Here, in the case of extracting only binary relations between the proteins of the second hierarchy that are already extracted for protein A, the pathway map is drawn as shown in Figure 50, and the number of proteins is smaller as compared with the pathway map shown in Figure 49. Figure 50 shows that we can make the content easier to understand. In addition, in the case of drawing a pathway map as Figure 50, as in the case of the above embodiment, we can have the advantage such as shortening the process time to extract binary relations or reducing the resources that compose the Biomedical Literature Information Processing System.

In the above seventh embodiment, we input (obtain) protein names into the system, but we can input the protein names obtained from probe IDs as an experimental result (for example, the gene cluster selected by limiting the threshold of gene expression amount) provided by DNA microarray analysis device 26.

In addition, in the Biomedical Literature Information Processing System concerning the above seventh embodiment, we extract binary relations in reference to a dictionary and Literature DB, but we can extract binary relations only in reference to Literature DB.

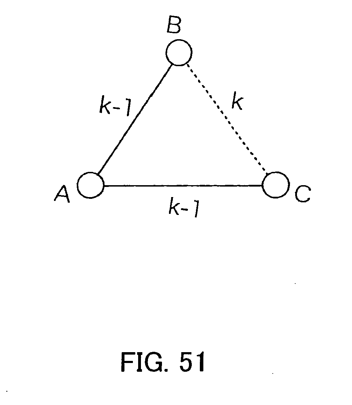

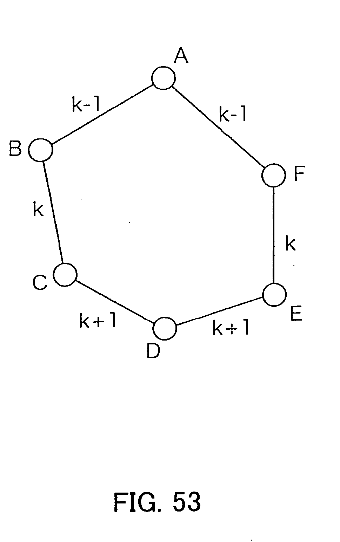

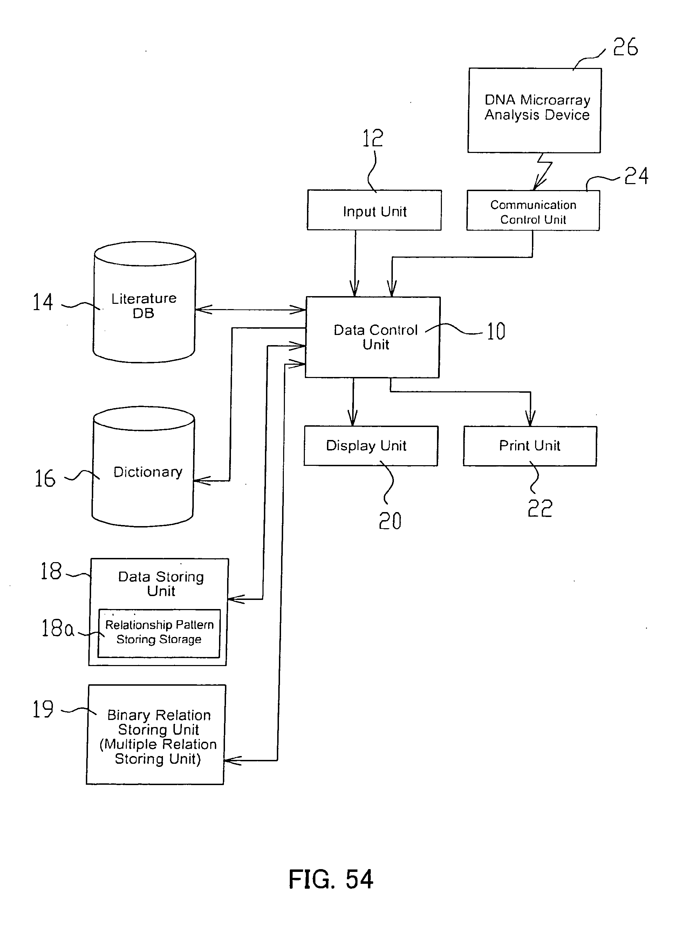

We can verify the reliability of drawn pathway maps based on relationships between nodes and edges. By setting the 'number k-1', 'number k', and 'number k+1' to the edges in the k-1, k, and k+1 hierarchy of the binary relations between protein names, we observe that the relationships as shown in Figure 51 -53. Consequently, as shown in Figure 54, these relationships are previously stored in Relationship Pattern Storage 18a that is set up in Data Storage Unit 18. Here we omit the detailed explanation of the system because the Biomedical Literature Information Processing System shown in Figure 54 is the same configuration as the Biomedical Literature Information Processing System concerning the first embodiment.

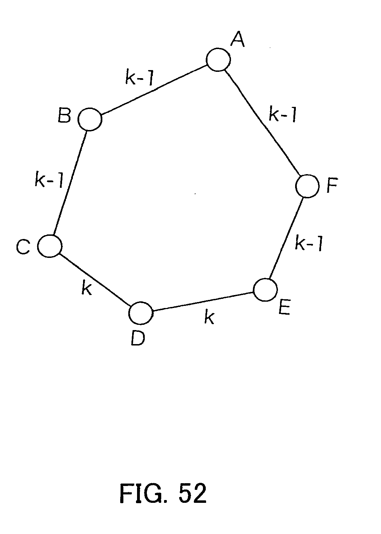

In the Biomedical Literature Information Processing System concerning this embodiment, we can mathematically verify the reliability of pathway maps by mapping (or homology mapping) the relation patterns stored in Relationship Pattern Storage 18a to the relations between nodes and edges in the drawn pathway map in Data Control Unit 10 where it functions as verification. For example, in the pathway map shown in Figure 45, there are closed part, for example, that are composed of protein A (black circle), proteins of the first hierarchy (circle of diagonal line), and edges with 'number 1' and 'number 2'. Here 'number 1' indicates the binary relation between protein A and proteins in the first hierarchy, and 'number 2' indicates the binary relations of the proteins in the second hierarchy. The interaction connection pattern formed by protein A (black circle), proteins of the first hierarchy (circle of diagonal line), and edges with 'number 1' and 'number 2', is identical with the pattern as shown in Figure 51 which is stored in the Relationship Pattern Storage 18a. This identification whether the pattern under consideration is identical with the pattern stored in Relationship Pattern Storage 18a is judged in the Data Control Unit 10 by using homology analysis. Similarly, by identifying the patterns formed by closed loop in Figure 50 with the stored patterns as shown in Figure 51 and 52, we can verify the reliability of the pathway map as shown in Figure51. Generally, Identifying the patterns of the closed loops found in the Pathway Map with the stored patterns, such as shown in Figure 51-53 verifies the reliability of the Pathway Map.

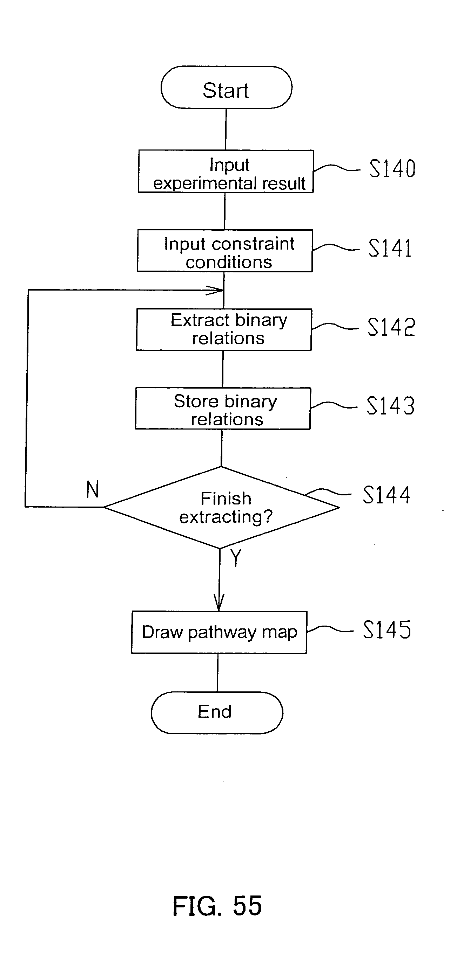

Now, we will explain the Biomedical Literature Information Processing System concerning the eighth embodiment. Figure 55 is a flow chart to explain the procedures of extractions of binary relations in Biomedical Literature Information Processing System concerning the eighth embodiment and drawing processes of pathway maps. In addition, we will explain in reference to Figure 1 because the system architecture of the Biomedical Literature Information Processing System concerning the eighth embodiment is the same as those concerning the first embodiment.

Data Control Unit 10 of Biomedical Literature Information Data System receives experimental results (Step S140). The detailed explanation of the process in Step S140 is omitted because the process is the same as those of Step S130 in Figure 43.

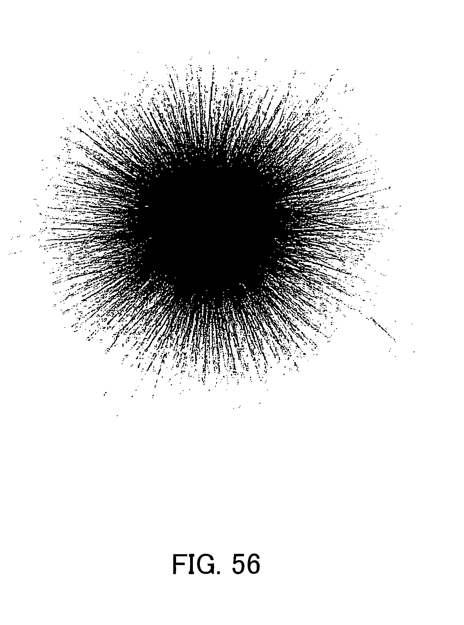

Next, we input the defined conditions that are used for drawing pathway maps (Step S141). For example, we input plural protein names (gene names) as element names of experimental results, then the system provides plural protein names as interacting partners that have binary relations with each input protein name, and also provides, by recursive searching, plural protein names as interacting partners that have binary relations with the first-extracted protein names by inputting first-extracted protein names. The number of total extracted protein names for drawing in a pathway map, as shown in Figure 56, is so many. Here Figure 56 shows the pathway map for indicating relations for micaroarray results for 17 α estradiol experiment mentioned before. The black circles indicate protein names (gene names), and the solid lines that connect the black circles indicate binary relations between protein names (gene names). In such a pathway map, it is difficult to understand the information in the network such as the extracted protein names and binary relations between protein names. Consequently, we must specify defined conditions for reducing nodes (protein names) and edges (binary relations between protein names) in the pathway map (big one) to re construct a pathway map (small one) that includes necessary information from nodes and edges as shown in Figure 56.

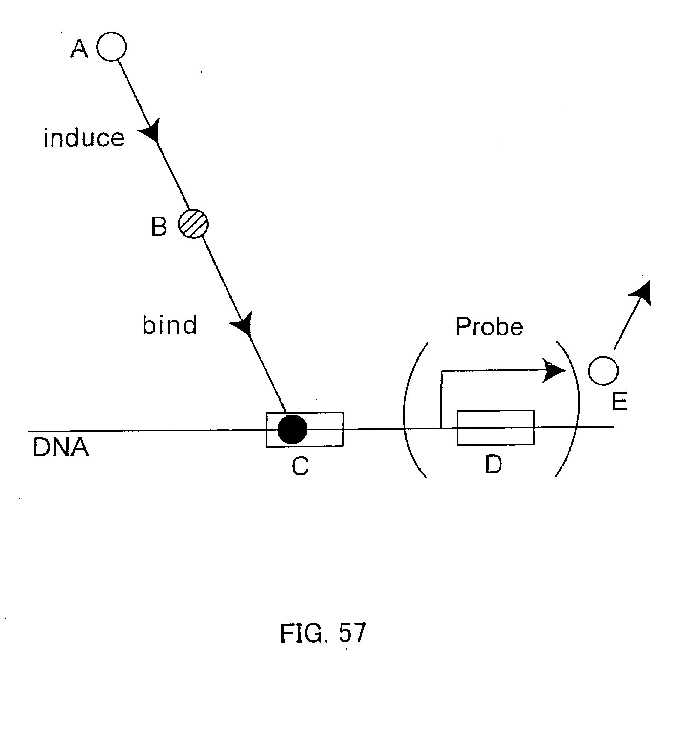

As shown in Figure 57, we often find transcription controls because we observe expressions of mRNA by DNA microarray analysis device 26. Figure 57 shows the signaling between proteins and genes: protein A induces protein B, protein B (which is transcription factor) binds to a promoter C (which is DNA) and then induces gene (probe) D, then gene D activate transcription of gene E (protein E). Consequently, we can use this flow of signaling as a defined conduction for drawing a pathway map, with keeping the necessary information.

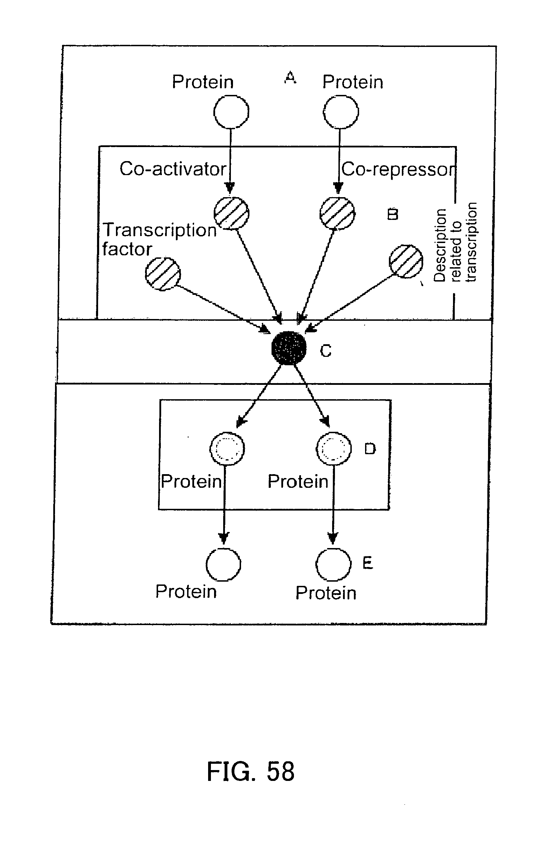

Figure 58 shows an example of the process of interactions that include a transcription factor (protein B) in the case of entering probe C (Note: Here promoter C and probe D in Figure 57 is treated as combined and denoted as probe C). Here, the term transcription factor means the factor that is necessary for starting transcription, and directly connects with DNA to control transcription (for example, Sp1, p53, NFkB, USF, sox9, etc.). We have Sin3, pRB, etc as a coactivator (transcription coactivator) and Sin3, pRB, etc as a corepressor (transcripotion corepressor). Coactivator and corepressor are factors that bind with transcription factors and induce or inhibit transcriptions. They do not bind directly with DNA, and function by forming a complex with other proteins. Furthermore, the descriptions in the text related to transcriptions are TNFa, IGF1, TGFB, BMP2, BMP9, etc. and although they are not transcription factors, but they have extremely important functions in transcriptions. Example of the description is "protein A activates the expression of E gene". Furthermore, even in the case where the process of interactions shows indirect relations such as A→B→C→D→E, if a description related to a transcription exists, it is considered to be an interaction related to a transcription.

Consequently, as shown in Figure 58, we can specify defined condition as the sequential flow of signaling represented as a set of binary relations: protein A binds with protein B (A→B), and protein B (a transcription factor) bind with probe (gene) C (B→C) (Here we suppose that C1=C2=C3=C4, in the following relations; promoter C1 activates transcription of gene C2, thus probe C3 measures mRNA of gene C2, and transcript of probe C3 is translate to protein C4), protein C activate protein D (C→D) , and moreover, protein D induces protein E (D →E). Consequently, we make a restriction in the interaction direction (direction of edges) based on relationships between subjective and objective of element names determined by natural language processing method. By the method as just described, we can reduce the size of the interaction map as can be seen the change from Figure 56 to Figure 59. In addition, the input defined conditions are stored in Data Control Unit 18.

Next, for protein names stored as experimental results, we extract binary relations between gene and protein names in reference to Dictionary 16 and Literature DB 14 (Step S142), and the extracted binary relations are stored in Binary Relation Storage Unit 19 (StepS143). The detailed explanation of the process is omitted because the process of Step S142 and S143 is the same as those of Step S221 and S222.

Next, for all of the gene names shown on experimental results, the system evaluates whether the extractions of binary relations are finished or not (Step S144). In cases where the extractions are not finished, the system goes back to Step S142 to extract binary relations of next protein names.

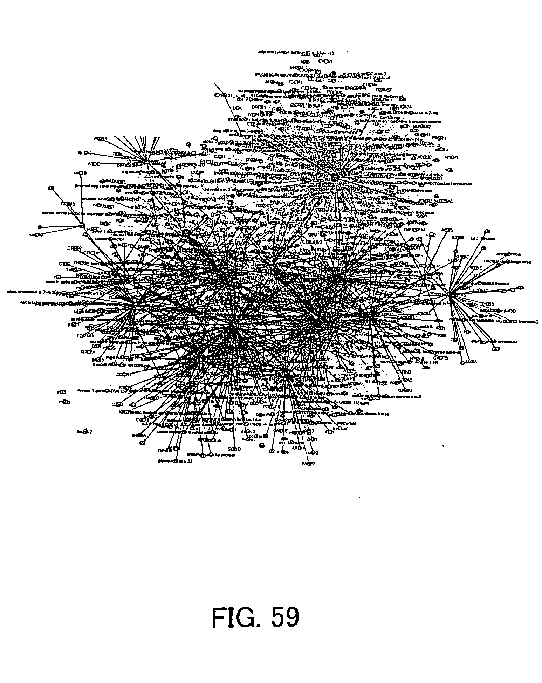

In the case where extractions of binary relations for all the protein names shown on experimental results are deemed to be finished, the pathway map is drawn based on the binary relations stored in Binary Relation Storage Unit 19 and the defined conditions stored in Data Storage Unit 18 (Step S145). In the case where the direction of edges is defined as one direction, for example, the pathway map (small one) is drawn as shown in Figure 59.

The Biomedical Literature Information Processing System concerning the eighth embodiment draws pathway maps based on defined conditions that define the drawing range of pathway maps. Consequently, the system can draw pathway maps using necessary information from extracted binary relations by specifying appropriate defined conditions.

In the Biomedical Literature Information Processing System concerning the eighth embodiment, using defined conditions for the pathway map, we can extracts the binary relations for smaller sized region as shown in Figure 59 from the large number of binary relations in the large sized map shown in Figure 56. Consequently, the system can extract small pathway maps that include necessary information from big pathway maps and obtain the information needed, that is, the pathway maps that contains the binary relations that users need to see.

In addition, the Biomedical Literature Information Processing System concerning the eighth embodiment can shorten time and draw pathway maps very quickly because the system draws small pathway maps that include necessary information based on the deifned conditions. The system makes it easy to visually understand binary relations between protein names shown as a pathway map.

In addition, in the Biomedical Literature Information Processing System concerning the eighth embodiment, by restricting the direction of edges, the smaller pathway map can be drawn. The system provides much smaller pathway map by imposing more defined conditions that restrict the direction of edges.

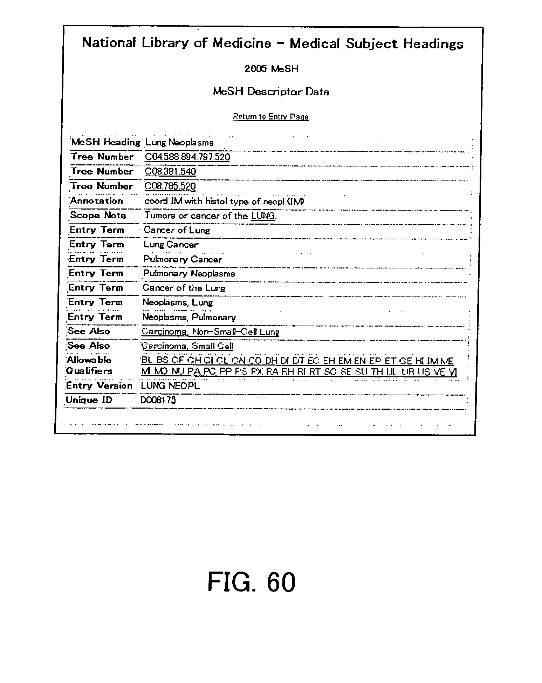

Here, in the medline, a public database that stores biomedical literature information, the database that stores information (mesh term) (for example, which disease the genes (proteins) and organs that are included in literature information are related to, or which cytoma (internal organ) the genes and the organs are related to, etc.) is formed. Consequently, we can store this mesh term in Literature DB14 and specify defined conditions using the stored mesh term (in reference to Figure60), and extract a small pathway from a big pathway map. That is, we extract nodes that have specific functions (for example, the node related to a specific disease such as cancer, the node related to a specific cytoma such as liver, etc.) in reference to a mesh term for the nodes (genes and proteins) that compose a big pathway map. Then we can draw a small pathway map using the extracted nodes and the edge that indicates the binary relation of the nodes. In this case, we can draw the pathway maps contains the nodes and interaction edges that directly relate to the specific disease, and we can see how the change interactions with development.

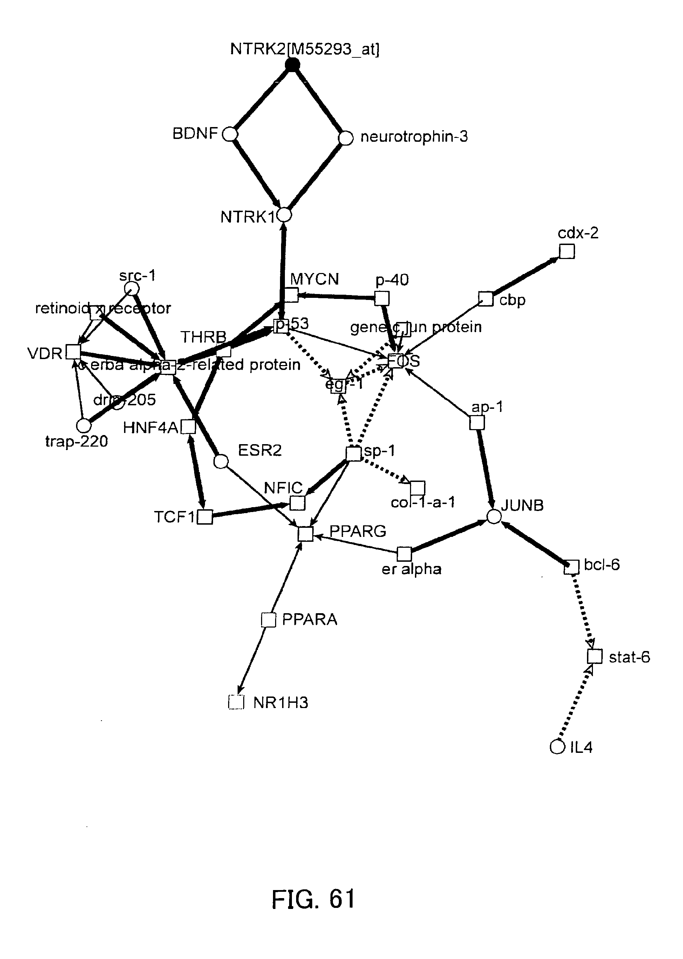

In the Biomedical Literature Information Processing System concerning the above eighth embodiment, from the pathway map whose direction of the edge is restricted, we can extract the pathway map whose range is more restricted. That is, we can draw pathway maps with the direction of edges and other defined conditions, such as restricting specific verbs in the binary relations. For example, for the pathway map shown in Figure 59 is restricted to only one direction of the edges, we can impose further restriction to verbs in the binary relations: we use only "bind" and "interact" interaction verbs and binary relations contained them, and draw a pathway map as shown in Figure 61. That is, the pathway map as shown in Figure 59 is the map that was obtained by imposing the restriction in edge directions, and the map includes many kinds of interactions. Consequently, by restricting verbs that indicate physical interactions between the neighbor nodes such as "bind" and "interact", we can obtain the pathway map as shown in Figure 61. In the pathway map shown in Figure 61, the 17α estradiol-specific interactions are indicated in bold solid line, the genistein-specific interactions are indicated in dot-line, and the common edges appearing both are indicated in thin solid line.

In addition, using multiple relations only, we can extract a small pathway map from a big pathway map. There are a large number of sentences in the texts in the literatures that provide binary relations, but the number of sentences in the texts of literatures that provide multiple relations including more than three proteins and genes is less than those that provide binary relations. Consequently, the extractions of the sentences that include at least more than three element names, and the mutual interactions thus obtained provide smaller sized pathway map. In addition, by restricting in using verbs of interactions to concerning control such as "induce", "inhibit", or "activate" in extracting multiple relations, we obtain information concerning control mechanisms that indicate non-physical, long-ranged, and semantic interactions. Alternatively, we can obtain information concerning protein complex with using the verbs that indicate physical interactions such as "bind", "interact", or "cooperative".

By using multiple relations we can extract a small pathway map from a big pathway map with restricting the range of network composed by extracting binary relations. That is, in the Biomedical Literature Information Processing System that is shown in Figure 1, operating Data Control Unit 10 as a multiple relation extracting means, we extract the multiple relations that indicate the relationships between more than three element names for the element names entered via Input Unit 12 in reference to the multiple relations stored in Data Control Unit 10. Next, operating Data Control Unit 10 as a binary relation extracting means, we extract binary relations for each element name extracted as having multiple relations with entered element names in reference to the binary relations stored in Binary Relation (Multiple Relation) Storage Unit 19. We can draw pathway maps based on the extracted multiple or binary relations by operating Data Control Unit 10 as a pathway map drawing means. In this case, by extracting binary relations after extracting multiple relations, we can select more important target for analyzing because the range of the relationships indicated by multiple relations that show relationships between more than three element names is smaller compared to the range of the relationships indicated by binary relations. We can do more exhaustive analysis to the target whose meaning is restricted by multiple relations by extracting binary relations after limiting analysis targets from semasiological information such as compound protein.

Suppose extracting multiple relations for instance, k-body (here k is positive integer) relations and k + 1-body relations. The more element names that compose multi-body (or multiple) relations, the more complex sentences that provide information about multiple relations, and then the less frequency the sentences appear. Therefore, the range of the network of the k + 1-body relations becomes narrower than that of k-body relations. But if the value of k becomes larger than some threshold value, the number of sentences becomes smaller, so we cannot see the network behavior composed of k-body interaction relations. Consequently, the values of k in the k-body relation should be k = 3, 4, 5, or 6 to obtain meaningful analysis results.

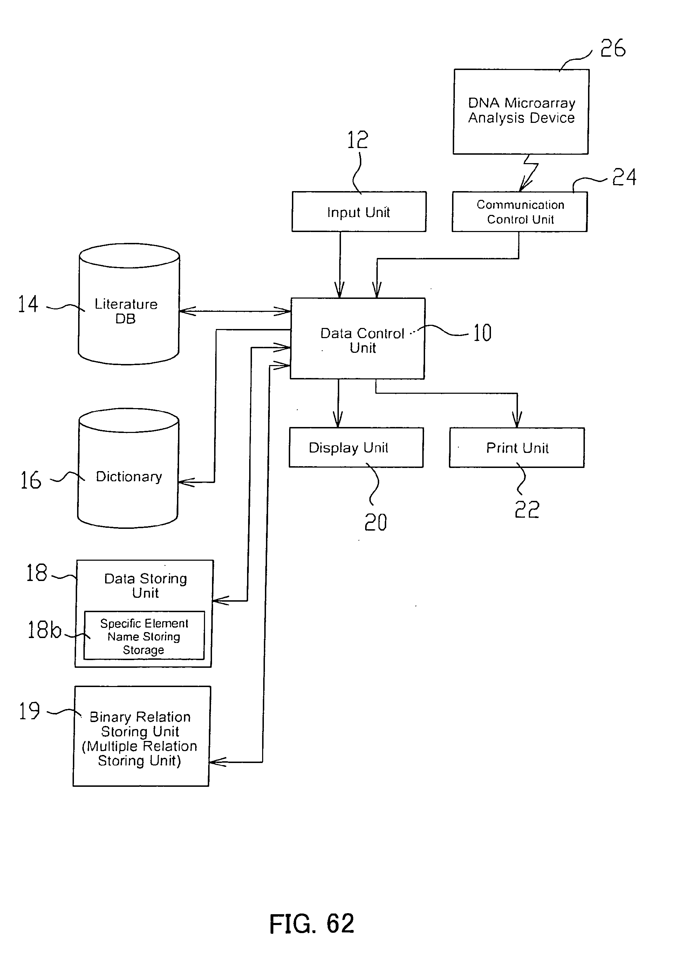

In addition, we can restrict the display of multiple relations related to specific element names that have interactions between plural element names (for example, display protein names that have binary relations with specific protein names) to draw a pathway map. Here protein names as nodes and interactions between protein names as edges. It is well known that specific protein nodes in the network have a vast number of edges, and these nodes are called hubs. The list representing hub proteins (the list of hub proteins) is stored in Specific Element Name Storage 18b set within Data Storage Unit 18 in advance, as shown in Figure 62. Then, we can change the display of edges which hub proteins have in Data Control Unit 10, which functions as a pathway map drawing means, and reference the list of hub proteins stored as specific element names in Specific Element Name Storage 18b. In addition, we omit the detailed explanation because the system architecture of the Biomedical Literature Information Processing System as shown in Figure 62 is the same as those concerning the first embodiment, except for adding Specific Element Name Storage 18b in Data Storage Unit 18.

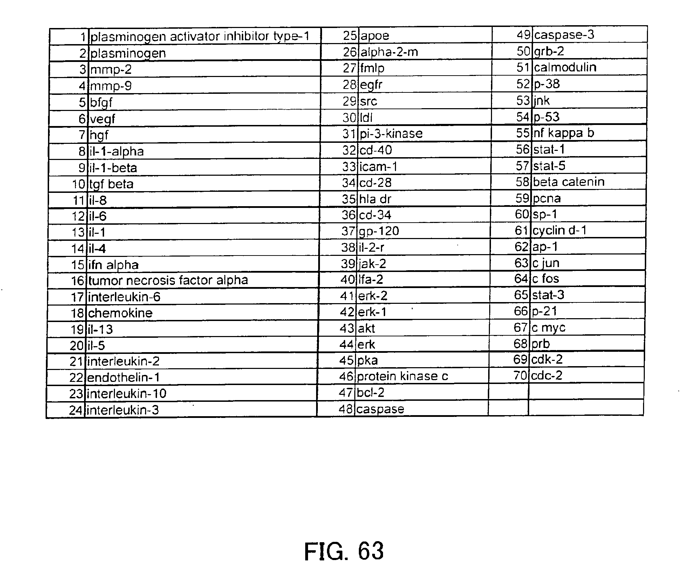

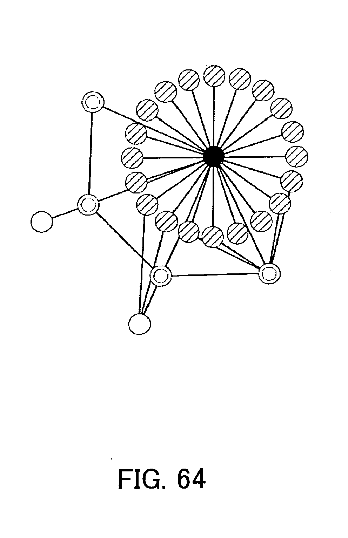

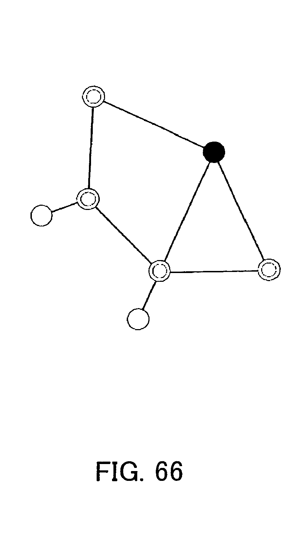

Here, for example, top 70 proteins in all proteins (in order of the number of edges) are stored as hub proteins (the list of hub proteins) in Data Storage Unit 18 as shown in Figure 63. As shown in Figure 64, we have trouble seeing the pathway map because hub proteins (black circle) have so many edges, and edges that hub proteins have and the other connected nodes (proteins) via the edges are displayed. In this case, by restricting direction of the interactions (direction of edges) about edges that hub proteins have to one direction (refer to Figure 66) or not displaying edges that hub proteins have as imposed by the defined condition (refer to Figure 66), displaying unnecessary edges and unnecessary nodes are avoided and we can make it easy to see pathway maps. In addition, in the case where the defined condition that changes the display of edges that hub proteins have, featured in the list of hub proteins, is shown, the process of extracting multiple relations based on hub proteins may be omitted by user's specification. In this case, we can shorten the whole processing time of extracting binary relations and reduce the stress on Biomedical Literature Information Processing System by abbreviating the extraction of multiple relations related to hub proteins that have multiple edges.

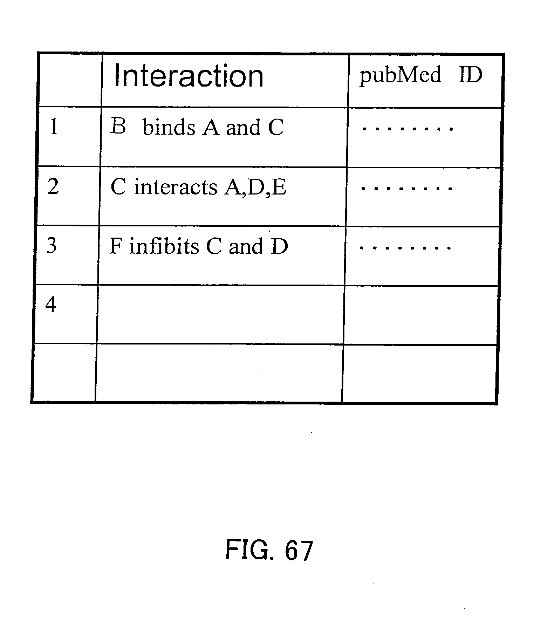

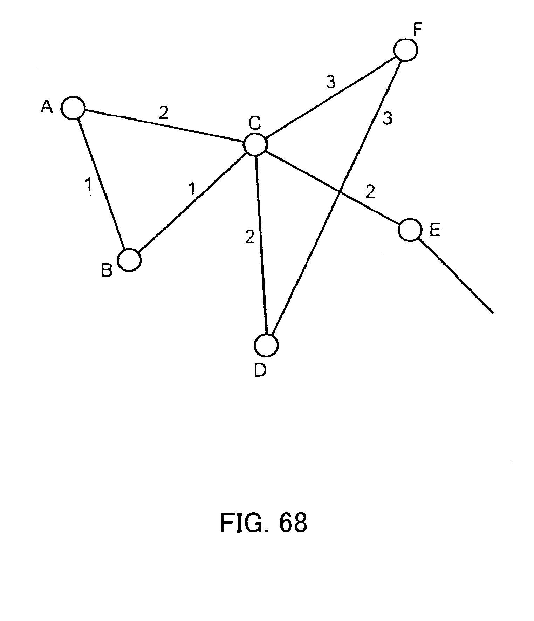

In addition, in Biomedical Literature Information Processing System concerning the above embodiment, in the case where multiple relations that include more than three element names are extracted, we can clarify the relationships between element names. For example, in the case where the interactions of the extracted multiple relations include more than three element names, the list that indicates relationships between element names is drawn up, and the list is stored in Data Storage Unit 18. That is, as shown in Figure 67, we make the list that shows the information (PubMedID), which indicates the locations of the literatures that the relationships on Literature BD14 are extracted and register the list in Data Storage Unit 10 to respond to the prescribed number (relationship identification number). In the case of drawing a pathway map in Data Control Unit 10 that functions as a pathway map drawing means, we can mark edges with relationship discerning number as shown in Figure 68 and draw a pathway map in reference to the list shown in Figure 67. In addition, when displaying a pathway map to show in Figure 68, by displaying the list for showing in Figure 67 together and referring to the list that users show in Figure 67, for example, we can make it easy to understand the following: 1) the relationship between element name B, A, and C show in Figure 68 is "protein B binds to A and C", 2) the relationship between element name C, A, D, and E is "C interact with A, D and E", and 3) the relationship between element name F, C, and D is "F inhibits a function of C and D". Moreover, in the list shown in Figure 67, by making hyperlink, for example, we can make it possible to refer to the literature that multiple relations are extracted in the part which PubMedID is shown on.

In addition, in Biomedical Literature Information Processing System concerning the above embodiment, in the case where the multiple relations that include more than three element names are extracted, we can allocate nodes according to the number of edges and categorize gene and proteins with a group of pathway function for drawing a pathway map. That is, when drawing a pathway map in Data Storage Unit 10 that stores various functions as a pathway map drawing means, we count the number of edges (multiple relations) that each node (gene and protein) has, and allocate the node that has the largest number of edges at the center. Next, around the node already located (in the circle centered on the node already located). We allocate nodes at an even interval in the order of the large number of edges. That is, the fewer the number of edges nodes have, the nodes are located upon a circle farther from the node at the center.

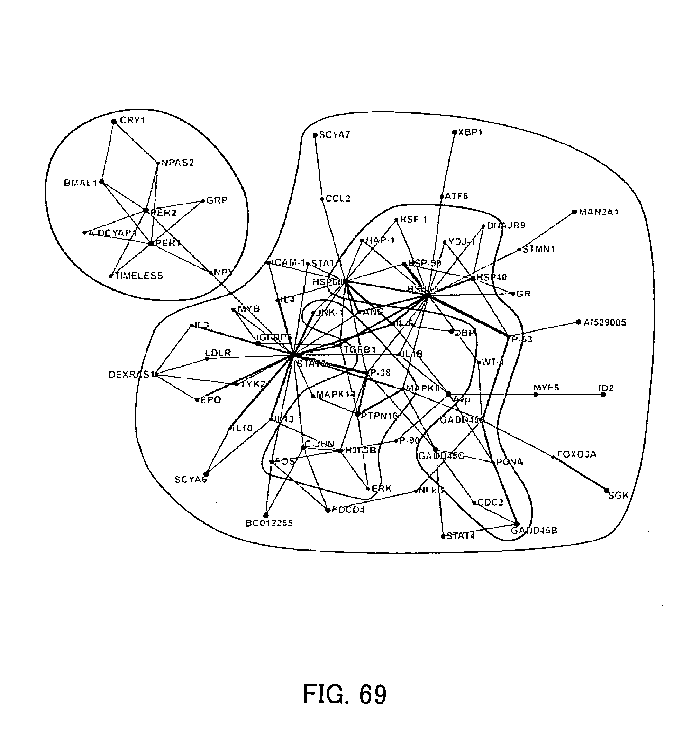

In a similar way, we can modify the configuration of nodes so as the closer the nodes according to the degree of the interaction represented by verb. Here the distances between nodes are adjusted according to the interaction strength obtained from the literature information. By locating the nodes in this way, pathway maps will be drawn as sets of groups so as each node in the group which has a defined relationship, such as some specific functions for the multiple relations, specific interactions that explain control, gathered similar functions. Then, within the pathway map drawn, taking the verb that shows the number of edges between nodes and relationship of nodes as a parameter, we make clustering nodes by general algorithms to form some functions or clusters (groups), as shown in Figure 69.

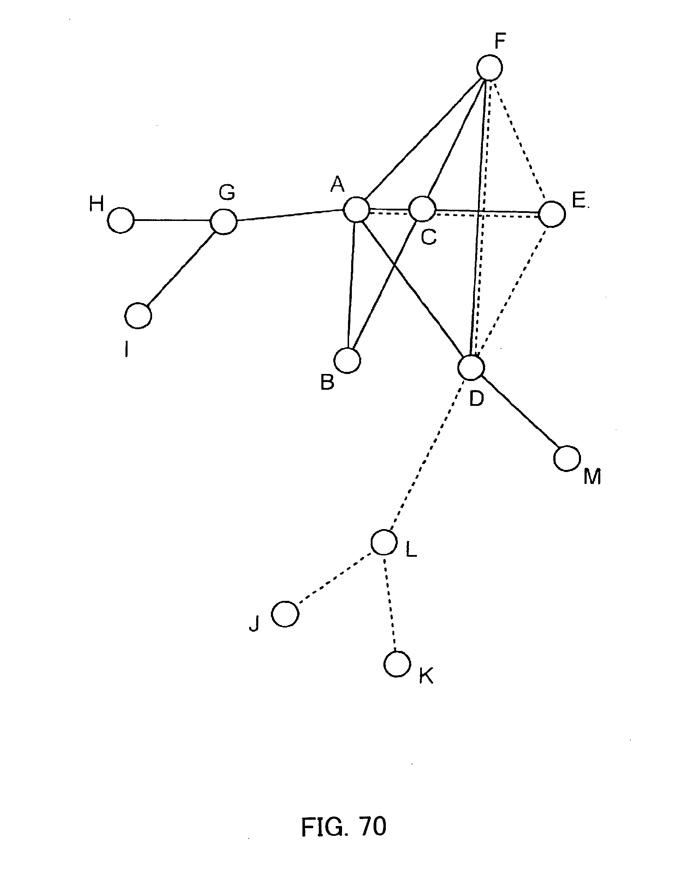

Furthermore, in the case where nodes are separated into groups that have defined function or groups that explain defined control, etc, we can display the nodes in the same group, cell type, for example. Within the group that explains the sense of time (such as cell cycle or circadian rhythms), it is separated into nodes related to brain, in reference to mesh term, and nodes related to liver. Next, the pathway map consisted of nodes related to brain (brain pathway map) and the pathway map consisted of nodes related to liver (liver pathway map) are drawn. Then, the nodes in common within brain pathway map and liver pathway map (nodes in common) are specified, and the nodes in common are located on the same position, locating each pathway map to overlap in an identifiable state. Figure 70 is a schematic chart in which the edges of brain pathway map is shown in a solid line, the edges of liver pathway map is shown in a broken line, and the nodes in common are located on the same position. In the case where the pathway map shown in Figure 70, the way to connect pathway to genes that control the sense of time (time genes), which are nodes in common, is G, H, and I within the brain, and L, J, K within the liver. Consequently, from the pathway map shown in Figure 70, brain and liver both have the function that controls the sense of time, but we can visually recognize that the regulatory pathway of specific genes differs entirely for brain and liver.

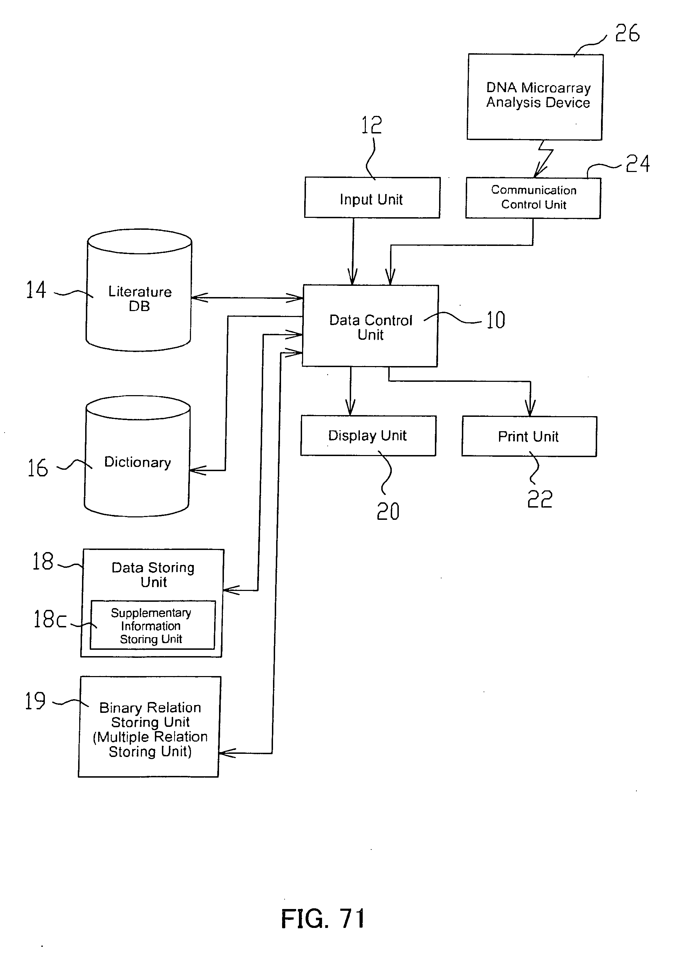

In addition, in Biomedical Literature Information Processing System concerning the above embodiment, we can draw a pathway map in reference to the supplementary information related to pathway maps. That is, as shown in Figure 71, supplementary information is stored in Supplementary Information Storage 18c set up in Data Storage Unit 18. And in Data Control Unit 10 that functions as a pathway map drawing means, we can draw a pathway map in reference to supplementary information stored in Supplementary Information Storage 18c when drawing a pathway map. In addition, we omit the detailed explanation because the system architecture of the Biomedical Literature Information Processing System shown in Figure 71 is the same as those concerning the first embodiment.

For example, we can display specific element names identifiable from other element names in reference to supplementary information. That is, the famous genes, such as Estrogen Receptor and Androgen Receptor, are often noted in two or three letters like "ER" or "AR" in literatures, but such omitted notations often differ in each field. Therefore, even if "ER" is noted in a literature, there is a possibility that "ER" does not always mean Estrogen Receptor.

Consequently, we collect element names whose number of characters is two or three beforehand, search cited literatures for each element name, categorize by field, and hierarchies by co-occurrence of element names and year of publication of the reference journal, etc. By sub typing, using statistics of frequency and graph theoretical analysis of element name network of more than 100 specific professionals who are users of literature information, and by synthesizing hierarchical element name information, we register beforehand supplementary information that handles element names in biomedical field as a whole in Supplementary Information Storage 18c set up in Data Storage Unit 18. Then we can refer to the supplementary information stored in Supplementary Information Storage 18c when drawing a pathway map, and we can draw user's attention by showing the configuration different from other genes in the case where extracted element names are included in supplementary information.

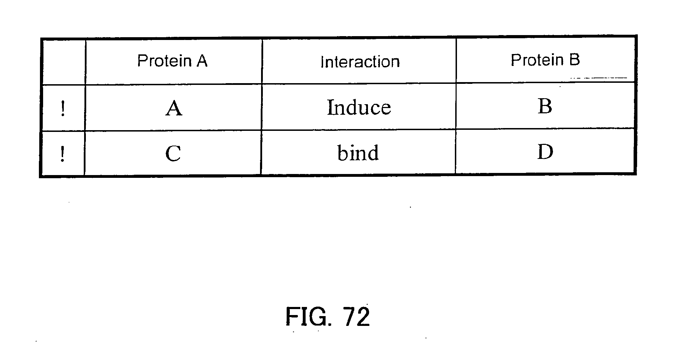

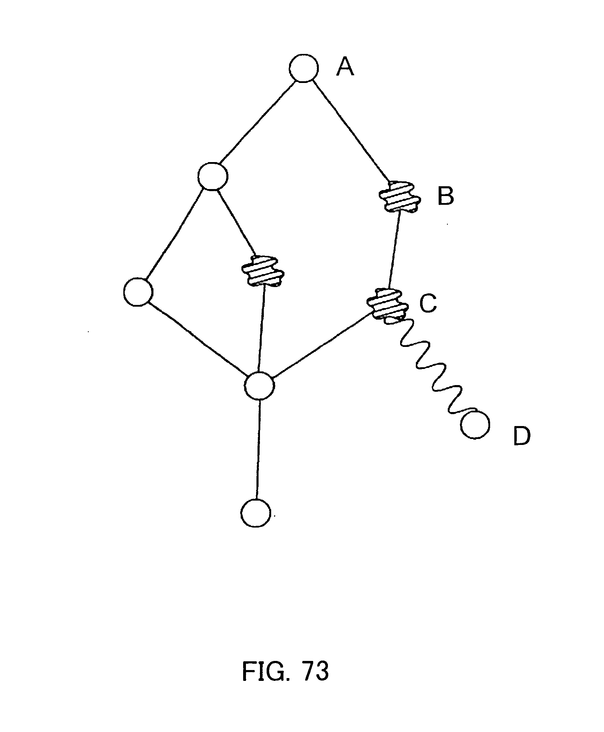

In addition, using the different form of the figure for displaying specific element names such as "ER" and "AR", we can make it enable to visually understand the possibility that the gene names erroneously indicate other elements. That is, for the element names that the event probability of error is high in searching literature information, we make up a table as shown in Figure 72 as supplementary information beforehand and register in Supplementary Information Storage 18c. When displaying a pathway map, we can draw user's attention by displaying the genes that the event probability of error is high with the figure of distorted circular configuration as shown in Figure 73, in reference to the table (supplementary information) shown in Figure 72 stored in Supplementary Information Storage 18c. In addition, we can draw user's attention by giving an exclamation mark to the table shown in Figure 72 as well as by displaying the edges that indicate the interactions by the genes that the event probability of error is high with broken lines as shown in Figure 73. Moreover, we can make the configuration upon displaying the element names that the event probability of error is high correspond to the developmental rate of error. For example, the higher the event probability of error is, the more distorted we can display the configuration.

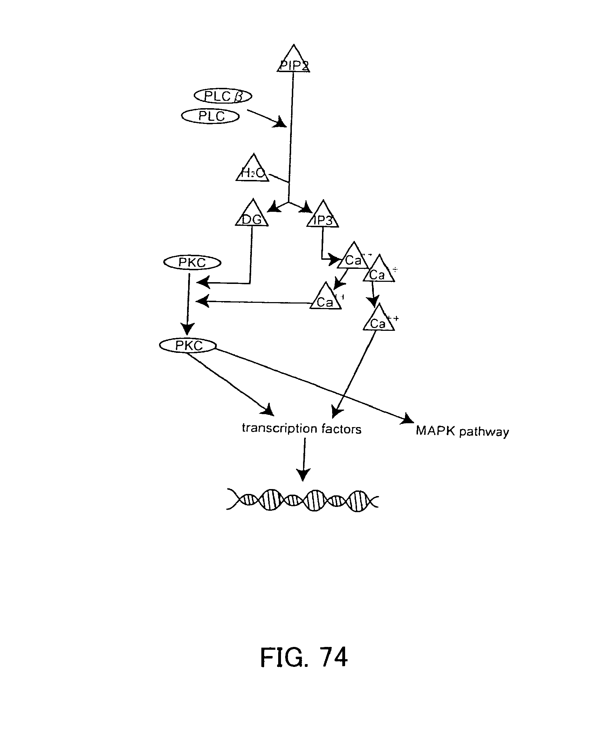

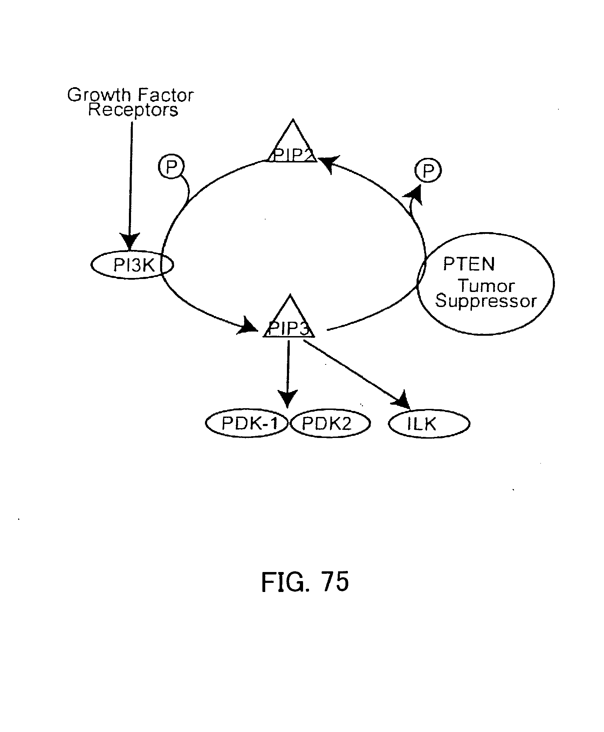

In addition, in Biomedical Literature Information Processing System concerning the above embodiment, we can display the important materials (not proteins or genes) in the process of interaction identifiable from proteins and genes. That is, we make the list that indicates the important materials in the process of interaction between genes/proteins (for example, the effects on interactions of phosphorylated, ubiquitination, methylation, mutation evolution, monoprotic polymorphism, permutation on chromosome, lipid, and carbohydrate) as supplementary information beforehand, and store the list in Supplementary Information Storage 18c set up in Data Storage Unit 18 (refer to Figure 71). When drawing a pathway map, we display the important materials contained in the list in reference to the list (supplementary information) stored in Supplementary Information Storage 18c. The example (not protein) of having a relationship with a signaling pathway is PIP2, IP3, Ca2⁺, ATP, GTP, AMP, and DG. Here, when PLC emerges, for example, DG, PIP2, H₂O, Ca²⁺ interact with IP3. Therefore, when entering proteins (indicate in circles) as shown in Figure 74, the materials whose relations are important but not proteins, DG RIP2, H₂O, Ca²⁺, and IP3 are displayed with triangle. In addition, when PI3K (phosphoinosiyol 3 phosphatase) emerges, P and PIP2 that are not proteins interact with PIP3. Therefore, we display these and P, PIP2, and PIP3 all together on a pathway map (refer to Figure 75).

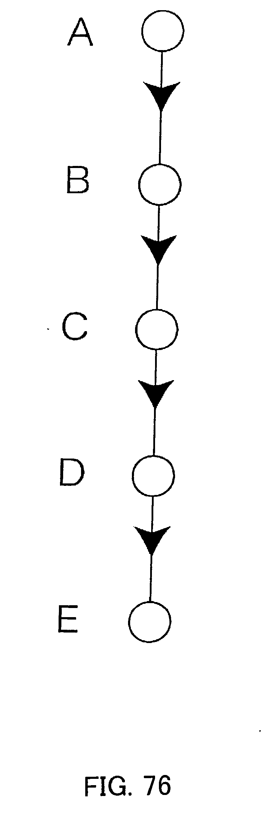

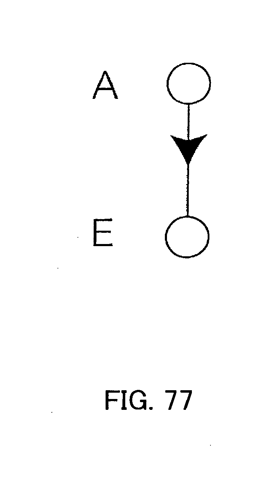

In addition, in Biomedical Literature Information Processing System concerning the above embodiment, we can draw a pathway map that includes interactions between element names that are omitted in literature information. For example, in the case of using the verbs such as "inhibit" or "induce", when protein A interacts with E via protein B, C, and D as shown in Figure 76, researchers often omit B, C, and D to describe, as shown in Figure 77,and describe as " A induces a function of E" or "A induces a function of E". Consequently, as shown in Figure 77, in case it is noted that the interactions to show in Figure 76 is omitted, we make the list (abbreviation list) that accommodates omitted notations and omitted contents as supplementary information beforehand, and store the supplementary information in Supplementary Information Storage 18c set up in Data Storage Unit 18 (refer to Figure 71). When drawing the pathway map, we can add omitted protein names, etc, in reference to the abbreviation list (supplementary information) stored in Supplementary Information Storage 18c.

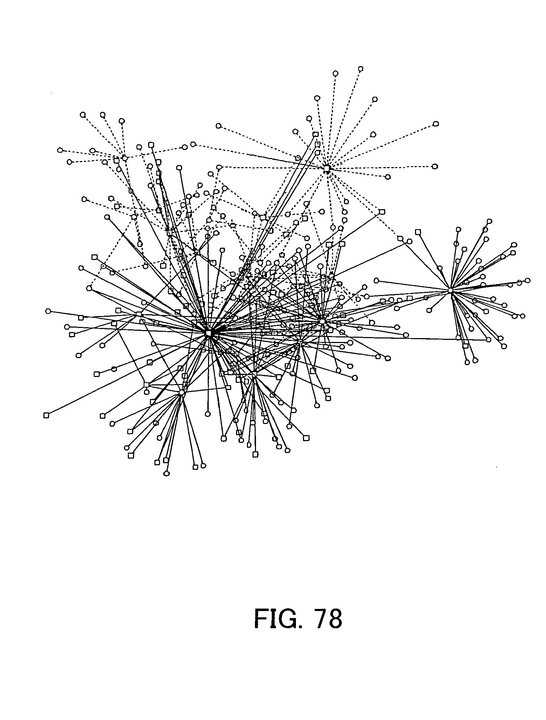

In addition, in Biomedical Literature Information Processing System concerning the above embodiment, we can draw the pathway that can compare different experimental results. For example, we make each experiment for the case that 17α estradiol concentration are 0.5 µg/kg and 1.0 µg/kg, and extract multiple relations based on each experimental result. Here, we calculate the union of sets of nodes and edges shown by the multiple relations extracted on the basis of the experimental results in the case of concentration 0.5 µ g/kg and those in the case of concentration 1.0 µ g/kg. Then, we draw the pathway map that allocates the common node in one position in the pathway map of the union of sets, that is, the node shown in the case of concentration 0.5µg/kg and that in the case of concentration 1.0 µg/kg (refer to Figure 78). In Figure 78, the edge that shows the case of concentration 0.5 µg/kg is displayed in broken line and the edge that shows the case of concentration 1.0 µg/kg is displayed in solid line.

As just described, by displaying two pathway maps in superimposed condition, we can make it easy to understand visually 1) the common edges and nodes, 2) the nodes and edges that emerge only in the case of concentration 0.5µg/kg, and 3) the nodes and edges that emerge only in the case of concentration 1.0 *µ*g/kg. In addition, in the above example, we can discern two pathway maps by displaying edges in solid line and broken line, but we can also display by using colors, for example, we can display the edge that composes the pathway map of concentration 0.5*µ*g/kg in blue and display the edge that composes the pathway map of concentration 1.0*µ*g/kg in purple.

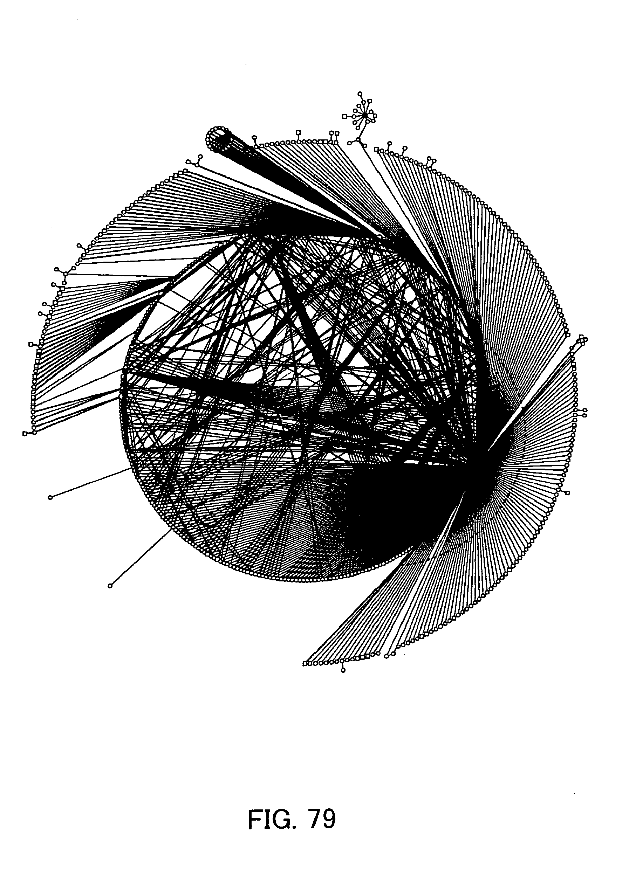

In addition, for the experimental results in the case where 17 αestradiol concentrations differ, for example, we can display the specific node in a visually-prehensible condition from the experimental result in the case of concentration 0.5 µg/kg and in the case of concentration 1.0 µg/kg. That is, we draw a pathway map by allocating the node with a single edge (displayed in white circle on the figure) outside the prescribed circle (refer to Figure 79). Here, the fact that the number of edge is one indicates that upon the experimental result of differing concentration, the node expresses only in the case of either concentration, and indicates that only one relation is extracted as a multiple relation with other nodes. Consequently, the nodes that are anomalous genes/proteins are arranged outside the prescribed circle, and we can recognize at a glance whether the genes/proteins are anomalous or not on the basis of the allocation.

In addition, in the case of extracting multiple relations, such as binary relations between proteins for example, in Biomedical Literature Information Processing System stated above, for the verb "bind", it is often unclear whether two proteins are directly connected or two proteins are connected via other proteins as a result. For example, even if the case is "protein A", "bind", "protein B" as an actual result that "protein A" binds to "protein C" and "protein C" binds to "protein B", only "protein A", "bind", "protein B" is often featured in literatures. In addition, it is recognized that the experimental result is "protein A", "bind", "protein B", but it is not clear whether the process is done via any proteins in between or not, and often only the clear parts ("protein A", "bind", "protein B") are featured. Consequently, in cases where the verb that indicates multiple relations is "bind", we can display the information that shows whether the function is direct or indirect (the function via any proteins) with a pathway map.

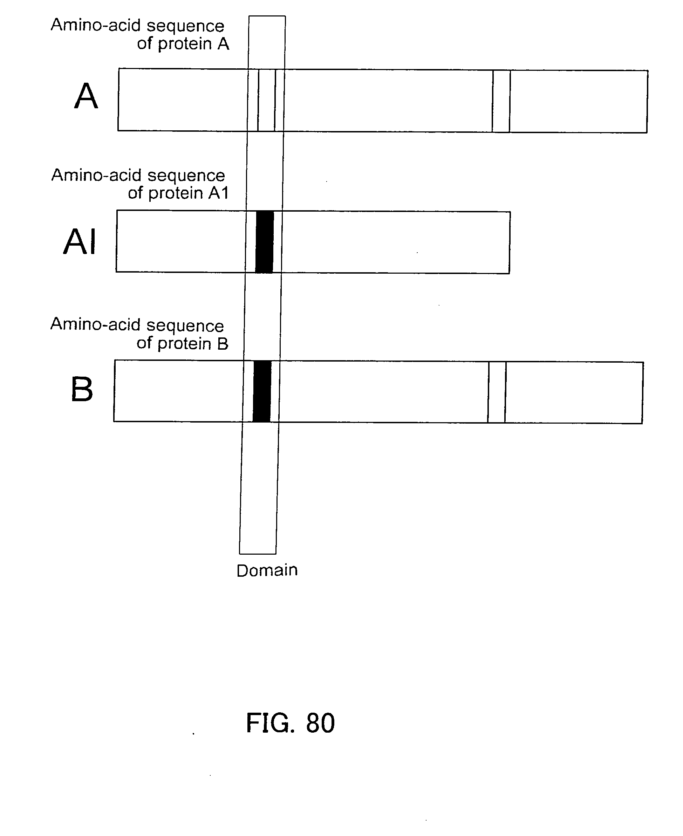

Here, proteins have domain structures (refer to Figure 80), and it is known that the protein that has certain domain structure directly binds to the proteins, which has a domain structure which responds to the domain structure. That is, the domain structures which respond to each structure exists, and it is known that a certain protein directly binds to the protein that has a domain structure which responds, but doesn' t directly bind to the proteins that doesn' t have a domain structure which responds. Consequently, by storing the information that shows response relations between domain structures of proteins as supplementary information in Supplementary Information Storage Unit 18c set up in Data Storage Unit 18 beforehand (refer to Figure 71), we can judge whether the function of "bind" is direct or not by using the stored supplementary information. For example, in cases where the domain structure of protein B shown in Figure 80 is "SH2", if protein A1 of protein A and protein A1 has the domain structure "SH2", we can expect that protein B has a high probability of binding directly to protein A1. In addition, even in cases where the function of "bind" is deemed to have a high probability of being indirect, we can indicate some possible proteins that have a high probability of intervening between proteins in reference to supplementary information.

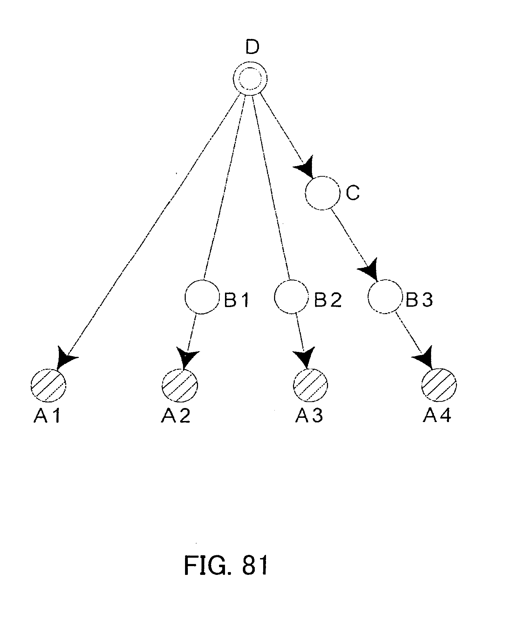

In addition, in Biomedical Literature Information Processing System concerning the above embodiment, we can display the pathway of interactions to proteins input as experimental results. That is, in Biomedical Literature Information Processing System, if we extract binary relations (multiple relations) and store the binary relations (multiple relations) in Binary Relation (Multiple Relation) Storage Unit, we can display a pathway of interactions in reference to binary (multiple) relations stored in the Binary Relation (Multiple Relation) Storage Unit. For example, as shown in Figure 81, in reference to binary (multiple) relations stored in the Binary Relation (Multiple Relation) Storage Unit, we search proteins (protein B1 - B3, protein D) that act on the entered proteins (protein A1 - A4). Next, in reference to binary (multiple) relations stored in the Binary Relation (Multiple Relation) Storage Unit, we search proteins that act on the searched proteins (protein B1 - B3, and protein D). Here, as shown in Figure 81, in cases where there is no protein that acts on protein D, we finish the process of searching proteins that act on protein D.

Next, protein D is searched as a protein that acts on protein B1 or protein B2, and protein C is searched as a protein that acts on protein B3. At this time, as described above, we finish the process of searching proteins that act on protein D because there is no protein which acts on protein D. At the same time, we search proteins that act on protein C in reference to binary (multiple) relations stored in the Binary Relation (Multiple Relation) Storage Unit. As shown in Figure 81, protein D is searched as a protein that acts on protein C, we finish the process of searching. And by referring to the pathway of the interaction shown in Figure 81, we can understand the shortest path of the interaction.

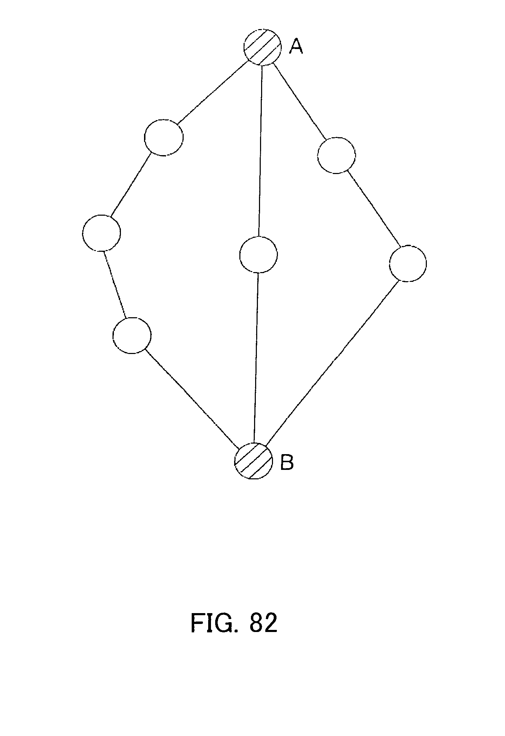

In addition, even if protein B is extracted as having binary relation with protein A, there is a possibility that other proteins intervene between protein A and protein B as described above. In such a case, we can display the pathway of the interaction that has a possibility of intervening between protein A and protein B, in reference to the binary relations (multiple relations) stored in Binary Relation (Multiple Relation) Storage Unit (refer to Figure 82).

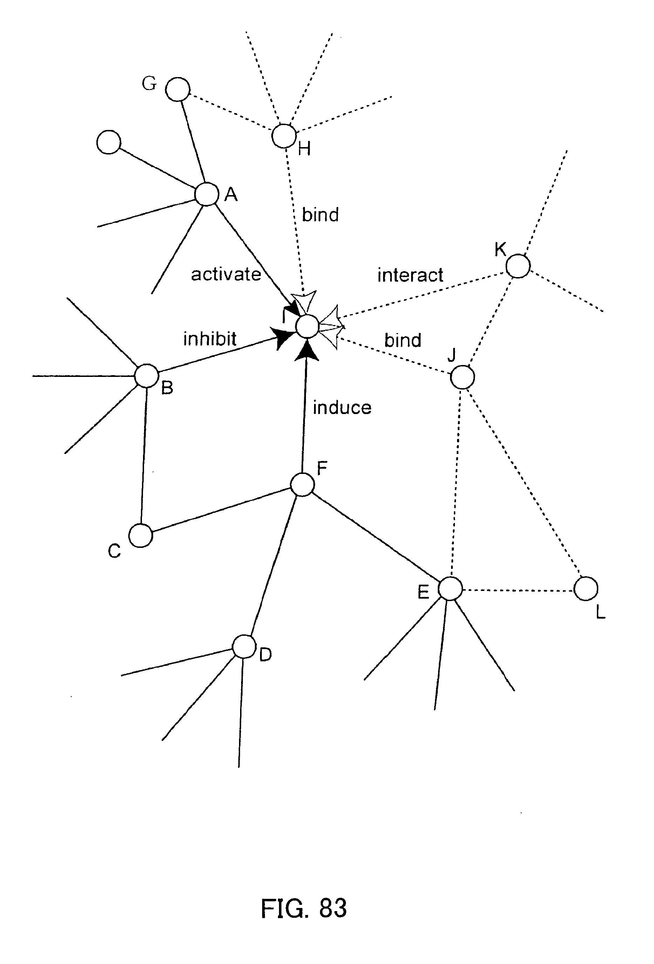

In addition, in Biomedical Literature Information Processing System concerning the above embodiment, we can display the nodes that counteract interactions in making the discernment possible. For example, the specific pathway map (pathway map of medicine A) are drawn for medicine 1 that indicates the binary relations extracted based on the proteins expressed to medicine A, and the specific pathway map (pathway map of medicine B) are drawn for medicine 2 that indicates the binary relations extracted based on the proteins expressed to medicine B. Here, as shown in Figure 83, we display the pathway map of medicine 1 that edges are indicated in solid line and that of medicine 2 that edges are indicated in broken line at the same time. From the pathway map shown in Figure 83, we can find that the following nodes and interactions exist: 1) node A, B, C, F, and D and interactions (edges) that emerge only in case of the either medicine, 2) node H, K, J, and L and interactions (edges), and 3) node G, I, and E that have interactions that respond to both medicines. In this case, node G, I, and E that have competing interactions that respond to both medicines have a possibility to counteract the effects from both medicine. Consequently, we specify the node that is affected by the counteract effect by counting the number of edges for each node by medicine. We can estimate the effects of the surrounding area on the specified node, based on the number of edges for each specified node and the contents of the interactions indicated by each edge. That is, in the case of showing Figure 83, we can find that node I is not directly affected by medicine A, because the edges of the interaction between node A - node I is "activate", the edges of the interaction between node B - node I is "inhibit", and the edges of the interaction between node F - node I is "induce". On the other hand, we can find that the edges of the interaction between node H - node I is "bind", the edges of the interaction between node K - node I is "interact", the edges of the interaction between node J - node I is "bind", and they are directly interacted by medicine 2. In addition, in Figure 83, we explained as an example of the nodes in common in the pathway map of medicine 1 and that of medicine 2, but we can specify the node in common in the pathway map composed of the proteins that express in normal cells and the pathway map composed of the proteins that express in diseased cells such as cancer.

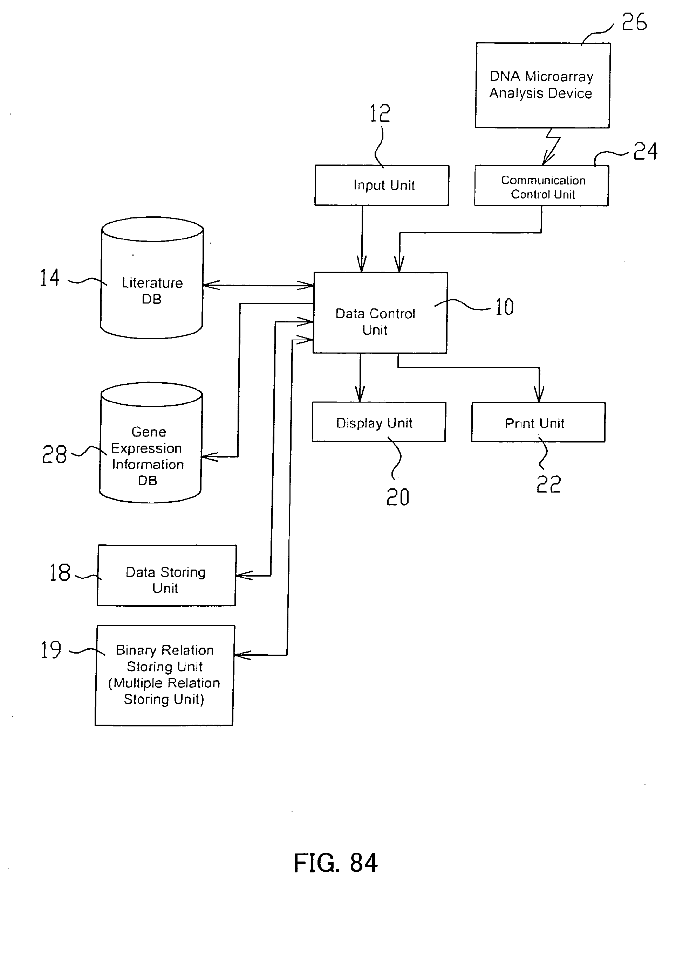

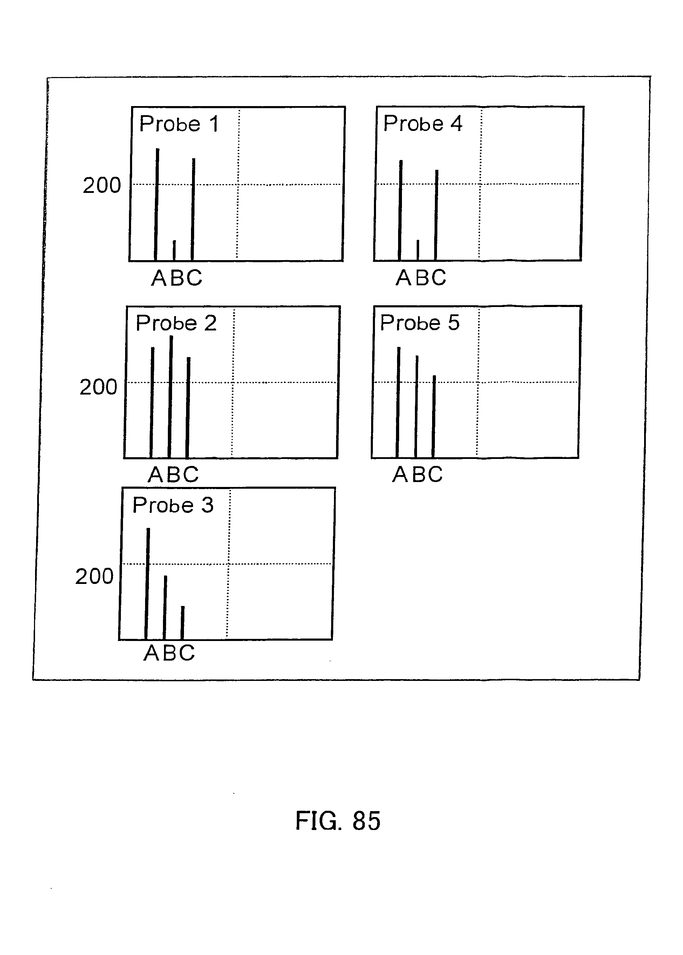

Next, we explain the ninth embodiment. Figure 84 is the flow chart to indicate the outline of the block configuration Figure of Biomedical Literature Information Processing System concerning the ninth embodiment. Biomedical Literature Information Processing System concerning this embodiment has Gene Expression Information DB28 that stores gene expression information (probe expression information) that are actual experimental results as a substitute for Dictionary 16 that Biomedical Literature Information Processing System concerning the first embodiment. The other configurations are omitted because those system configurations are the same as the Biomedical Literature Information Processing System concerning the first embodiment. In addition, Gene Expression Information DB28 stores actual experimental results (gene expression information that are the results of the experiments actually done), for example, the representation to organs A - C related to probe 1 - 5 as shown in Figure 85.

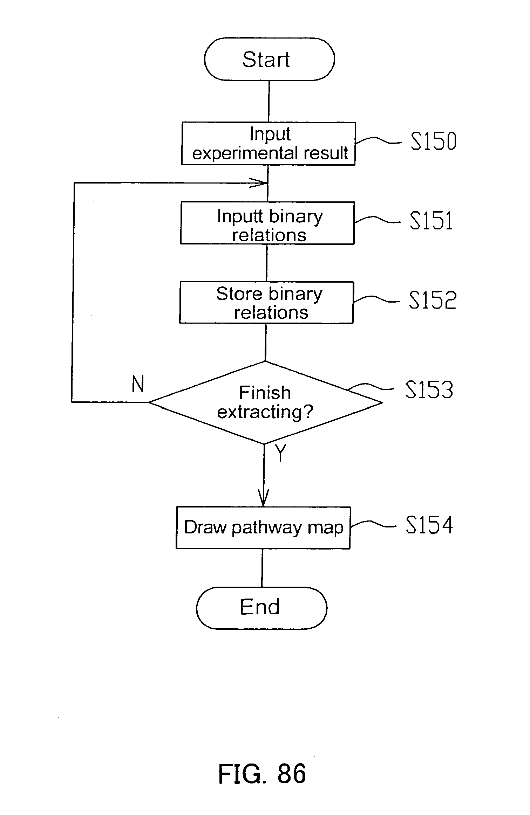

Next, we explain the process of drawing pathway maps on Biomedical Literature Information Processing System concerning the ninth embodiment in reference to the flow chart of Figure 86. In the Biomedical Literature Information Processing System concerning this embodiment, we verify the actual experimental results based on the literature information. That is, it is not known whether the proteins (partner proteins) that indicate interactions obtained by literature actually express by experiments or not. With that, we determine if the proteins actually express at mRNA level or not by using probe expression information of each organ stored in Gene Expression Information DB28.

First, Data Control Unit 10 of the Biomedical Literature Information Processing System obtains experimental results (Step S150) . The detailed explanation of the process is omitted because the process of Step S150 is the same as those of Step S130 in Figure 43. In addition, in what follows, we will explain by taking the case of verifying the expression to the organ A - C of the obtained probe 1-5 (obtained as experimental results) as an example.

Next, we extract binary relations in reference to Literature DB and Gene expression Information DB28 (Step S151), and store the extracted binary relations in Binary Relation Storage Unit 19 (Step S152). The detailed explanation of the process is omitted because the process of Step S150 - S151 is the same as those of Step S132 - S133 in Figure 43.

Next, we evaluate whether extractions of binary relations for all the probes shown on experimental results are finished or not (Step S153), and in the case where the extractions are not finished for all binary relations, we go back to Step S1511 to extract the binary relations of next probes.

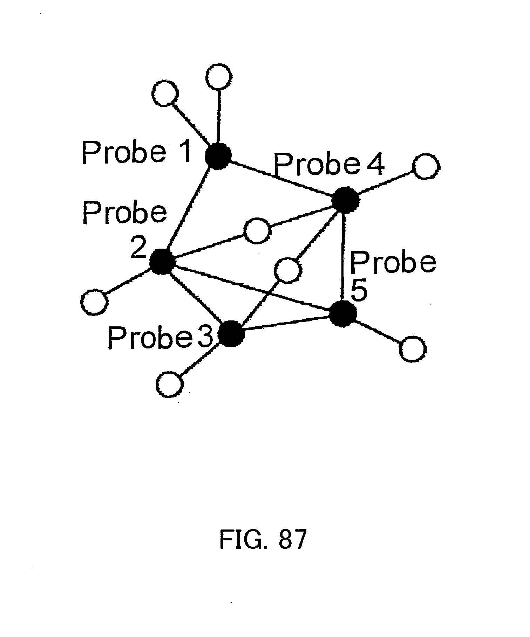

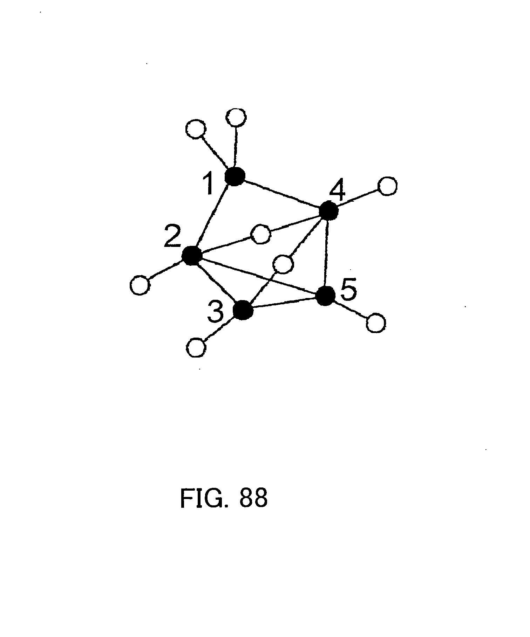

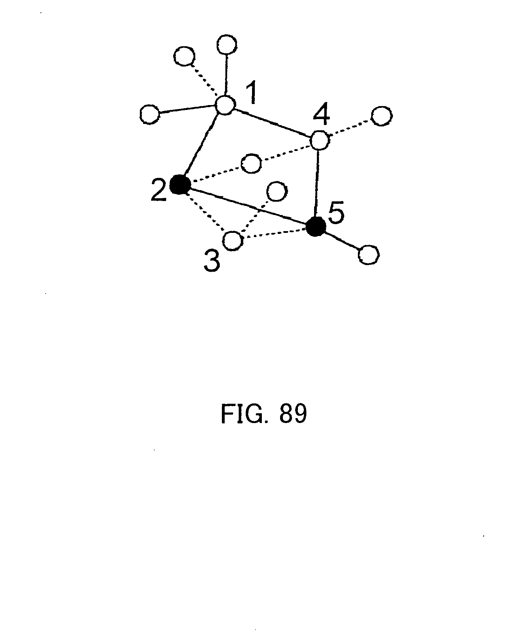

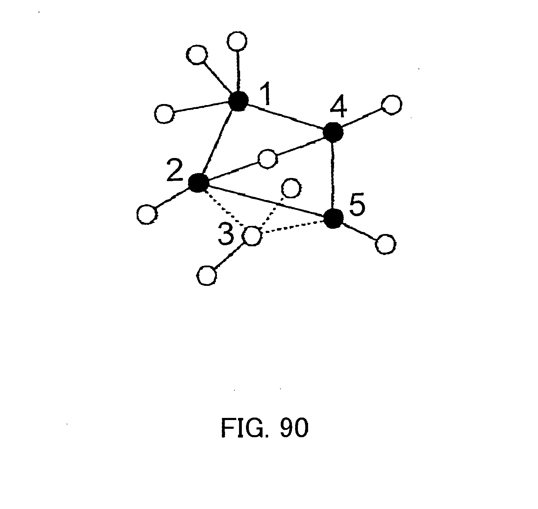

In the case where the extractions of the binary relations for all the protein names shown on the experimental results are deemed to be finished in Step S153, the pathway map is drawn based on the binary relations stored in Binary Relation Storage Unit 19 (Step S154). For example, the representation to the organ A -C concerning probe 1 - 5 is as shown in Figure 85, and in Biomedical Literature Information Processing System, the pathway map in the case of entering probe 1 - 5 is as shown in Figure 87. In this case, the probes expressing in organ A when setting the threshold at 200 is, as shown in Figure 85, all of probe 1 - 5. Consequently, the pathway map that indicates expressing probes in black circle as an organ A-specific pathway map is drawn (refer to Figure 88). In addition, the proteins expressing in organ B when setting the threshold at 200 is, as shown in Figure 85, probe 2 and probe 5. Consequently, the pathway map that indicates expressing probes in black circle as an organ B-specific pathway map is drawn (refer to Figure 89). In the same way, the pathway map as shown in Figure 90 is drawn as an organ C-specific pathway map. Furthermore, other than the pathway maps specific to an organ as shown in Figure 88 - 90, we can draw pathway maps dependent on the derivation of whether the cell is cancer or not, etc.

In the Biomedical Literature Information Processing System concerning the ninth embodiment, we can examine the actual experimental results based on literature information, because the system draws pathway maps based on the multiple relations extracted in reference to Gene Expression Information DB that stores gene expression information and Literature DB. That is, in the case where the pathway map dependent on an organ-specific pathway map and derivation of cell is drawn, we can do various analyses by analyzing and organizing drawn pathway maps. For example, we can extract different and common points on pathway maps of each organ and pathway maps of cancer and those of non-cancer. Consequently, we can draw the pathway map of probes expressed in specific organs by combining the data of experimental results (for example, Gene Expression Information Database) and literature database (the database of literature information).

In addition, in the above embodiment, we have extracted multiple relations for the literatures of biomedical field, based on the verbs that indicate interactions between elements, and have drawn pathway maps by setting protein and gene names as elements (nodes). We can also draw interactions between elements (nodes) on pathway maps for the literatures in the field of social science. In this case, we can indicate human relationships (relative, blood relationship, lover, married couple, friends, and family name) and personal connections on pathway maps by setting a "human" in literatures as an element (node) and extracting multiple relations based on the verbs that indicate interactions between elements and by drawing pathway maps. These pathway maps can be effectively used as information to figure out the human relationships and personal connections in the field of sports, movies, and politics.

In addition, we can draw interactions between elements (nodes) on pathway maps for the literatures of economic field. In this case, we can indicate relationships between companies (capital, business tieup, flow of money, and personal relationships), capital ties, etc. on pathway maps by setting a company name in literatures as an element (node) and extracting multiple relations based on the verbs that indicate interactions between elements and by drawing pathway maps. These pathway maps can be effectively used as one unit of information to make decisions in business and stock market.

In addition, we can draw interactions between elements (nodes) on pathway maps for the literatures of the military field. In this case, we can indicate background between cases, organs, cultures, economy, and personal relationships, etc. on pathway maps by setting a case name in literatures as an element (node) and extracting multiple relations based on the verbs that indicate interactions between elements and by drawing pathway maps. These pathway maps can be effectively used as information for analyzing information, analyzing historical information, and making decisions.

In addition, we can draw interactions between elements (nodes) on pathway maps for the literatures of the urban planning field. In this case, we can indicate relationships of electric power, water line, sewage, oil, and traffic on pathway maps by setting City name in literatures as an element (node) and extracting multiple relations based on the verbs that indicate interactions between elements and by drawing pathway maps. These pathway maps can be effectively used as information to make decisions in business and stock market.

In addition, we can draw interactions between elements (nodes) on pathway maps for the literatures of the legal field. In this case, we can indicate relationships between letters and systems of law on pathway maps by setting the law name in literatures as an element (node) and extracting multiple relations based on the verbs that indicate interactions between elements and by drawing pathway maps. These pathway maps can be effectively used as information to make decisions in business and politics.

In the above explanation concerning this invention, we have made an explanation for English-language literatures, but we can apply these to various languages (for example, Russian, Chinese, Korean, Japanese, Latin, etc.) that are used in history or at the present day by using the standard technology of the current natural language processing in the same way.

### INDUSTRIAL APPLICABILITY

As stated above, the literature information processing system of this invention is suitable for analyzing literature information by natural language processing and expeditiously putting analysis results.

## Claims

1. A literature information processing system, **characterized by** comprising:
a dictionary to store data of element names and verbs indicating mutual interaction relations between the element names,
a literature database to store a large number of data for literature information,
an input means to enter plural element names,
a multi-body interaction relations extracting means to extract multi-body interaction relations for every plural element name entered in reference to the above dictionary or the above literature database,
an overlapping part extracting means to extract overlapping parts of the multi-body interaction relations extracted for each of plural element names, and
a pathway map drawing means to draw a pathway map indicating the overlapping parts extracted by the above overlapping part extracting means as an information.

2. A literature information processing system, **characterized by** comprising:
a dictionary to store data of element names and verbs indicating mutual interaction relations between the element names,
a literature database to store a large number of data for literature information,
an input means to enter plural element names,
a decision making means to decide whether multi-body interaction relations between the above element names are extracted or not,
a multi-body interaction relations extracting means to extract the multi-body interactions between the element names whose the multi-body interaction relations are deemed not to be performed searching by the above decision making means in reference to the above dictionary or the above literature database, and
a pathway map drawing means to draw a pathway map on the basis of the multi-body interaction relations extracted by the above multi-body interaction relations extracting means.

3. The literature information processing system as defined in claim 1 or 2, **characterized in that** the above dictionary also stores noun phrases and adjective phrases indicating mutual interaction relations between the element names.

4. A literature information processing system, **characterized by** comprising:
a literature database to store a large number of data for literature information,
an input means to enter plural element names,
a multi-body interaction relations extracting means to extract multi-body interaction relations on the basis of verbs which indicate mutual interaction relations between the above element names in reference to the above literature database for each of plural element name entered,
an overlapping part extracting means to extract overlapping parts of the multi-body interaction relations extracted for each of plural element names, and
a pathway map drawing means to draw a pathway map indicating the overlapping parts extracted by the above overlapping part extracting means as one unit of information.

5. The literature information processing system as defined in claim 4, **characterized in that** the above multi-body interaction relations extracting means also extracts multi-body interaction relations on the basis of noun phrases and adjective phrases indicating mutual interaction relations between the element names.

6. A literature information processing system, **characterized by** comprising:
a literature database to store a large number of data for literature information,
an input means to enter plural element names,
a decision making means to decide whether multi-body interaction relations between the above element names are extracted or not, on the basis of verbs indicating mutual interaction relations between the above element names,
a multi-body interaction relations extracting means to extract the multi-body interaction relations between the element names whose the multi-body interaction relations are deemed not to be performed searching by the above decision making means in reference to the above literature database, and
a pathway map drawing means to draw a pathway map on the basis of the multi-body interaction relations extracted by the above multi-body interaction relations extracting means.

7. The literature information processing system as defined in claim 6, **characterized in that** the decision making means discriminates whether the extraction of the multi-body interaction relations is done or not on the basis of noun phrases or adjective phrases indicating mutual interaction relations between the element names.

8. The literature information processing system as defined in any one of claims 1 to 7, **characterized in that** the multi-body interaction relations extracting means extracts the multi-body interaction relations between the element names which are previously searched and deemed to have multi-body interaction relations with the element names entered by the above input means and re-extracts the multi-body interaction relations of the element name extracted.

9. The literature information processing system as defined in claim 8, **characterized by** further comprising the extracting range specifying means to decide the range of extraction of the multi-body interaction relations by the above multi-body interaction extracting means on the basis of the element names entered by the above input means.

10. The literature information processing system as defined in claim 8 or 9, **characterized in that** the above pathway map drawing means determines and indicates the element names entered by the above input means and the previously obtained element names as a result of the searching the multi-body interaction relations from the entered element names using the above input means by the multi-body interaction relations extracting means.

11. The literature information processing system as defined in any one of claims 1 to 10, **characterized by** further comprising a multi-body interaction displaying means to display the multi-body interaction relations extracted by the above multi-body interaction relations extracting means, and in that the above multi-body interaction displaying means can also discriminate and display the multi-body interaction relations in the affirmative expression and those in the negative expression in the text.

12. A literature information processing system, **characterized by** comprising:
a dictionary to store data of element names and verbs indicating mutual interaction relations between element names,
a literature database to store a large number of data for literature information,
a first multi-body interaction relations extracting means to extract multi-body interaction relations for every plural element name entered in reference to the above dictionary or the above literature database,
a multi-body interaction storing means to store the multi-body interactions extracted by the above first multi-body interaction relations extracting means,
an input means to enter plural element names,
a second multi-body interaction relations extracting means to extract the multi-body interaction relations for every plural element name entered in reference to the multi-body interaction relations stored in the above multi-body interaction storing means, and
an overlapping part extracting means to extract overlapping parts of multi-body interaction relations extracted by the second multi-body interaction relations extracting means,
a pathway map drawing means to draw a pathway map indicating the overlapping parts extracted by the above overlapping part extracting means as one unit of information.

13. A literature information processing system, **characterized by** comprising:
a dictionary to store data of element names and verbs indicating mutual interaction relations between the element names,
a literature database to store a large number of data for literature information,
a first multi-body interaction relations extracting means to extract multi-body interaction relations for every plural element name entered in reference to the above dictionary or the above literature database,
a multi-body interaction storing means to store the multi-body interactions extracted by the above first multi-body interaction relations extracting means,
an input means to enter plural element names,
a decision making means to decide whether the multi-body interaction relations between the above element names are extracted or not,
a second multi-body interaction relations extracting means to extract the multi-body interaction relations of the element names whose multi-body interaction relations are deemed not to be done by the above decision making means in reference to the multi-body interaction stored by the above multi-body interaction storing means, and
a pathway map drawing means to draw a pathway map on the basis of the multi-body interactions extracted by the above second multi-body interaction relations extracting means.

14. The literature information processing system as defined in claim 12 or 13, **characterized in that** the dictionary also stores noun phrases and adjective phrases that indicate mutual interaction relations between the element names.

15. The literature information processing system as defined in any one of claims 12 to 14, **characterized in that** the above second multi-body interaction relations extracting means extracts the multi-body interaction relations for the element names as having the multi-body interaction relations with the element names entered by the above input means and also extracts the multi-body interaction relations for the above-extracted element names.

16. The literature information processing system as defined in claim 15, **characterized by** further comprising an extracting range deciding means that decides the extraction range of multi-body interaction relations by the second multi-body interaction relations extracting means on the basis of the element names entered by the above input means.

17. The literature information processing system as defined in claim 15 or 16, **characterized in that** the above pathway map drawing means discriminates and displays the element names entered by the above input means and those extracted from the element names entered using the above input means by the second multi-body interaction relations extracting means.

18. The literature information processing system as defined in any one of claims 12 to 17, **characterized by** further comprising:
a multi-body interaction categorizing means to categorize multi-body interaction relations stored in the above multi-body interaction storing means on the basis of verbs that indicate mutual interaction relations between the above element names, and
a reliability deciding means to decide the multi-body interaction' s reliability of each of the above verbs on the basis of the multi-body interaction relations of each of the above verbs categorized by the above multi-body interaction categorizing means.

19. The literature information processing system as defined in claim 18, **characterized in that** the reliability deciding means has a graph drawing means for drawing a graph indicating the relations between nodes as the element names and edges as the relationship between the elements, and decides the reliability on the basis of the graph drawn by the above graph drawing means.

20. The literature information processing system as defined in any one of claims 1 to 19, **characterized in that** the above literature information includes content of Internet information.

21. The literature information processing system as defined in any one of claims 1 to 20, **characterized in that** the above element names are protein names or gene names.

22. The literature information processing system as defined in any one of claims 1 to 21, **characterized by** further comprising a search result input means to input the element names based on the results obtained by DNA microarray analysis device.

23. The literature information processing system as defined in claim 22,**characterized in that** the above search result input means enters the element names that are the experimental results obtained from at least two experiments of the above DNA microarray analysis device.

24. The literature information processing system as defined in claim 23, **characterized in that** the above pathway map drawing means classifies and displays every element name based on each experiment.

25. The literature information processing system as defined in claim 23, **characterized in that** the above pathway map drawing means displays all the element names based on each experiment as the element names drawn on the above pathway map.

26. The literature information processing system as defined in claim 23, **characterized in that** the above pathway map drawing means displays the intersections of the element names based on each experiment as the element names drawn on the above pathway map.

27. The literature information processing system as defined in claim 23, **characterized in that** the above pathway map drawing means displays the differences of the element names based on each experiment as the element names drawn on the above pathway map.

28. The literature information processing system, **characterized by** comprising:
a multi-body interaction storing means to store multi-body interaction relations extracted for each of element names,
an input means to enter a set of element names,
an extraction range deciding means to decide extraction range of multi-body interaction relations on the basis of the element names entered by the above input means,
a multi-body interaction relations extracting means that extracts the multi-body interaction relations to the range decided by the above extraction range deciding means and extracts the multi-body interaction relations existing between the element names in the range of consideration already extracted, and
a pathway map drawing means to draw a pathway map on the basis of the multi-body interaction relations extracted by the above multi-body interaction relations extracting means.

29. The literature information processing system as defined in claim 28, **characterized by** comprising:
a relationship pattern storing storage to store the relationship patterns between the element names, and
an identifying means to identify the relationships between the element names on the pathway map drawn by the above pathway map drawing means in reference to the relationship patterns stored in the above relationship pattern storing storage.

30. The literature information processing system, **characterized by** comprising:
a multi-body interaction storing means to store multi-body interaction relations extracted for each of multiple element names,
an input means to enter element names,
a defined restriction condition input means to enter defined restriction conditions that define the range of pathway map to display,
a multi-body interaction relations extracting means to extract multi-body interaction relations for each of plural element names entered in reference to the above multi-body interaction storing means, and
a pathway map drawing means to draw a pathway map on the basis of the multi-body interaction relations extracted by the above multi-body interaction relations extracting means and the defined restriction conditions entered by the above defined restriction condition input means.

31. The literature information processing system as defined in any one of claims 28 to 30, **characterized by** further comprising a specific element name storing storage to store specific element names that have interaction relations with multiple element names, and in that the above pathway map drawing means to modify the display of the attributes of the multi-body interaction relations regarding existing specific element names in reference to the specific element names stored in the above specific element name storing storage.

32. The literature information processing system as defined in any one of claims 28 to 31, **characterized in that** the above pathway map drawing means displays the information showing relationships of each element name on the above pathway map in cases where the multi-body interaction relations extracted by the above multi-body interaction relations extracting means include at least three element names.

33. The literature information processing system as defined in any one of claims 28 to 32, **characterized by** comprising a supplementary information storing storage to store supplementary information regarding the above pathway map, and in that the above pathway map drawing means draws the above pathway map in reference to the supplementary information stored in the above supplementary information storing storage.

34. The literature information processing system as defined in claim 33, **characterized in that** the above supplementary information include the information indicating prescribed abbreviated element names and those indicating the prescribed figure used to show existing prescribed element names, and **in that** the above pathway map drawing means draws a pathway map in reference to the supplementary information with the above prescribed figure when displaying the above prescribed element names.

35. The literature information processing system as defined in claim 33, **characterized in that** the above supplementary information include the information indicating material names that have prescribed relations with the interactions between the above element names, and **in that** the pathway map drawing means draws the pathway map that includes the above material names in reference to the supplementary information.

36. A literature information processing system, **characterized by** comprising:
a literature database to store multiple literature information,
a gene expression information database to store gene expression information,
an input means to enter element names,
a multi-body interaction relations extracting means to extract the multi-body interactions for each of multiple element names entered by the above input means in reference to the above literature database and the above gene expression information database, and
a pathway map drawing means to draw a pathway map on the basis of the multi-body interactions relations extracted by the above multi-body interaction relations extracting means.

37. The literature information processing system as defined in any one of claims 28 to 36, **characterized in that** the above literature information include Internet information.

38. The literature information processing system as defined in any one of claims 28 to 37, **characterized in that** the above element names are protein names or gene names.

39. The literature information processing system as defined in claim 38, **characterized by** assessing whether the multi-body interaction relations extracted by the above multi-body interaction relations extracting means are direct interactions or not in reference to supplementary information stored in supplementary information storing storage that stores the supplementary information indicating the relations of the reactions between the domain structures of existing proteins/each proteins in cases where the above element names are proteins.

40. A literature information processing system, **characterized by** comprising:
a binary relation storing means to store binary relations extracted from each of multiple protein names and gene names,
an input means to enter protein names and gene names,
a defined condition input means to enter binary relation indicating that first protein does the first interaction with the transcription factor which is a gene, binary relation indicating that the above transcription factor does second interaction with the gene of probe, and binary relation indicating that the above gene of probe does third interaction with the above second protein, as defined conditions,
a binary relation extracting means to extract binary relation for each of entered protein names and gene names in reference to the above binary relation storing means, and
a pathway map drawing means to draw a pathway map on the basis of the binary relations extracted by the above binary relation extracting means and the defined conditions entered by the above defined condition input means.

41. The literature information processing system as defined in claim 40, **characterized in that** the above defined condition input means also enters information to limit specific verbs as the verbs prescribing binary relations.

42. A literature information processing system, **characterized by** comprising:
a multi-body interaction storing means to store binary relations indicating relationships between two element names and multi-body interaction relations indicating relationships between more than three element names,
an input means to enter element names,
a multi-body interaction relations extracting means to extract the multi-body interaction relations for each of the element names entered by the input means in reference to the above multi-body interaction storing means, and
a binary relation extracting means to extract the binary relations in reference to the multi-body interaction storing means for each of the element names having the multi-body interaction relations with the entered element names and extracted by the multi-body interaction relations extracting means,
a pathway map drawing means to draw a pathway map on the basis of the multi-body interaction relations extracted by the above multi-body interaction relations extracting means and the binary relations extracted by the above binary relation extracting means.

43. The literature information processing system as defined in claim 42, **characterized in that** the above multi-body interaction relations extracting means extracts multi-body interaction relations indicating relationship between 3, 4, 5, or 6 element names.
